# EUROPEAN PATENT SPECIFICATION

(11) **EP 4 602 064 B1**
(45) Date of publication and mention of the grant of the patent: **13.05.2026**
(21) Application number: 23789524.8
(22) Date of filing: 09.10.2023
(51) Int. Cl.: C07K 14/605, A61K 47/54

(54) **METHOD OF MANUFACTURING A PEPTIDE WITH A LYSINE DERIVATIVE**
VERFAHREN ZUR HERSTELLUNG EINES PEPTIDS MIT EINEM LYSINDERIVAT
PROCÉDÉ DE FABRICATION D'UN PEPTIDE AVEC UN DÉRIVÉ DE LYSINE

(30) Priority: 10.10.2022 CH 11902022; 27.10.2022 CH 12692022
(43) Date of publication of application: 20.08.2025
(73) Proprietor: Bachem Holding AG, 4416 Bubendorf (CH)
(72) Inventor: SCHÖNLEBER, Ralph O., 4416 Bubendorf (CH); KÜMIN, Michael, 4416 Bubendorf (CH); ERMERT, Philipp, 4416 Bubendorf (CH); HINTERMANN, Tobias, 4416 Bubendorf (CH); SCHWINDLING, Joachim, 4416 Bubendorf (CH); RÜEFLI, Pascal, 4416 Bubendorf (CH); WILLIG, Felix, 4416 Bubendorf (CH)
(74) Representative: Jacobi, Markus Alexander
(86) International application number: PCT/EP2023/077884
(87) International publication number: WO 2024/079043

(56) References cited:
- CN-A- 104 356 224
- CN-A- 111 378 028
- MARTIN VINCENT ET AL: "Greening the synthesis of peptide therapeutics: an industrial perspective", RSC ADVANCES, vol. 10, no. 69, 1 January 2020 (2020-01-01), pages 42457 - 42492, XP055948862, DOI: 10.1039/D0RA07204D
- LOUIS A. CARPINO ET AL: "Rapid, Continuous Solution-Phase Peptide Synthesis: Application to Peptides of Pharmaceutical Interest", ORGANIC PROCESS RESEARCH & DEVELOPMENT, vol. 7, no. 1, 1 January 2003 (2003-01-01), pages 28 - 37, XP055008431, ISSN: 1083-6160, DOI: 10.1021/op0202179

## Description

The present invention relates to a method of manufacturing a peptide, which contains a lysine residue with a fatty acid conjugation motif covalently linked to its epsilon nitrogen atom, the method comprising a condensing of an alpha amino acid derivative with a lysine derivative, which possesses the fatty acid conjugation motif covalently linked to its epsilon nitrogen atom and which possesses a specific amino protecting group, namely 1,1-dioxobenzo[b]thiophen-2-ylmethyloxycarbonyl, at its alpha nitrogen atom. It relates further to the lysine derivative and a method of manufacturing the lysine derivative. It relates also to a lysine precursor, which possesses a part of the fatty acid conjugation motif covalently linked to its epsilon nitrogen atom and which possesses the specific amino protecting group at its alpha nitrogen atom, and a method of manufacturing the lysine precursor. It relates additionally to a method of manufacturing a lysine intermediate, which possesses the specific amino protecting group at its alpha nitrogen atom.

Semaglutide (CAS-No. 910463-68-2) is an active pharmaceutical ingredient and is known as a glucagon-like peptide-1 receptor agonist. Semaglutide, when it is written in the three-letter-code of peptides, has the following formula:

H-His¹-Aib-Glu-Gly-Thr⁵-Phe-Thr-Ser-Asp-Val¹⁰-Ser-Ser-Tyr-Leu-Glu¹⁵-Gly-Gln-Ala-Ala-
Lys²⁰(HO-CO-(CH₂)₁₆-CO-gamma-Glu-2-[2-(2-aminoethoxy)ethoxyacetyl]-2-[2-(2-
aminoethoxy)ethoxyacetyl])-Glu-Phe-Ile-Ala-Trp²⁵-Leu-Val-Arg-Gly-Arg³⁰-Gly-OH
(SEQ ID NO:5)

Accordingly, Semaglutide has a linear 31-mer peptide backbone and carries at the epsilon nitrogen atom of its lysine²⁰ a fatty acid side-chain, the epsilon nitrogen atom of lysine²⁰ is substituted with HO-CO-(CH₂)₁₆-CO-gamma-Glu-2-[2-(2-aminoethoxy)ethoxy]acetyl]-2-[2-(2-aminoethoxy)ethoxyacetyl].

WO 2006-097537 A2 discloses in its example 4 the peptide Semaglutide. Semaglutide is synthesized by an acylation of the eplison-nitrogen atom of lysine²⁰ at an already complete linear peptide backbone. Accordingly, the fatty acid side chain at lysine²⁰ is introduced after the complete linear peptide backbone of Semaglutide has been synthesized.

CN 104356224 A discloses a synthesis of Semaglutide, which employs as a building block a Fmoc-protected lysine derivative, which carries already the fatty acid side chain. Accordingly, lysine²⁰ carrying already a fatty acid side chain is introduced during the synthesis of the linear peptide backbone of Semaglutide. The employed Fmoc-protected lysine derivative (CAS-No. 1662688-20-1) is depicted below

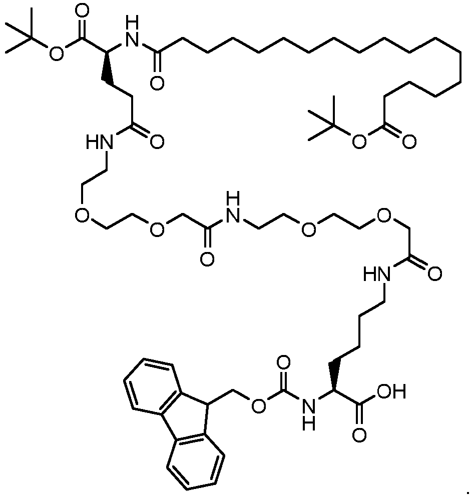

CN 113461801 A discloses at its example 1 a solid phase peptide synthesis of the aforementioned Fmoc-protected lysine derivative (CAS-No. 1662688-20-1) via a solid-phase conjugated Alloc-protected lysine derivative (CAS-No. 2721349-46-6), which is depicted below

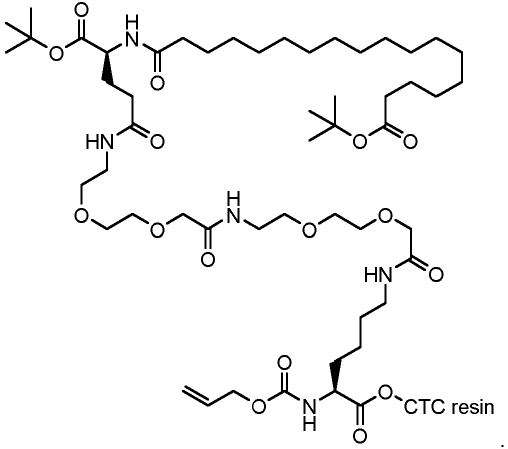

CN 115677827 A discloses at its example 4 a synthesis of Boc-Lys(tert-BuO-CO-(CH₂)₁₆-CO-gamma-Glu-2-[2-(2-aminoethoxy)ethoxyacetyl]-2-[2-(2-aminoethoxy)ethoxyacetyl])-Ala-OAII (CAS-No. 2921565-74-2) via a Boc-protected lysine derivative (CAS-No. 2921565-73-1), which is depicted below

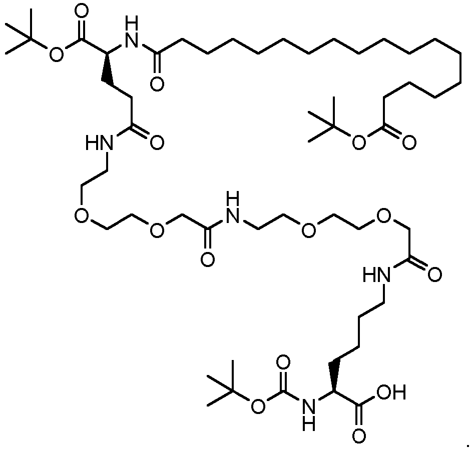

WO 96-25394 discloses 2-(4-nitrophenylsulfonyl)ethoxycarbonyl respectively Nsc-group as an amino-protecting group in solid phase peptide chemistry and states on its page 18 that Nsc-group is perfectly resistant to the action of acidic reagents usually used for the cleavage of protective groups of tert-butyl type.

Protein and Peptide Letters (1997), vol. 4, No. 5, p. 307-312 discloses a comparative study of Fmoc- and Nsc-groups in automated solid phase peptide synthesis. It is concluded that comparative synthesis of three test peptides result in similarly looking HPLC-UV chromatograms. On page 312, a retention time of Nsc-Phe-OH with 16 minutes versus a retention of Fmoc-Phe-OH with 24 minutes is stated for an analytical HPLC with a C18 column and 0.1 % trifluoroacetic acid in a water / acetonitrile gradient. A decomposition seems not to occur at Nsc-Phe-OH in view of the statement of one retention time.

Tetrahedron Letters (1994), vol. 35, no. 42, p. 7821-7824 discloses on its page 7822 the preparation of Nsc-Lys(Boc)-OH via its trimethylsilyl derivative. A yield of 83% is reported for a homogeneous product after recrystallization. During the work-up, an exposure to an aqueous 5% NaHCO₃ solution at an extraction has taken place.

Organic Letters (2001), vol. 3, no. 5, p. 781-783 discloses solid phase peptide synthesis with alpha-azide-protected amino acids and states at its table 2 that alpha-azido acids outperform comparative Fmoc-amino acid at several tested peptide sequences.

Journal American Chemical Society (1997), vol. 119, p. 9915-9916 discloses Bsmoc group, which is an abbreviation for 1,1-dioxobenzo[b]thiophene-2-ylmethyloxycarbonyl, as an amino-protecting group in peptide synthesis.

Bulletin of Korean Chemical Society (1998), vol. 19, no. 6, p. 696-698 discloses 2-(phenylsulfonyl)ethoxycarbonyl respectively Psc group for the orthogonal protection of the epsilon amino group of lysine in conjunction with the Boc group, i.e. Boc-Lys(Psc)-OH, and its usage in liquid phase peptide synthesis.

RU 2196144 C1 discloses at its example 11 a peptide Psc-D-Phe-Cys(Bzm)-Phe-D-Trp(For)-Lys(Psc)-Thr-Cys(Bzm)-Thr-ol(Psc)2 (SEQ ID NO:12) with Psc meaning 2-(phenylsulfonyl)ethoxycarbonyl.

There is still a need for further improvement. An increase of the yield of a condensing reaction is desirable. Especially, if the condensing reaction is a part of a multi-step reaction scheme for a larger molecule, for example a molecule with a molecular weight above 1000 g / mol - which is often the case for a (poly-) peptide. A reason is that a removal of those one or more byproducts, which are generated due to an ineffective condensing reaction and are comparatively large molecules, from the targeted molecule turns often out to be difficult. This is often due to an overall still similar physical behavior and necessitates in the case of a (poly-) peptide often a purification by a preparative high performance liquid chromatography. Thus, an initial purity of the crude material containing the targeted larger molecule is of relevance and contributes to an effectiveness of the condensing reaction. Furthermore, it is beneficial, if an applied starting material has a sufficient, at least temporary stability against degradation at a contact with a base or an acid, for example at a contact with trifluoroacetic acid in the technical area of peptide synthesis. The meaning of 'at least temporary' refers to a sufficient stability under common conditions like room temperature, exposure times of for example up to an hour and for example a presence of water. On the one hand, this allows for example typically a benign synthetic accessibility of the applied starting material, for example during an aqueous work-up. Here again, an initial purity of a crude material containing the starting material and thus an avoidance of a preparative liquid chromatography is of relevance. On the other hand, a possibility for an analytical monitoring of the starting material with a typically acidic analytical reverse phase high performance liquid chromatography in case of a (poly-) peptide allows a better insight into and thus steering of the condensing reaction. Another desirable aspect in case of a (poly-) peptide is that a condensing reaction allows to remain in the scheme of a base-catalyzed deprotection of an amino-protecting group for a further condensing reaction. Thus, a new reactive amine group respectively a new reactive imine group for the further condensing reaction to form an amide bond can be generated without endangering an acid-sensitive linking group to a resin or endangering an acid-sensitive protection group at a side chain of a respective, already condensed amino acid in the pre-existing peptide. Furthermore, a condensing reaction, which does not lead to a presence of a heavy metal during a following deprotection reaction of an amino-protecting group, is sometimes desirable.

The peptide P is preferably a compound of formula Pr-L-S. It will be understood that the term "peptide P" is a designation of the peptide that is synthesized. The designation "P" may also be omitted or set in parentheses without altering the meaning of the peptide which is defined by structural characteristics. The term "Pr-L-S" describes the structure as defined in the depicted structures. It is intended to show the structural elements as laid out below. This designation "Pr-L-S" may also be omitted or set in parentheses without altering the meaning of the peptide which is defined in the depicted structures.

It has now been found a method of manufacturing a peptide P, the method comprises a step (c)
(c) condensing an alpha amino acid derivative S-am, which has one unprotected alpha amino group or one unprotected alpha imino group,
with a compound of formula Pr-L 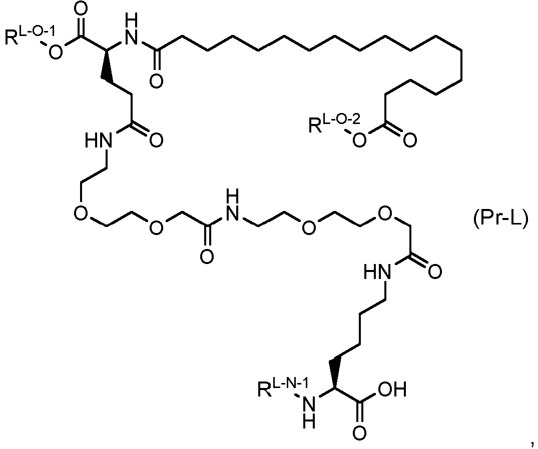
wherein
R^{L-O-1} and R^{L-O-2} are independently of each other a carboxylic acid protecting group,
to obtain a peptide Pr-L-S,
characterized in that R^{L-N-1} is an amino protecting group of formula Bsmoc 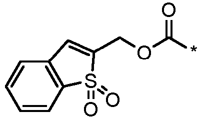 with * indicating the bond to the nitrogen atom
(Bsmoc).

For condensing the one unprotected alpha amino group or the one unprotected alpha imino group of the alpha amino acid derivative S-am with the compound of formula Pr-L, the carboxylic acid group of the compound of formula Pr-L is typically activated with a condensing agent of step (c). The condensing agent of step (c) leads to an activated compound of formula Pr-L, which contains instead of the hydroxy group at the carboxylic acid group a leaving group, which makes the activated compound of formula Pr-L more reactive towards the unprotected alpha amino group or the unprotected alpha imino group of the alpha amino acid derivative Sam. Dependent on the specific condensing agent of step (c), the activated compound of formula Pr-L is an intermediate suitable for an isolation at room temperature or an intermediate unsuitable for an isolation at room temperature. The condensing agent of step (c) is for example a carbodiimide derivative. The carbodiimide derivative is for example diisopropylcarbodiimide, dicyclohexylcarbodiimide, 1-tert-butyl-3-ethylcarobdiimide or 1-ethyl-3-(3-dimethylaminopropyl)-carbodiimide. Preferably, the carbodiimide derivative is diisopropylcarbodiimide, dicyclohexylcarbodiimide or 1-tert-butyl-3-ethylcarbodiimide. More preferably, the carbodiimide derivative is diisopropylcarbodiimide or 1-tert-butyl-3-ethylcarbodiimide. Very preferably, the carbodiimide derivative is diisopropylcarbodiimide. Preferably, the carbodiimide is applied together with a coupling additive of step (c). The coupling additive of step (c) is for example cyano-hydroxyimino-acetic acid ethyl ester, 1-hydroxybenzotriazole, 1-hydroxy-7-azabenzotriazole, 5-hydroxyimino-1,3-dimethylpyrimidine-2,4,6(1H,3H,5H)-trione, ethyl 1-hydroxy-1,2,3-triazole-4-carboxylate, 2-hydroxypryridine N-oxide or a mixture thereof. Preferably, the coupling additive of step (c) is cyano-hydroxyimino-acetic acid ethyl ester, 1-hydroxybenzotriazole, 1-hydroxy-7-azabenzo-triazole or 2-hydroxypryridine N-oxide. More preferably, the coupling additive of step (c) is cyano-hydroxyimino-acetic acid ethyl ester or 1-hydroxybenzotriazole. Very preferably, the coupling additive of step (c) is cyano-hydroxyimino-acetic acid ethyl ester. In case that the alpha amino acid derivative S-am contains an unprotected carboxylic acid group, the activation of the compound of formula Pr-L has to occur prior to a contact with the alpha amino acid derivative Sam. In case the alpha amino acid derivative S-am possesses a functional group, which would interfere with the condensing of step (c) and which is different to the one unprotected alpha amino group or the one unprotected alpha imino group, then the functional group is protected by a suitable protecting group. The alpha amino acid derivative S-am is preferably free of an unprotected guanidino group or an unprotected mercapto group.

Preferred is a method, wherein at step (c) the compound of formula Pr-L is activated with a condensing agent of step (c).

Preferred is a method, wherein at step (c) a coupling additive of step (c) is present.

Preferred is a method, wherein at step (c) the compound of formula Pr-L is activated with a condensing agent of step (c), which is a carbodiimide derivative, and a coupling additive of step (c), which is cyano-hydroxyimino-acetic acid ethyl ester, is present.

Step (c) takes preferably place in the presence of a solvent of step (c). The solvent of step (c) dissolves the compound of formula Pr-L. In case the alpha amino acid derivative S-am is covalently linked to a resin, the solvent of step (c) preferably swells the resin. A solvent of step (c) is for example N,N-dimethylformamide, N-methyl-2-pyrrolidone, N-butyl-pyrrolidone, dimethyl isosorbide, gamma-valerolactone, dihydrolevo-glucosenone (e.g. commercially available Cyrene (TM)), dimethyl sulfoxide, tetrahydropyran, tetrahydrofuran, 2 methyltetrahydrofuran, 1,4-dioxane, 1,3-dioxolane, ethyl acetate, dichloromethane, acetonitrile, toluene, water and mixtures thereof. Preferably, the solvent of step (c) comprises N,N-dimethylformamide. More preferably, the solvent of step (c) comprises at least 20 vol.% N,N-dimethylformamide, very preferably 40 vol.%, especially 70 vol.%, more especially 90 vol.% and very especially more than 95 vol.%. Preferably, the solvent of step (c) is N,N-dimethylformamide.

Preferred is a method, wherein the step (c) is conducted in a solvent of step (c) and the solvent of step (c) comprises N,N-dimethylformamide.

Preferably, the step (c) is conducted at a temperature between 5 °C and 50 °C, more preferably between 10 °C and 40 °C, very preferably between 15 °C and 35 °C, particularly between 18°C and 30 °C, more particularly between 19 °C and 28 °C, very particularly between 20 °C and 27°C and especially between 22 °C and 25 °C. Between 22 °C and 25 °C is herein defined as at room temperature.

Preferred is a method, wherein the step (c) is conducted at a temperature between 15 °C and 35 °C.

Preferably, the compound of formula Pr-L is applied in a molar excess versus the alpha amino acid derivative S-am at step (c). The molar excess is for example a molar amount of the compound of formula Pr-L, which is between 1.05 to 3.5 times the molar amount of the alpha amino acid derivative S-am. Preferably, the molar amount is between 1.3 to 3.0 times, more preferably between 1.5 to 2.5 times, very preferably between 1.7 to 2.3 times, particularly between 1.8 to 2.2 times and more particularly between 1.9 to 2.1 times.

Preferably, the condensation agent of step (c) is applied in a molar excess versus the compound of formula Pr-L at step (c). The molar excess is for example a molar amount of the condensation agent of step (c), which is between 1.05 and 3 times the molar amount of the compound of formula Pr-L. Preferably, the molar amount is between 1.3 to 2.5 times, more preferably between 1.5 to 2.3 times, very preferably between 1.7 to 2.2 times, particularly between 1.8 to 2.2 times and more particularly between 1.9 to 2.0 times. The overall amount of the condensation agent of step (c) is for example added to the compound of formula Pr-L at once or in two or more portions. A portion of the condensation agent of step (c), for example between 50 to 70 wt.% of the overall amount of the condensation agent of step (c), is for example added to the compound of Pr-L, which is dissolved in the solvent of step (c). The resulting solution is stirred for example between 5 and 30 minutes and then added to the alpha amino acid derivative S-am.

Preferably, the coupling additive of step (c) is applied in a molar amount versus the compound of formula Pr-L, which molar amount is between 0.5 and 3 times the molar amount of the compound of formula Pr-L. The molar amount is for example between 0.8 to 2.5 times the molar amount of the compound of formula Pr-L. Preferably, the molar amount is between 0.9 to 2.2 times the molar amount of the compound of formula Pr-L, more preferably between 1.1 to 2.0, very preferably between 1.3 to 1.7 and particularly between 1.4 to 1.6 times.

A peptidic bond is herein understood as a covalent bond between an alpha amino group or an alpha imino group of a first alpha amino acid residue and an alpha carboxylic acid group of a second alpha amino acid residue. The alpha amino acid derivative S is herein understood as either a compound, which is free of a peptidic bond, or a compound, which has at least one peptidic bond. In the latter case, the alpha amino acid derivative S is for example a dipeptide derivative, a tripeptide derivate or a tetrapeptide derivative. At both compounds, there is a N-terminal alpha amino group, which can also be an alpha imino group, of the N-terminal amino acid residue and a C-terminal carboxylic acid group or a carboxamide group of the C-terminal amino acid residue. At the compound, which is free of a peptidic bond, the N-terminal alpha amino group, which can also be an alpha imino group, and the C-terminal carboxylic acid group or carboxamide group are substituents of the same alpha carbon atom respectively belong to the same alpha amino acid residue. The alpha amino acid derivative S-am is for example linked to a resin. The resin comprises a linking group and a polymeric support. The linking group is covalently attached to the polymeric support. The linking group serves to attach covalently an alpha amino acid residue, wherein the covalent attachment of the alpha amino acid residue is cleavable. Typically, the C-terminal alpha amino acid residue is covalently attached to the resin. The resin is for example resin-1 or resin-2. Resin-1 possesses a linking group, which is especially suited for a covalent attachment of an oxygen atom of a carboxylic acid group. The linking group of resin-1 - written as a di-yl-substituent - is for example 2-chlorotrityl-p-amidomethyl, 2-chlorotrityl-yl or 4-(methyleneoxy)benzyl. Resin-2 possesses a linking group, which is especially suited for a covalent attachment of a nitrogen atom of a carboxylic acid amide group. The linking group of resin-2 - written as a di-yl-substituent - is for example xanthen-3-(oxymethylene)-9-yl or alpha-(2,4-dimethoxyphenyl)-alpha-(4-(N-methyleneamido-methyleneoxy)phenyl)methyl. The polymeric support of resin-1 or resin-2 is for example polystyrene, a copolymer containing polymerized styrene units and polymerized ethylene oxide units or polyethylene oxide. The alpha amino acid derivative S-am is for example a single molecule. Preferably, the alpha amino acid derivative S-Am is covalently linked to a resin. Preferably, the alpha amino acid derivative S-Am is a single molecule. Preferably, the alpha amino acid derivative S-Am is free of an unprotected carboxylic acid group.

The peptide P contains a structural element of formula L 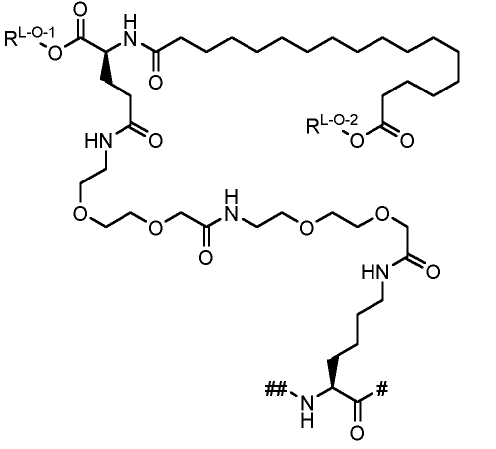 with ^{#} and ^{##} indicating covalent connections
(L),
wherein
R^{L-O-1} and R^{L-O-2} are independently of each other H or a carboxylic acid protecting group.

The covalent connection of formula L, which is indicated by #, is towards the alpha nitrogen atom of the alpha amino acid residue, which is the N-terminal alpha amino acid residue of the alpha amino acid derivative S-am. The covalent connection of formula L, which is indicated by ##, is towards for example a hydrogen atom, an amino protecting group or the alpha carbonyl group of the alpha amino acid residue, which is the C-terminal alpha amino acid residue of a further alpha amino acid derivative.

The alpha amino acid derivative S-am, which has one unprotected alpha amino group or one unprotected alpha imino group, is preferably an alpha amino acid derivative of formula S-I-am or S-II-am 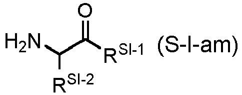 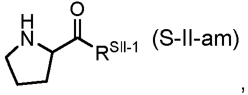 wherein
R^{SI-1} is -(AA^{nx})ₘ-O-[resin-1], -(AA^{nx})ₘ-N-[resin-2], -O-[resin-1], -N-[resin-2], -(AA^{nx})ₘ-OR^{SI-1-1}, OR^{SI-1-1} or NH₂,
R^{SI-2} is H, C₁₋₆ alkyl or C₁₋₆ alkyl mono-substituted with OR^{SI-2-1}, SR^{SI-2-2}, SCH₃, NR^{SI-2-3}R^{SI-2-4}, CO-OR^{SI-2-5}, CO-NR^{SI-2-6}R^{SI-2-7}, N'-R^{SI-2-8}-N"-R^{SI-2-9}-guanidino, phenyl, para-(R^{SI-2-10}O)-phenyl, 1-R^{SI-2-11}-imidazol-4-yl or 1-R^{SI-2-12}-indol-3-yl, -(AA^{nx})ₘ- is a substituent consisting of m condensed alpha amino acid residues AA^{nx} with each x being an integer and x running from 1 to m,
each condensed alpha amino acid residue AA^{nx} is independently chosen and in case it possesses a side chain with a functional group, the functional group is unprotected or protected by a protecting group provided that an interfering functional group is protected,
m is an integer from 1 to 30,
R^{SI-1-1} is H or a carboxylic acid protecting group,
R^{SI-2-1} is H or a hydroxy protecting group,
R^{SI-2-2} is a thiol protecting group,
R^{SI-2-3} and R^{SI-2-4} are H, an amino protecting group or form together an amino protecting group provided that not both are H,
R^{SI-2-5} is H or a carboxylic acid protecting group,
R^{SI-2-6} and R^{SI-2-7} are H or an amide protecting group,
R^{SI-2-8} and R^{SI-2-9} are H or a guanidino protecting group provided that not both are H,
R^{SI-2-10} is a protecting group for an aromatic hydroxy group,
R^{SI-2-11} is H or a protecting group for an imidazole nitrogen atom,
R^{SI-2-12} is H or a protecting group for an indole nitrogen atom,
R^{SII-1} is as defined for R^{SI-1}.

A carboxylic acid protecting group is for example tert-butyl, benzyl, phenacyl, 2-phenyl-isoprop-2-yl or methyl, preferably tert-butyl. Preferably, the carboxylic acid protecting group is tert-butyl or 3-methlyl-pent-3-yl. More preferably, the carboxylic acid protecting group is tert-butyl. Preferably, R^{L-O-1} and R^{L-O-2} are a carboxylic acid protecting group, which is removed under acidic conditions, more preferably under exposure to trifluoracetic acid. Preferably, R^{L-O-1} and R^{L-O-2} are independently of each other tert-butyl or 3-methyl-pent-3-yl. More preferably, R^{L-O-1} and R^{L-O-2} are the same and tert-butyl or 3-methyl-pent-3-yl. Very preferably, R^{L-O-1} and R^{L-O-2} are tert-butyl. A hydroxy protecting group is for example tert-butyl, benzyl, 2-bromobenzyloxycarbonyl, trityl or 2-chlorotrityl, preferably tert-butyl. In case of an alpha amino acid residue, which comprises a hydroxy group in beta-position, the hydroxy protecting group is also for example a group 1,1-dimethyldiyl, which forms an oxazolidine ring together with the nitrogen atom in alpha-position, the carbon atom in alpha position and the oxygen atom of the hydroxy group in beta-position (so-called pseudoproline). A thiol protecting group is for example tert-butyl, 4-methylbenzyl, acetamidomethyl, trityl, tert-butylsulfanyl, tetrahydropyran-2-yl, diphenylmethyl or 2,4,6-trimethoxybenzyl. An amino protecting group is for example tert-butyloxycarbonyl, 9-fluorenyl-methoxycarbonyl, benzyloxycarbonyl or allyloxycarbonyl. An amide protecting group is for example 9-H-xanthen-9-yl, 2,4,6-trimethoxybenzyl, dimethylcyclopropylmethyl, trityl or 3-methylpent-3-yl. A guanidino protecting group is for example 2,2,4,6,7-pentamethyldihydrobenzofuran-5-ylsulfonyl (= Pbf), 2,2,5,7,8-pentamethylchroman-6-ylsulfonyl, mesitylsulfonyl, tosyl, trityl or methoxytrityl, preferably 2,2,4,6,7-pentamethyldihydrobenzofuran-5-ylsulfonyl. A protecting group for an aromatic hydroxy group is for example tert-butyl, benzyl, 2-chlorotrityl or 2-bromogenzyloxycarbonyl. A protecting group for an imidazole nitrogen is for example tert-butyloxycarbonyl, 2,4-dinitrophenyl, benzyloxymethyl, trityl or methoxytrityl, preferably tert-butyloxycarbonyl. A protecting group for an indole nitrogen is for example tert-butyloxycarbonyl or formyl, preferably tert-butyloxycarbonyl.

AAⁿ¹ connects covalently with the nitrogen atom of its alpha amino group to the carbon atom of the carbonyl group depicted at formula S-I-am or S-II-am. AA^{nm} connects covalently with the carbon atom of a carbonyl group to an oxygen or a nitrogen atom. The carbonyl group is preferably the alpha carbonyl group of AA^{nm}. An example of a functional group of a side chain, which interferes with condensing at step (c) is for example an amino group. Accordingly, the amino group is protected by a protecting group.

AA^{nx} is for example, in the respective condensed form as an alpha amino acid residue, glycine, alanine, serine, serine with its side chain protected by tert-butyl, threonine, threonine with a side chain protected by trityl, cysteine with its side chain protected by tert-butyl, valine, leucine, isoleucine, methionine, proline, phenylalanine, tyrosine with its side chain protected by tert-butyl, tryptophane with its side chain protected by tert-butyloxycarbonyl, aspartic acid with its side chain protected by tert-butyl, glutamic acid with its side chain protected by tert-butyl, asparagine, asparagine with its side chain protected by trityl, glutamine, glutamine with its side chain protected by dimethylcyclopropylmethyl, histidine with its side chain protected by trityl, lysine with its side chain protected by allyloxycarbonyl or arginine with its side chain protected by 2,2,4,6,7-pentamethyldihydrobenzofuran-5-ylsulfonyl.

An example for the substituent -(AA^{nx})ₘ- with m = 3, AAⁿ¹ = Gly, AAⁿ² = Arg(Pbf) and AAⁿ³ = Gly is -(Gly-Arg(Pbf)-Gly)- as depicted below 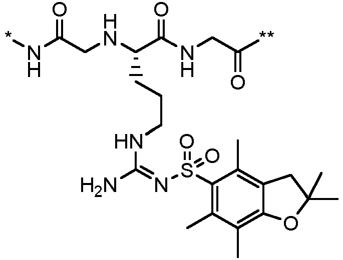 with * and ** indicating the covalent connections.

C₁₋₆ alkyl is for example methyl, 1-methylethyl, 1-methylpropyl or 2-methylpropyl. C₁₋₆ alkyl mono-substituted with OR^{SI-2-1} is for example hydroxymethyl, hydroxymethyl protected by R^{SI-2-1}, 1-hydroxyethyl or 1-hydroxyethyl protected by R^{SI-2-1}. C₁₋₆ alkyl mono-substituted with SR^{SI-2-2} is for example mercaptomethyl protected with R²². C₁₋₆ alkyl mono-substituted with SCH₃ is for example 2-(methylthio)ethyl. C₁₋₆ alkyl mono-substituted with NR^{SI-2-3}R^{SI-2-4} is for example 4-aminobutyl protected by R^{SI-2-3}, R^{SI-2-4} or R^{SI-2-3} and R^{SI-2-4}. C₁₋₆ alkyl mono-substituted with CO-OR^{SI-2-5} is for example carboxymethyl protected with R^{SI-2-5} or 2-carboxyethyl protected by R^{SI-2-5}. C₁₋₆ alkyl mono-substituted with CO-NR^{SI-2-6}R^{SI-2-7} is for example 2-amino-2-oxoethyl or 2-amino-2-oxoethyl protected by R^{SI-2-6}, R^{SI-2-7} or R^{SI-2-6} and R^{SI-2-7}. C₁₋₆ alkyl mono-substituted with CO-NR^{SI-2-6}R^{SI-2-7} is for example also 3-amino-3-oxopropyl or 3-amino-3-oxopropyl protected by R^{SI-2-6}, R^{SI-2-7} or R^{SI-2-6} and R^{SI-2-7}. C₁₋₆ alkyl mono-substituted with N'-R^{SI-2-6}-N"-R^{SI-2-9}-guanidino is for example 3-guanidino-propyl protected by R^{SI-2-6}, R^{SI-2-9} or R^{SI-2-8} and R^{SI-2-9}. C₁₋₆ alkyl mono-substituted with phenyl is for example benzyl. C₁₋₆ alkyl mono-substituted with para-(R^{SI-2-10}O)-phenyl is for example 4-hydroxybenzyl protected by R^{SI-2-10}. C₁₋₆ alkyl mono-substituted with 1-R^{SI-2-11}-imidazol-4-yl is for example imidazol-4-ylmethyl or imidazol-4-ylmethyl protected by R^{SI-2-11} at the 1-position of the imidazole ring. C₁₋₆ alkyl mono-substituted with 1-R^{SI-2-12}-indol-3-yl is for example indol-3-ylmethyl or indol-3-ylmethyl protected by R^{SI-2-12} at the 1-position of the indole ring.

Preferably, R^{SI-1} is -(AA^{nx})ₘ-O-[resin-1], -(AA^{nx})ₘ-N-[resin-2], -O-[resin-1], -N-[resin-2] or NH₂. More preferably, R^{SI-1} is -(AA^{nx})ₘ-O-[resin-1], -(AA^{nx})ₘ-N-[resin-2], -O-[resin-1] or -N-[resin-2].

Preferably, R^{SI-1-1} is a carboxylic acid protecting group. Preferably, R^{SI-2-5} is a carboxylic acid protecting group. Preferably, all AA^{nx} are free of an unprotected carboxylic acid. More preferably, R^{SI-1-1} and R^{SI-2-5} are independently from each other a carboxylic acid protecting group and all AA^{nx} are free of an unprotected carboxylic acid group. More preferably, the alpha amino acid derivative of formula S-I-am or S-II-am is free of an unprotected carboxylic acid group.

Preferably, R^{SI-2} is different to H. More preferably, R^{SI-2} is C₁₋₆ alkyl or C₁₋₆ alkyl mono-substituted with OR^{SI-2-1}, SR^{SI-2-2}, SCH₃, NR^{SI-2-3}R^{SI-2-4}, CO-OR^{SI-2-5}, CO-NR^{SI-2-6}R^{SI-2-7}, N'-R^{SI-2-8}-N"-R^{SI-2-9}-guanidino, phenyl, para-(R^{SI-2-10}O)-phenyl, 1-R^{SI-2-11}-imidazol-4-yl or 1-R^{SI-2-12}-indol-3-yl. Very preferably, R^{SI-2} is C₁₋₆ alkyl mono-substituted with OR^{SI-2-1}, SR^{SI-2-2}, SCH₃, NR^{SI-2-3}R^{SI-2-4}, CO-OR^{SI-2-5}, CO-NR^{SI-2-6}R^{SI-2-7}, N'-R^{SI-2-8}-N"-R^{SI-2-9}-guanidino, phenyl, para-(R^{SI-2-10}O)-phenyl, 1-R^{SI-2-11}-imidazol-4-yl or 1-R^{SI-2-12}-indol-3-yl. Particularly, R^{SI-2} is C₁₋₆ alkyl mono-substituted with CO-OR^{SI-2-5} or CO-NR^{SI-2-6}R^{SI-2-7}.

Preferably, m is an integer from 1 to 25, more preferably from 1 to 20, very preferably from 1 to 15, particularly from 2 to 12, more particularly from 3 to 11 and very particularly from 4 to 10.

Preferably, the alpha carbon atom of the compound of formula S-I-am, which is substituted by R^{SI-2}, is in the S-configuration provided that R^{SI-2} is different to H. Preferably, the alpha carbon atom of the compound of formula S-II-am, which is substituted by the carbonyl group covalently linked to R^{SII-1}, is in the S-configuration.

When the alpha amino acid derivative S-am at step (c) is a compound of formula S-I-am or S-II-am, then the peptide Pr-L-S obtained at step (c) is a compound of formula Pr-L-S-I or Pr-L-S-II 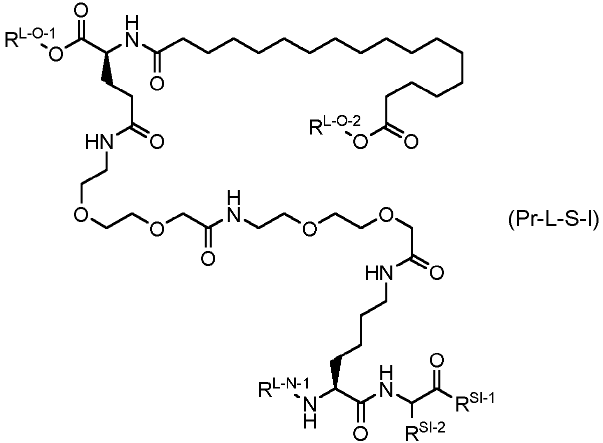 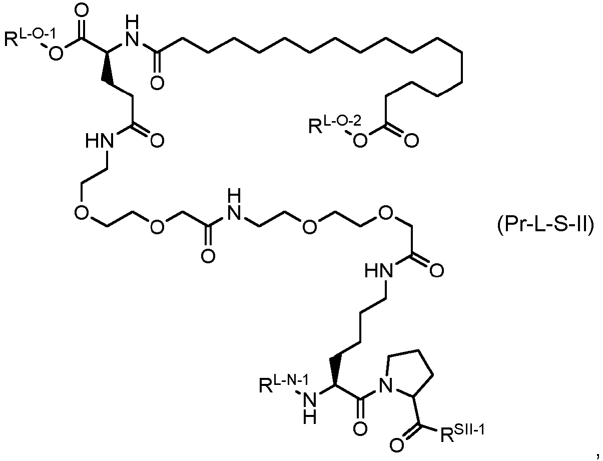 wherein
R^{L-O-1}, R^{L-O-2} and R^{L-N-1} are defined as for formula Pr-L,
R^{SI-1}, R^{SI-2} and R^{SII-1} are defined as for formula S-I-am or S-II-am.

Preferred is a method, wherein the alpha amino acid derivative S-am is a compound of formula S-I-am or S-II-am and the peptide Pr-L-S obtained at step (c) is a compound of formula Pr-L-S-I or Pr-L-S-II.

Preferred is a method, wherein the alpha amino acid derivative S-am of step (c) is of formula SI-am with R^{SI-1} is a -(AA^{nx})₁₀-O-[resin-1], AAⁿ¹ is Phe, AAⁿ² is Ile, AAⁿ³ is Ala, AAⁿ⁴ is a Trp with a protected sidechain, AAⁿ⁵ is Leu, AAⁿ⁶ is Val, AAⁿ⁷ is an Arg with a protected sidechain, AAⁿ⁸ is Gly, AAⁿ⁹ is an Arg with a protected sidechain and AAⁿ¹⁰ is Gly, resin-1 is a 2-chlorotritylamido-methyl resin and R^{SI-2} is 2-(tert-butyloxycarbonyl)ethyl.

Preferred is a method, wherein the alpha amino acid derivative S-am of step (c) is of formula SI-am with R^{SI-1} is a -(AA^{nx})₁₀-O-[resin-1], AAⁿ¹ is Phe, AAⁿ² is Ile, AAⁿ³ is Ala, AAⁿ⁴ is a Trp with a protected sidechain, AAⁿ⁵ is Leu, AAⁿ⁶ is Val, AAⁿ⁷ is an Arg with a protected sidechain, AAⁿ⁸ is Gly, AAⁿ⁹ is an Arg with a protected sidechain and AAⁿ¹⁰ is Gly, resin-1 is a 2-chlorotrityl resin and R^{SI-2} is 2-(tert-butyloxycarbonyl)ethyl.

Preferred is a method, wherein R^{L-O-1} and R^{L-O-2} are independently of each other tert-butyl or 3-methyl-pent-3-yl.

Preferred is a method, wherein the compound of formula Pr-L is compound 508. Compound 508 is depicted at Example B-16-1.

A chemical name for the molecular structure of formula Bsmoc is 1,1-dioxobenzo[b]thiophene-2-ylmethyloxycarbonyl, which is commonly also named benzo[b]thiophenesulfone-2-methyloxy-carbonyl.

Preferably, the method comprises a step (d) of removing the amino protecting group R^{L-N-1} at the compound of formula Pr-L-S-I or Pr-L-S-II to obtain a compound of formula L-S-I-am or L-S-II-am 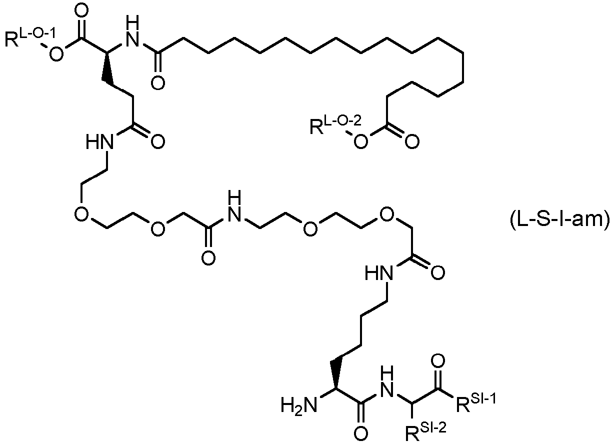 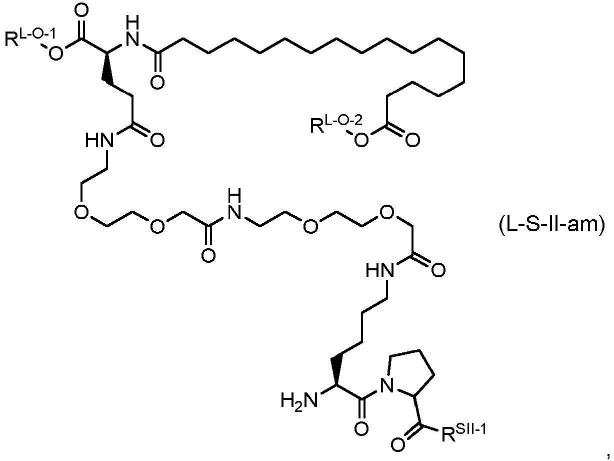 wherein
R^{L-O-1}, R^{L-O-2}, R^{SI-1}, R^{SI-2} and R^{SII-2} are defined as for formula Pr-L-S-I or Pr-L-S-II.

The removal of the amino protecting group R^{L-N-1} is conducted with a deprotection composition of step (d). A deprotection composition of step (d) is for example 20 vol.% piperidine in N,N-dimethylformamide. A suitable selection of the other protecting groups of the compound of formula Pr-L-S-I or Pr-L-S-II leads to a compound of formula L-S-I-am or L-S-II-am, which contains the deprotected alpha amino group and is free of another unprotected amino group or an unprotected imino group, preferably also free of an unprotected carboxylic acid group.

Preferred is a method, which comprises the step (d)
(d) removing the amino protecting group R^{L-N-1} at the compound of formula Pr-L-S-I or Pr-L-S-II to obtain a compound of formula L-S-I-am or L-S-II-am.

Preferably, the method comprises a step (e) of condensing the compound of formula L-S-I-am or L-S-II-am with an alpha-amino acid derivative of formula T

R^{T-N-1}-(AA^{py})_{q}-OH (T)

wherein
R^{T-N-1} is an amino protecting group,
-(AA^{py})_{q}- is a substituent consisting of q condensed alpha amino acid residues AA^{py} with each y being an integer and y running from 1 to q,
each condensed alpha amino acid residue AA^{py} is independently chosen and in case it possesses a side chain with a functional group, the functional group is unprotected or protected by a protecting group provided that an interfering functional group is protected,
AA^{pq} connects covalently with the carbon atom of its alpha carbonyl group to the hydroxy group,
q is an integer from 1 to 30,
to obtain a compound of formula T-L-S-I or T-L-S-II 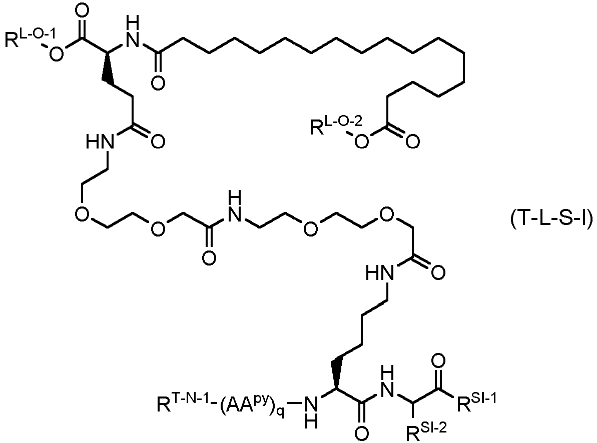 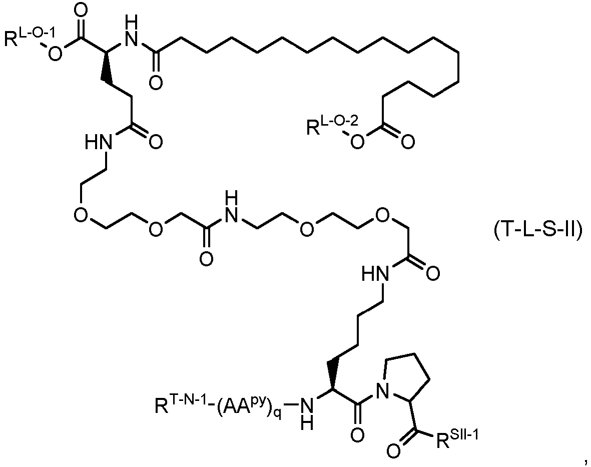 **wherein**
R^{L-O-1}, R^{L-O-2}, R^{SI-1}, R^{SI-2} and R^{SII-2} are defined as for formula L-S-I-am or L-S-II-am,
R^{T-N-1} and -(AA^{py})_{q}- are defined as for formula T.

The alpha amino group of AA^{p1} is protected by R^{T-N-1}. AA^{pq} connects covalently with the carbon atom of its alpha carbonyl group to the depicted hydroxy group of formula T at formula T. AA^{pq} connects covalently with the carbon atom of its alpha carbonyl group to the depicted nitrogen atom of the alpha nitrogen atom of formula T-L-S-I or T-L-S-II at formula T-L-S-I or T-L-S-II. The compound of formula T contains one unprotected carboxylic acid group. The one unprotected carboxylic acid group is formed by the alpha carbonyl group of the amino acid residue AA^{pq} and the hydroxy group depicted at formula T. An example of a functional group of a side chain, which interferes with condensing at step (e) is for example an amino group. Accordingly, the amino group is protected by a protecting group. Examples for AA^{py} are those listed for AA^{nx} at step (c).

An example of a compound of formula T with q = 1, R^{T-N-1} = 9-fluorenylmethyloxycarbonyl and AA^{p1} = alanine residue is for example Fmoc-Ala-OH as depicted below

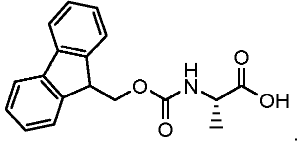

Preferably, q is an integer from 1 to 25, more preferably from 1 to 20, very preferably from 1 to 15, particularly from 1 to 10, more particularly from 1 to 8, very particularly from 1 to 5, especially from 1 to 3, more especially from 1 to 2 and very especially q is 1.

For condensing the one unprotected alpha amino group of the compound of formula L-S-I-am or L-S-II-am, the carboxylic acid group of the compound of formula T is typically activated with a condensing agent of step (e). The condensing agent of step (e) leads to an activated compound of formula T, which contains instead of the hydroxy group at the carboxylic acid group a leaving group, which makes the activated compound of formula T more reactive towards the unprotected alpha amino group of the compound of formula L-S-I-am or L-S-II-am. Dependent on the specific condensing agent of step (e), the activated compound of formula T is an intermediate suitable for an isolation at room temperature or an intermediate unsuitable for an isolation at room temperature. In case that the compound of formula L-S-I-am or L-S-II-am contains an unprotected carboxylic acid group, the activation of the compound of formula T has to occur prior to a contact with the compound of formula L-S-I-am or L-S-II-am. The condensing agent of step (e) is for example a carbodiimide. The carbodiimide is for example diisopropylcarbodiimide. Preferably, the carbodiimide is applied together with a coupling additive of step (e). The coupling additive of step (e) is for example cyano-hydroxyimino-acetic acid ethyl ester.

Preferred is a method, which comprises the step (e)
(e) condensing the compound of formula L-S-I-am or L-S-II-am with an alpha-amino acid derivative of formula T to obtain a compound of formula T-L-S-I or T-L-S-II.

Preferably, the method comprises after the condensing at step (c) one or more further condensing cycles to elongate the peptide Pr-L-S obtained at step (c) by one or more further alpha amino acid residues. A condensing cycle comprises a first step of removing the amino protecting group of the alpha amino group of the N-terminal alpha amino acid residue of the peptide to be elongated to obtain one unprotected amino group at the peptide to be elongated.

The amino group the alpha amino group of the N-terminal alpha amino acid residue means also an imino group, for example in case of proline being the N-terminal alpha amino acid residue of the peptide to be elongated. The condensing cycle comprises also a second step of condensing the one unprotected amino acid group from the first step with an alpha amino acid derivative, which has an alpha amino group at its N-terminal amino acid residue, which is protected by an amino protecting group and which has one unprotected alpha carboxylic acid group, which is located at its C-terminal amino acid residue, to obtain a peptide resulting from the condensing cycle. An example of the first step is the step (d). An example of the second step is the step (e).

Optionally, a condensing step is followed by a capping step, i.e. treating of a reaction mixture, which contains the obtained peptide of the condensing step, by acetic acid anhydride. The capping step is prior to removing an amino protecting group, e.g. a first step of removing an amino protecting group as part of a condensing cycle. Potential residual amino groups or imino groups, which have not reacted in the condensing step, are thus acetylated and not amenable in the next condensing step.

Preferred is a method, which comprises one or more further condensing cycles applied to the compound of formula T-L-S-I or T-L-S-II, each of the one or more further condensing cycles comprises
a first step of removing the amino protecting group R^{T-N-1} of the compound of formula T-L-S-I or T-L-S-II respectively the amino protecting group of the alpha amino group of the N-terminal amino acid residue of the peptide resulting from the previous condensing cycle to obtain the related peptide with one unprotected amino group, and
a second step of condensing the unprotected amino group of the related peptide obtained at the first step with an alpha amino acid derivative, which has an alpha amino group at its N-terminal amino acid residue, which is protected by an amino protecting group and which has one unprotected alpha carboxylic acid group, which is located at its C-terminal amino acid residue, to obtain a peptide resulting from the condensing cycle.

In case the alpha amino acid derivative S-am is covalently linked to a resin, the peptide Pr-L-S obtained at step (c) is also covalently linked to the resin. This applies also to an elongated peptide obtained in one or more further condensing cycles. The peptide obtained at the last conducted condensing step can be cleaved from the resin by a cleavage composition to obtain a cleaved peptide. Whether protecting groups of the elongated peptide are retained, partly removed or completely removed at the cleaved peptide depends inter alia on the cleavage composition, the reaction conditions and the specific protecting groups.

Preferably, the alpha amino acid derivative S-am at step (c) is covalently linked to a resin and the method comprises a step (x) of
(x) cleaving the peptide obtained from the last conducted condensing step from the resin by a cleaving composition to obtain a cleaved peptide.

In case the alpha amino acid derivative S-am at step (c) is covalently linked to a resin via a carbonyl group of its C-terminal alpha amino acid residue, the cleaved peptide contains a carboxylic acid group at its C-terminal alpha amino acid residue, which has an unprotected alpha carboxylic acid group or an unprotected alpha carboxamide group.

Preferred is a method, wherein the alpha amino acid derivative S-am at step (c) is covalently linked to a resin and the method comprises the step (x)
(x) cleaving the peptide obtained from the last conducted condensing step from the resin by a cleaving composition to obtain a cleaved peptide.

Preferably, the method comprises a step (y) of
(y) removing all remaining protecting groups from the peptide obtained from the last conducted condensing step to obtain a peptide free of protecting groups.

Removing of a protecting group occurs by a deprotection composition. If the protecting groups of the peptide obtained from the last conducted condensing step are all removable by the same deprotection composition, e.g. all protecting groups are labile towards trifluoroacetic acid, then the removal requires only one deprotection composition of step (y). In case the peptide obtained from the last obtained from the last conducted condensing step is covalently linked to a resin, the step (y) and the step (x) can occur at the same time, if the cleavage composition of step (x) acts also as a deprotection composition of step (y) for all protecting groups of the peptide obtained from the last conducted condensing step, e.g. all protecting groups are labile towards trifluoroacetic acid and the covalent bond to the linking group of the resin is also labile towards trifluoroacetic acid.

Preferred is a method, wherein the method comprises the step (y)
(y) removing all remaining protecting groups from the peptide obtained from the last conducted condensing step to obtain a peptide free of protecting groups.

An example of a peptide P, which is free of protecting groups, is semaglutide (CAS-No. 910463-68-2) as depicted in the three letter code of alpha amino acid residues (except for the condensed L-lysine residue with its fatty acid conjugation motif covalently linked to its epsilon nitrogen atom, which is depicted as a structural element of formula L) with the numbering of the alpha amino acid residues starting with 1 for N-terminal condensed L-histidine residue and ending with 31 for C-terminal condensed glycine residue (corresponding to SEQ ID NO:5):

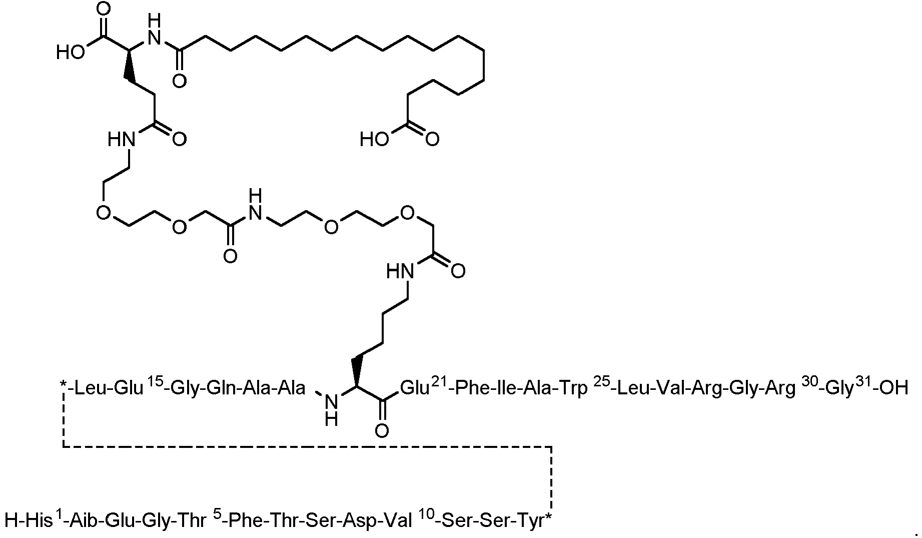

The explanations and preferences described above for the method of manufacturing a peptide P and for its steps apply similarly to further embodiments of the invention.

A further embodiment of the invention is a compound of formula Pr-L 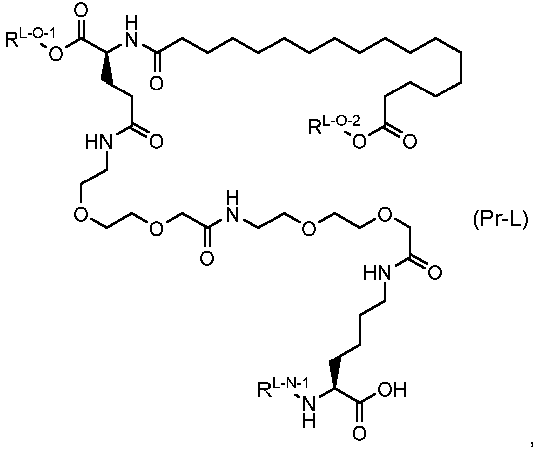 wherein
R^{L-O-1} and R^{L-O-2} are independently of each other a carboxylic acid protecting group,
R^{L-N-1} is an amino protecting group of formula Bsmoc 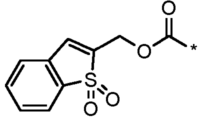 with * indicating the bond to the nitrogen atom
   (Bsmoc).

Preferred is a compound of formula Pr-L, wherein R^{L-O-1} and R^{L-O-2} are independently of each other tert-butyl or 3-methyl-pent-3-yl.

Preferred is a compound of formula Pr-L, wherein R^{L-O-1} and R^{L-O-2} are tert-butyl.

Preferred is a compound of formula Pr-L, which is compound 508. Compound 508 is depicted at Example B-16-1.

A further embodiment of the invention is a use of a compound of formula Pr-L 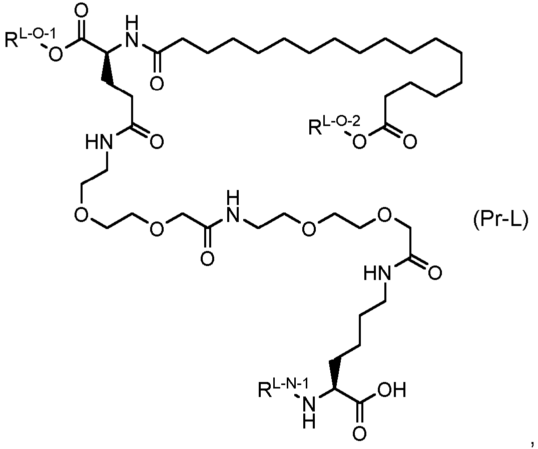 wherein
R^{L-O-1} and R^{L-O-2} are independently of each other a carboxylic acid protecting group,
R^{L-N-1} is an amino protecting group of formula Bsmoc 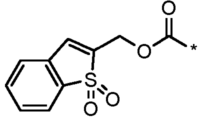 with * indicating the bond to the nitrogen atom
   (Bsmoc),

in the synthesis of a peptide P.

A further embodiment of the invention is a method of manufacturing a compound of formula Pr-L 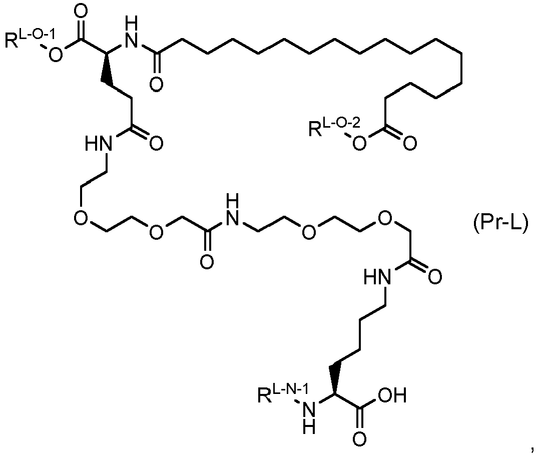 wherein
R^{L-O-1} and R^{L-O-2} are independently of each other a carboxylic acid protecting group,
R^{L-N-1} is an amino protecting group of formula Bsmoc 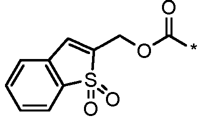 with * indicating the bond to the nitrogen atom
   (Bsmoc),
the method comprises steps (prl-b) and (prl-c)
(prl-b) removing an amino protecting group R^{A-N-1} at the peptide of formula Pr-A 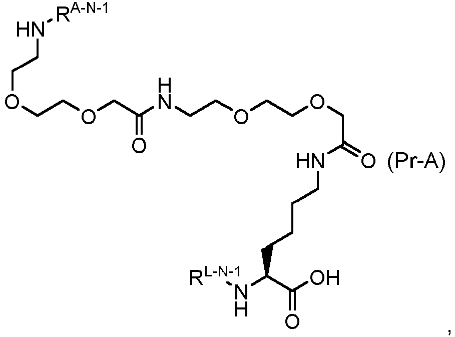 wherein
   R^{A-N-1} is an amino protecting group different to R^{L-N-1},
   R^{L-N-1} is defined as for formula Pr-L,
   to obtain a compound of formula Pr-A-am 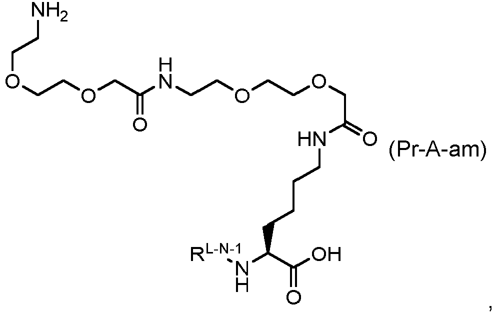 wherein
   R^{L-N-1} is defined as for formula Pr-L,
(prl-c) condensing the compound of formula Pr-A-am with a compound of formula B 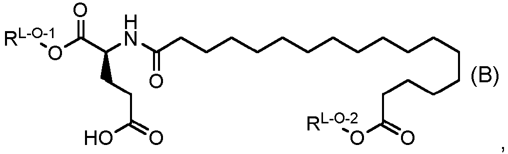 wherein
   R^{L-O-1} and R^{L-O-2} are defined as for formula Pr-L,
   to obtain the compound of formula Pr-L.

Preferred is the method of manufacturing a compound of formula Pr-L, wherein at step (prl-c) the compound of formula Pr-A-am is persilylated to obtain a persilylated intermediate compound, the compound of formula B is activated with a condensing agent of step (prl-c) to obtain an activated compound of formula B, the persilylated compound and the activated compound are combined and react with each other.

Preferably, the method of manufacturing a peptide P employs a compound of formula Pr-L, which is obtained from the method of manufacturing a compound of formula Pr-L.

A further embodiment of the invention is a compound of formula Pr-A 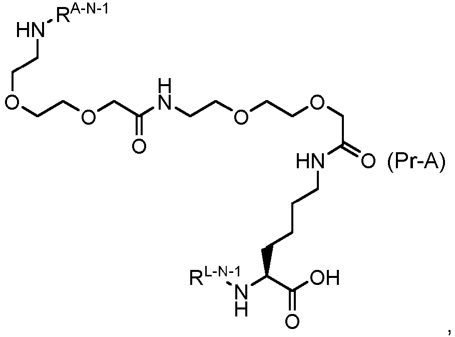 wherein
R^{A-N-1} is an amino protecting group different to R^{L-N-1}, 1-(4,4-dimethyl-2,6-dioxocyclohexylidene)ethyl and 1-(4,4-dimethyl-2,6-dioxocyclohexylidene)-3-methylbutyl,
R^{L-N-1} is an amino protecting group of formula Bsmoc 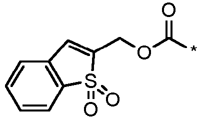 with * indicating the bond to the nitrogen atom
   (Bsmoc).

Preferably, R^{A-N-1} is tert-butyloxycarbonyl.

Preferably, the method of manufacturing a peptide P employs at its step (c) a compound of formula Pr-L, which is obtained by using a compound of formula Pr-A.

A further embodiment of the invention is a method of manufacturing a compound of formula Pr-A 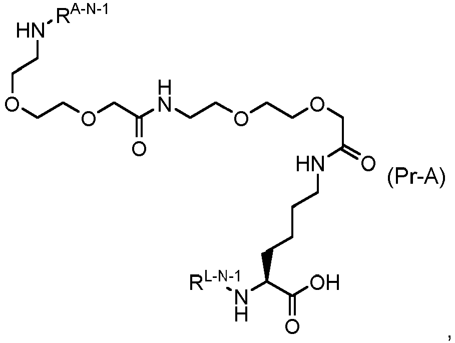 wherein
R^{A-N-1} is an amino protecting group different to R^{L-N-1},
R^{L-N-1} is an amino protecting group of formula Bsmoc 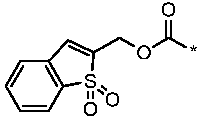 with * indicating the bond to the nitrogen atom
   (Bsmoc),
the method comprises steps (pra-b) and (pra-c)
(pra-b) removing an amino protecting group R^{IntA-N-1} at the compound of formula Pr-Int-A 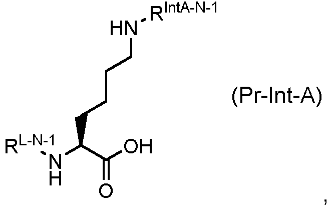 wherein
   R^{IntA-N-1} is an amino protecting group different to R^{L-N-1}
   R^{L-N-1} is defined as for formula Pr-A,
   to obtain a compound of formula Pr-Int-A-ep-am 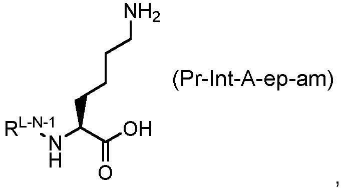 wherein
   R^{L-N-1} is defined as for formula Pr-A,
(pra-c) condensing the compound of formula Pr-Int-A-ep-am with a compound of formula E 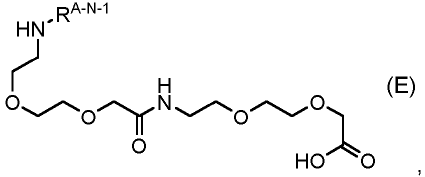 wherein
   R^{A-N-1} **is** defined as for formula Pr-A,
   to obtain the compound of formula Pr-A.

Preferably, the method of manufacturing a peptide P employs at its step (c) a compound of formula Pr-L, which is obtained by using a compound of formula Pr-A, which is obtained from the method of manufacturing a compound of formula Pr-A.

Preferably, the method of manufacturing a peptide P employs at its step (c) a compound of formula Pr-L, which is obtained from the method of manufacturing a compound of formula Pr-L, which employs at its step (prl-b) a compound of formula Pr-A, which is obtained from the method of manufacturing a compound of formula Pr-A.

A further embodiment of the invention is a method of manufacturing a compound of formula Pr-Int-A 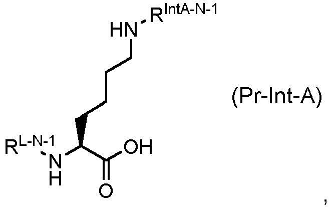 wherein
R^{IntA-N-1} is an amino protecting group different to R^{L-N-1}
R^{L-N-1} is an amino protecting group of formula Bsmoc 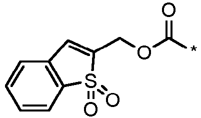 with * indicating the bond to the nitrogen atom
   (Bsmoc),
the method comprises the step
(c-int) reacting a compound of formula Int-A-am 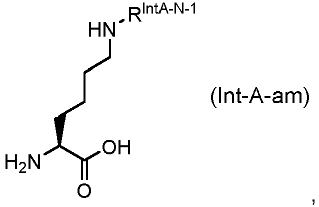 wherein
R^{IntA-N-1} is defined as for formula Pr-Int-A,
with a compound of formula Pr-F 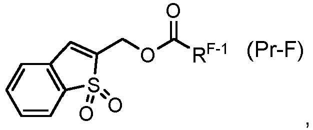 wherein
R^{F-1} is p-nitrophenoxy, (4,6-dimethylpyrimidin-2-yl)-mercapto-yl or phthalimido-N-oxy,
to obtain the compound of formula Pr-Int-A.

Preferably, the method of manufacturing a peptide P employs at its step (c) a compound of formula Pr-L, which is obtained by using a compound of formula Pr-A, which is obtained from the method of manufacturing a compound of formula Pr-A, which employs at its step (pra-b) a compound of formula Pr-Int-A, which is obtained from the method of manufacturing a compound of formula Pr-Int-A.

Preferably, the method of manufacturing a peptide P employs at its step (c) a compound of formula Pr-L, which is obtained from the method of manufacturing a compound of formula Pr-L, which employs at its step (prl-b) a compound of formula Pr-A, which is obtained from the method of manufacturing a compound of formula Pr-A, which employs at its step (pra-b) a compound of formula Pr-Int-A, which is obtained from the method of manufacturing a compound of formula Pr-Int-A.

### Brief description of the Figures

Fig. 1 depicts a complete HPLC-UV chromatogram from D-02-1. One of the two peaks represents compound 701 and the other one is a decomposition product. Both peaks are marked and their area percentages are stated.
Fig. 2 depicts an extract of the HPLC-UV chromatogram from D-02-1 of Fig. 1 between around 14 and 20 minutes. The two peaks, one of which represents compound 701, are marked and their area percentages are stated.
Fig. 3 depicts a complete HPLC-UV chromatogram from D-02-2. A peak representing compound 702 is marked and its area percentage is stated.
Fig. 4 depicts an extract of the HPLC-UV chromatogram from D-02-2 of Fig. 3 between around 10 and 22 minutes. The peak representing compound 702 is marked and its area percentage is stated.
Fig. 5 depicts a complete HPLC-UV chromatogram from D-02-3. A peak representing compound 702 is marked and its area percentage is stated.
Fig. 6 depicts an extract of the HPLC-UV chromatogram from D-02-3 of Fig. 5 between around 14 and 24 minutes. The peak representing compound 702 is marked and its area percentage is stated.
Fig. 7 depicts a complete HPLC-UV chromatogram from D-02-4. A peak representing compound 702 is marked and its area percentage is stated.
Fig. 8 depicts an extract of the HPLC-UV chromatogram from D-02-4 of Fig. 7 between around 11 and 22 minutes. The peak representing compound 702 is marked and its area percentage is stated.
Fig. 9 depicts a complete HPLC-UV chromatogram from D-02-5. A peak representing compound 702 is marked and its area percentage is stated.
Fig. 10 depicts an extract of the HPLC-UV chromatogram from D-02-5 of Fig. 9 between around 14 and 20 minutes. The peak representing compound 702 is marked and its area percentage is stated.
Fig. 11 depicts a complete HPLC-UV chromatogram from D-02-6. A peak representing compound 702 is marked and its area percentage is stated.
Fig. 12 depicts an extract of the HPLC-UV chromatogram from D-02-6 of Fig. 11 between around 14 and 20 minutes. The peak representing compound 702 is marked and its area percentage is stated.
Fig. 13 depicts a complete HPLC-UV chromatogram from D-04-2. A peak representing compound 704 is marked and its area percentage is stated.
Fig. 14 depicts an extract of the HPLC-UV chromatogram from D-04-2 of Fig. 13 between around 10 and 22 minutes. The peak representing compound 704 is marked and its area percentage is stated.
Fig. 15 depicts a complete HPLC-UV chromatogram from D-04-3. A peak representing compound 704 is marked and its area percentage is stated.
Fig. 16 depicts an extract of the HPLC-UV chromatogram from D-04-3 of Fig. 15 between around 14 and 24 minutes. The peak representing compound 704 is marked and its area percentage is stated.
Fig. 17 depicts a complete HPLC-UV chromatogram from D-04-4. A peak representing compound 704 is marked and its area percentage is stated.
Fig. 18 depicts an extract of the HPLC-UV chromatogram from D-04-4 of Fig. 17 between around 11 and 22 minutes. The peak representing compound 704 is marked and its area percentage is stated.
Fig. 19 depicts a complete HPLC-UV chromatogram from D-04-5. A peak representing compound 704 is marked and its area percentage is stated.
Fig. 20 depicts an extract of the HPLC-UV chromatogram from D-04-5 of Fig. 19 between around 14 and 20 minutes. The peak representing compound 704 is marked and its area percentage is stated.
Fig. 21 depicts a complete HPLC-UV chromatogram from D-04-6. A peak representing compound 704 is marked and its area percentage is stated.
Fig. 22 depicts an extract of the HPLC-UV chromatogram from D-04-6 of Fig. 21 between around 14 and 22 minutes. The peak representing compound 704 is marked and its area percentage is stated.
Fig. 23 depicts a complete HPLC-UV chromatogram from D-06-1. A peak representing compound 706 is marked and its area percentage is stated.
Fig. 24 depicts an extract of the HPLC-UV chromatogram from D-06-1 of Fig. 23 between around 9 and 14 minutes. The peak representing compound 706 is marked and its area percentage is stated.
Fig. 25 depicts a complete HPLC-UV chromatogram from D-07-1. A peak representing compound 707 is marked and its area percentage is stated.
Fig. 26 depicts an extract of the HPLC-UV chromatogram from D-07-1 of Fig. 25 between around 10 and 14 minutes. The peak representing compound 707 is marked and its area percentage is stated.
Fig. 27 depicts a complete HPLC-UV chromatogram from D-08-1. A peak representing compound 708 is marked and its area percentage is stated.
Fig. 28 depicts an extract of the HPLC-UV chromatogram from D-08-1 of Fig. 27 between around 10 and 22 minutes. The peak representing compound 708 is marked and its area percentage is stated.
Fig. 29 depicts a complete HPLC-UV chromatogram from D-09-1. A peak representing compound 709 is marked and its area percentage is stated.
Fig. 30 depicts an extract of the HPLC-UV chromatogram from D-09-1 of Fig. 29 between around 14 and 24 minutes. The peak representing compound 709 is marked and its area percentage is stated.
Fig. 31 depicts a complete HPLC-UV chromatogram from D-10-1. A peak representing compound 710 is marked and its area percentage is stated.
Fig. 32 depicts an extract of the HPLC-UV chromatogram from D-10-1 of Fig. 31 between around 11 and 22 minutes. The peak representing compound 710 is marked and its area percentage is stated.
Fig. 33 depicts a complete HPLC-UV chromatogram from D-11-1. A peak representing compound 711 is marked and its area percentage is stated.
Fig. 34 depicts an extract of the HPLC-UV chromatogram from D-11-1 of Fig. 33 between around 14 and 22 minutes. The peak representing compound 711 is marked and its area percentage is stated.

### Sequence Listing

Compound 701
   KEFIAWLVRGRG (SEQ ID NO:1)
   K at position 1 = 2-(p-nitrophenylsulfonyl)ethoxycarbonyl-lysine(17-carboxy-heptadecacarbonyl-gamma-Glu-2-[2-(2-aminoethoxy)ethoxyacetyl]-2-[2-(2-aminoethoxy)ethoxyacetyl])
Compound 702
   KEFIAWLVRGRG (SEQ ID NO:2)
   K at position 1 = lysine(17-carboxy-heptadecacarbonyl-gamma-Glu-2-[2-(2-aminoethoxy)-ethoxyacetyl]-2-[2-(2-aminoethoxy)ethoxyacetyl])
Compound 703
   AKEFIAWLVRGRG (SEQ ID NO:3)
   A at position 1 = 9-fluorenylmethyloxycarbonyl-alanine
   K at position 2 = lysine(17-carboxy-heptadecacarbonyl-gamma-Glu-2-[2-(2-aminoethoxy)-ethoxyacetyl]-2-[2-(2-aminoethoxy)ethoxyacetyl])
Compound 704
   AKEFIAWLVRGRG (SEQ ID NO:4)
   K at position 2 = lysine(17-carboxy-heptadecacarbonyl-gamma-Glu-2-[2-(2-aminoethoxy)-ethoxyacetyl]-2-[2-(2-aminoethoxy)ethoxyacetyl])
Compound 705 (Semaglutide)
   HXEGTFTSDVSSYLEGQAAKEFIAWLVRGRG (SEQ ID NO:5)
   X at position 2 = Aib
   K at position 20 = lysine(17-carboxy-heptadecacarbonyl-gamma-Glu-2-[2-(2-aminoethoxy)-ethoxyacetyl]-2-[2-(2-aminoethoxy)ethoxyacetyl])
Compound 706
   FIAWLVRGRG (SEQ ID NO:6)
Compound 707
   EFIAWLVRGRG (SEQ ID NO:7)
Compound 708
   KEFIAWLVRGRG (SEQ ID NO:8)
   K at position 1 = alpha-azido-lysine(17-carboxy-heptadecacarbonyl-gamma-Glu-2-[2-(2-aminoethoxy)ethoxyacetyl]-2-[2-(2-aminoethoxy)ethoxyacetyl])
Compound 709
   KEFIAWLVRGRG (SEQ ID NO:9)
   K at position 1 = 9-fluorenylmethyloxycarbonyl-lysine(17-carboxy-heptadecacarbonyl-gamma-Glu-2-[2-(2-aminoethoxy)ethoxyacetyl]-2-[2-(2-aminoethoxy)ethoxyacetyl])
Compound 710
   KEFIAWLVRGRG (SEQ ID NO:10)
   K at position 1 = 2-(phenylsulfonyl)ethoxycarbonyl-lysine(17-carboxy-heptadecacarbonyl-gamma-Glu-2-[2-(2-aminoethoxy)ethoxyacetyl]-2-[2-(2-aminoethoxy)ethoxyacetyl])
Compound 711
   KEFIAWLVRGRG (SEQ ID NO:11)
   K at position 1 = 1,1-dioxobenzo[b]thiophene-2-yl-methyloxycarbonyl-lysine(17-carboxy-hepta-decacarbonyl-gamma-Glu-2-[2-(2-aminoethoxy)ethoxyacetyl]-2-[2-(2-aminoethoxy)ethoxy-acetyl])

### Examples

### A) general

### A-1) abbreviations

- %: if not stated differently, a percentage value refers to weight percent

- ACN: acetonitrile
- Aib: 2-aminoisobutyric acid
- Bsmoc: 1,1-dioxobenzo[b]thiophene-2-ylmethyloxycarbonyl (commonly also named: benzo[b]thiophenesulfone-2-methyloxycarbonyl)
- Boc: tert-butyloxycarbonyl
- DBU: 1,8-diazabicyclo(5.4.0)undec-7-ene
- DEPBT: 3-(diethoxyphosphoryloxy)-3H-benzo[d][1,2,3]triazin-4-one
- DIC: diisopropylcarbodiimide
- DIPEA: diisopropylethylamine
- DMF: N,N-dimethylformamide
- EDT: ethane-1,2-dithiol
- Fmoc: 9-fluorenylmethyloxycarbonyl
- HPLC: high performance liquid chromatography
- IPA: isopropanol
- Mpe: 3-methylpent-3-yl
- MTBE: methyl tert-butyl ether
- N₃: azido
- NMP: N-methylpyrrolidone
- Nsc: 2-(p-nitrophenylsulfonyl)ethoxycarbonyl
- OxymaPure (TM): cyano-hydroxyimino-acetic acid ethyl ester
- Pbf: 2,2,4,6,7-pentamethyl-2,3-dihydrobenzfuran-5-ylsulfonyl
- Psc: 2-(phenylsulfonyl)ethoxycarbonyl
- Psi(Me,Me)pro: 1,1-dimethylmethdiyl, which is covalently linked to the beta oxygen atom of serine or threonine and covalently linked to the alpha-nitrogen of serine or threonine (pseudoproline)
- RT: room temperature
- TBTU: (benzotriazolyl)tetramethyluronium tetrafluoroborate
- tBu: tert-butyl
- TFA: trifluoroacetic acid
- TIS: triisopropylsilane
- Trt: trityl
- UV: ultraviolet
- vol.%: volume percents

If not stated differently, alpha amino acids different to glycine are in L-form. Room temperature means herein a temperature between 22 °C and 25 °C.

### A-2) chemicals

All reagents and solvents are obtained from standard suppliers of raw materials for peptide synthesis and are used as received.

### A-3) analytical methods

### A-3-1: HPLC method 1

HPLC method 1 is an analytical HPLC, which is performed on a Dionex (TM) Ultimate (TM) 3000 RS UHPLC system using ACQUITY (TM) UPLC BEH 130 C18 (1.7 µm, 2.1 × 50 mm) column with a flow rate of 0.4 mL / min, an UV detection at 220 nm or a CAD detection and an oven temperature of 25 °C. Buffer A is 0.05 vol.% TFA in ACN / H₂O (1:99 v/v) and buffer B is 0.05 vol.% TFA in ACN.

### A-3-2: HPLC method 2

HPLC method 2 is an analytical HPLC, which is performed on a Thermo Scientific (TM) Vanquish (TM) UHPLC system or a Dionex (TM) Ultimate (TM) 3000 RS UHPLC system using ACQUITY (TM) UPLC BEH 130 C18 (1.7 µm, 2.1 × 150 mm) column with a flow rate of 0.4 mL / min, UV detection at 220 nm and 50 °C oven temperature. In case of a mass spectroscopy, the MS analysis is performed on a UHPLC coupled with Bruker (TM) maXis II (TM) spectrometer with ultra-high resolution QTOF technology equipped with electron-transfer dissociation (ETD) capabilities. Buffer A is 0.05 vol.% TFA in ACN / H₂O (1:99 v/v) and buffer B is 0.05 vol.% TFA in ACN.

Gradient program at HPLC method 2

| time (min) | buffer A | buffer B |
|---|---|---|
| 0 | 100 | 0 |
| 30 | 0 | 100 |

### A-4) synthetic general procedures

### A-4-1: general procedure 1 for condensing a lysine derivative

200 mg of compound 612-SP (0.22 mmol / g, 0.044 mmol), which is depicted at example C-06-1, is pre-swelled two times in DMF (10 mL / g resin) each for 15 min at RT. A respective lysine derivative (0.088 mmol, 2.0 eq.) and OxymaPure (19.4 mg, 0.136 mmol, 3.1 eq.) are dissolved in DMF (10 mL / g resin). To this solution, 17.7 µL DIC (0.114 mmol, 2.6 eq.) are added. The solution is stirred for 15 min at RT and afterwards added to the reaction mixture. After 20 min of reaction time at RT, an additional portion of 8.8 µL DIC (0.057 to 0.143 mmol, 1.3 eq.) is added to the reaction mixture and stirred for typically 24 h at RT. The liquid parts are removed, the resin material is washed three times alternating with DMF / IPA and three times with IPA (each washing step with 10 mL / g resin). The washed peptide material is dried 24 h in high vacuum at RT to obtain the respective product from the condensing of the lysine derivative with compound 612-SP.

For analytical purposes, a small-scale test cleavage can be performed.

### A-4-2: general procedure 2 for test cleavage

Peptide is cleaved from the resin in a 2 mL plastic syringe (50 to 100 mg peptide resin) upon treatment of the peptide resin with 0.5 to 1.0 mL cleavage cocktail (TFA / TIS / H₂O = 90 / 5 / 5 based on volume) for 2 h at RT. The cleaved peptide in solution is then precipitated in 5 to 10 mL cold diisopropyl ether (< -15 °C), centrifuged and washed two additional times with cold diisopropyl ether. The obtained crude peptide, which is typically solid, is dried in high vacuum.

### A-4-3: general procedure 3 for Fmoc-Ala-OH condensing followed by removing Fmoc

A procedure is described for 200 mg of compound 614-SP as starting material. The procedure can be adjusted to a lower or higher amount of starting material.

200 mg of compound 614-SP (0.21 mmol / g resin), which is depicted at example C-08-1, is pre-swelled two times in DMF (10 mL / g resin) each for 15 min at RT. In case compound 614-SP is already a resin material in contact with DMF, then the pre-swelling can be omitted. 27.7 mg Fmoc-Ala-OH (0.084 mmol, 2.0 eq.) and 18.5 mg OxymaPure (0.130 mmol, 3.1 eq.) are dissolved in 2.0 mL DMF. To this solution, 16.9 µL DIC (0.109 mmol, 2.6 eq.) are added, the solution is stirred for 15 min at RT and added afterwards to pre-swelled respectively DMF-contacted compound 614-SP. After 20 min of reaction time at RT, an additional portion of 8.5 µL DIC (0.059 mmol, 1.3 eq.) are to the reaction mixture and stirred for typically 17 h at RT. The liquid parts are removed, and the resin material is washed three times alternating with DMF / IPA and three times with IPA (each washing step with 10 mL / g resin) to obtain compound 615-SP, which is depicted at example C-09-1.

Compound 615-SP is treated with 20 vol.% piperidine in DMF (10 mL / g resin) twice with a duration of 20 min for the first treatment and a duration of 60 min for the second treatment.

Afterwards, the resin material is washed three times alternating with DMF / IPA and three times with IPA (each washing step with 10 mL / g resin). The washed resin material can be employed at a further reaction without drying or is dried. A part of the washed resin material is dried for 24 h in high vacuum at RT to obtain compound 616-SP.

For analytical purposes, a small-scale test cleavage can be performed to obtain compound 704, which is depicted at example D-04-1.

### B) synthesis of lysine derivatives and some of their starting materials

### Example B-01-1: synthesis of compound 101

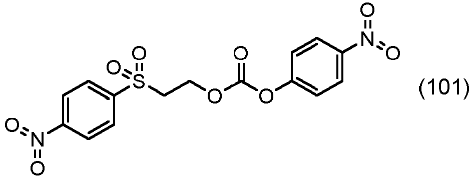

Compound 101 is obtainable for example from a condensing reaction of compound 102 (CAS-No. 21386-32-3) 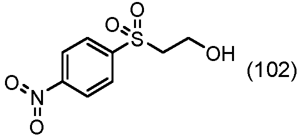 with compound 103 (CAS-No. 7693-46-1) 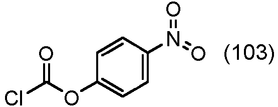 in analogy to molecule II in International Journal Peptide Research (1975) volume 7, p. 295-305.

### Example B-02-1: synthesis of compound 104

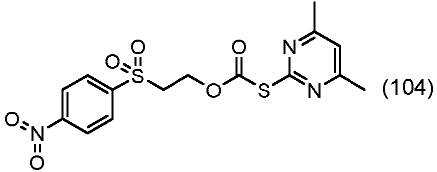

Compound 104 is obtainable for example by a condensing reaction of compound 105 (CAS-No. 102093-85-6) 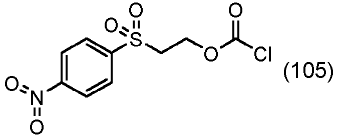 with compound 106 (CAS-No. 22325-27-5) 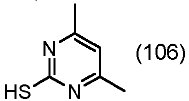 in analogy to the preparation of molecule 10 at US 3791830.

### Example B-02-2: synthesis of compound 104

Compound 104 is obtainable for example by a condensing reaction of compound 102 (CAS-No. 21386-32-3) 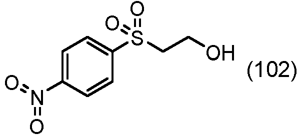 with compound 107 (CAS-No. 41840-26-0) 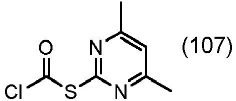 in analogy to example 5 of US 3936452.

### Example B-03-1: synthesis of compound 108

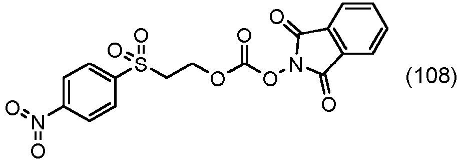

Compound 108 is obtainable for example by a condensing reaction of compound 105 (CAS-No. 102093-85-6) with N-hydroxyphthalimide in analogy to molecule IVb in International Journal Peptide Research (1975) volume 7, p. 295-305.

### Example B-04-1: synthesis of compound 109 in the form of a hydrochloride salt

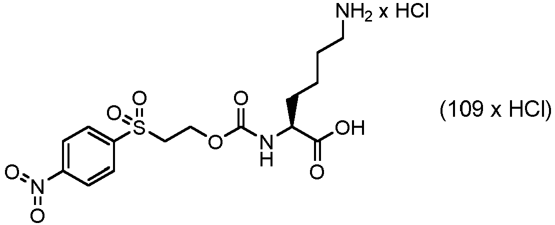

Compound 109 (WO 2002-098903 A1, table 1 / entry 19) in the form of a hydrochloride salt is obtainable for example by a deprotecting reaction of compound 110 (CAS-No. 160422-23-1) 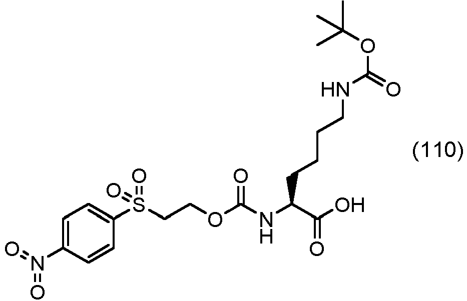 with HCl in dioxane.

### Example B-05-1: synthesis of compound 110

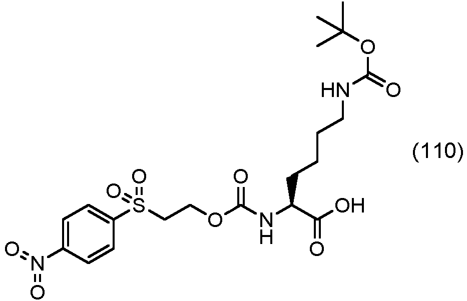

Compound 110 (CAS-No. 160422-23-1) is obtainable for example as described in example 3f in Tetrahedron Letters (1994), vol. 35, No. 42, p. 7821-7824, i.e. by a protecting reaction of compound 111 (CAS-No. 2418-95-3) 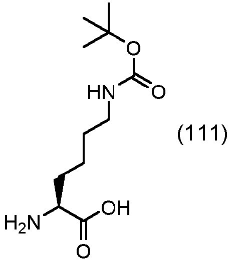 with chlorotrimethylsilane to obtain compound 112 as shown at scheme 1 in Tetrahedron Letters (1994), vol. 35, No. 42, p. 7821-7824, 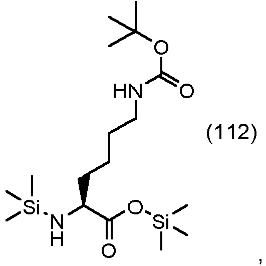 which is afterwards reacted with compound 105 (CAS-No. 102093-85-6).

### Example B-05-2: synthesis of compound 110

Compound 110 (CAS-No. 160422-23-1) is obtainable for example by a protecting reaction of compound 111 (CAS-No. 2418-95-3) with compound 101 in analogy to p. 302 / general procedure method B in International Journal Peptide Research (1975) volume 7, p. 295-305.

### Example B-05-3: synthesis of compound 110

Compound 110 (CAS-No. 160422-23-1) is obtainable for example by a protecting reaction of compound 111 (CAS-No. 2418-95-3) with compound 104 in analogy to example 26 of US 3936452.

### Example B-05-4: synthesis of compound 110

Compound 110 (CAS-No. 160422-23-1) is obtainable for example by a protecting reaction of compound 111 (CAS-No. 2418-95-3) with compound 108 in analogy to the solid phase synthesis at example 7 of WO 2004-065412.

### Example B-06-1: synthesis of compound 201

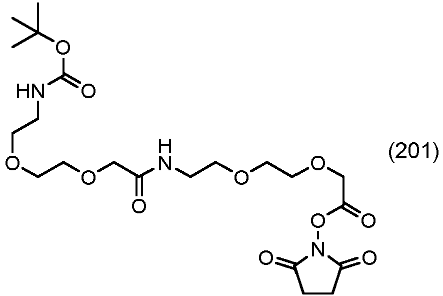

Compound 201 is obtainable for example by a condensing reaction of compound 202 (CAS-No. 1069067-08-8) 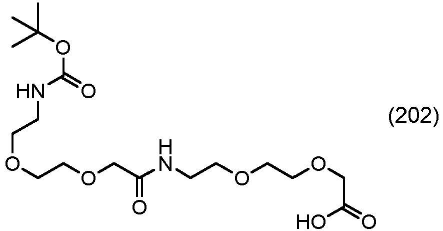 with N-hydroxysuccinimide supported by dicyclohexylcarbodiimide in analogy to example 3 c) of CN 113121627 A.

### Example B-07-1: synthesis of compound 301

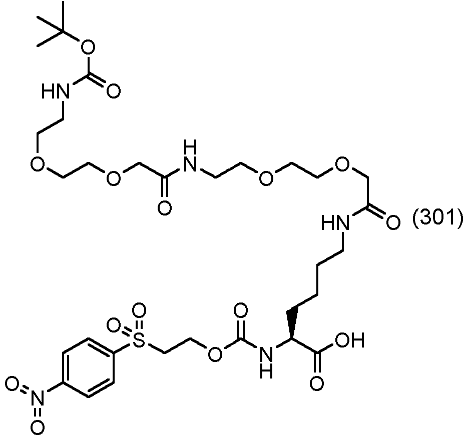

Compound 301 is obtainable for example by a protecting reaction of compound 109 with chlorotrimethylsilane in analogy to step 3b of EP 3819308 A1 to obtain compound 113 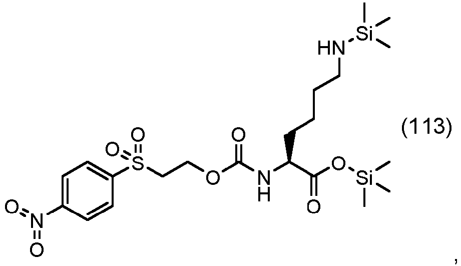 which is afterwards reacted with compound 201 in analogy to step 3b of EP 3819308 A1.

### Example B-08-1: synthesis of compound 302 in the form of a hydrochloride salt

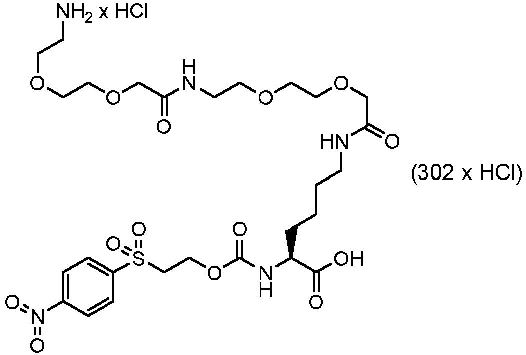

Compound 302 in the form of a hydrochloride salt is obtainable for example by a deprotecting reaction of compound 301 with HCl in dioxane.

### Example B-09-1: synthesis of compound 303 in the form of a hydrochloride salt

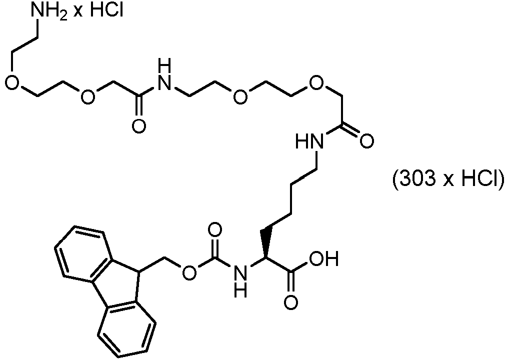

Compound 303 (CAS-No. 1662688-19-8) in the form of a hydrochloride salt is obtainable for example by a deprotecting reaction of compound 304 (CAS-No. 1662688-18-7) 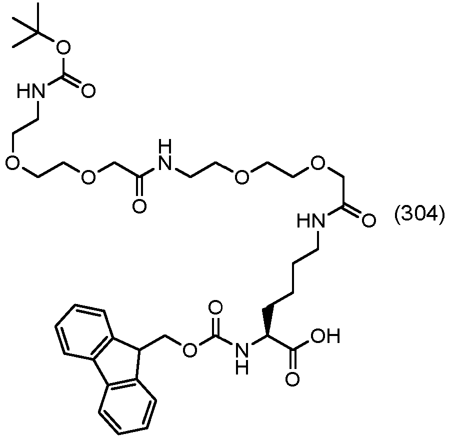 with HCl in dioxane.

### Example B-10-1: synthesis of compound 501

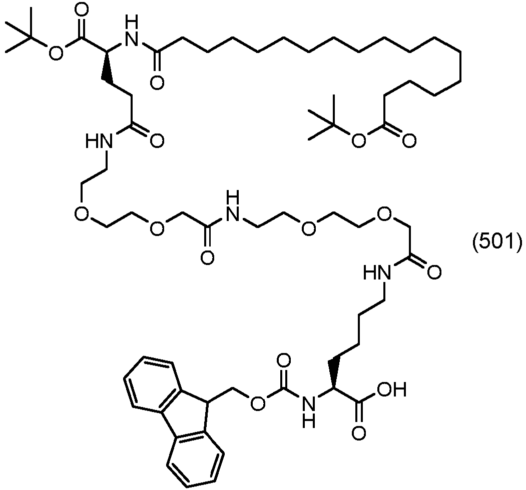

Compound 501 (CAS-No. 1662688-20-1) is obtainable for example as described in example 9 of CN 104356224 A, i.e. by a coupling reaction of compound 303 (CAS-No. 1662688-19-8) 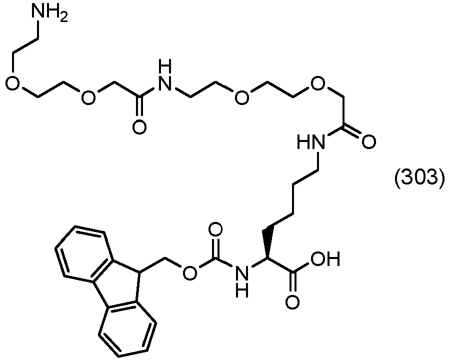 with compound 203 (CAS-No. 1188328-22-4)

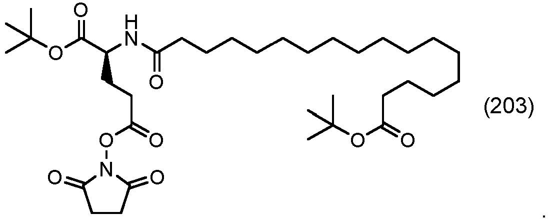

### Example B-10-2: synthesis of compound 501

Compound 501 (CAS-No. 1662688-20-1) is obtainable for example by a protecting reaction of compound 303 (CAS-No. 1662688-19-8) in the form of a hydrochloride salt with chlorotrimethylsilane to obtain compound 305 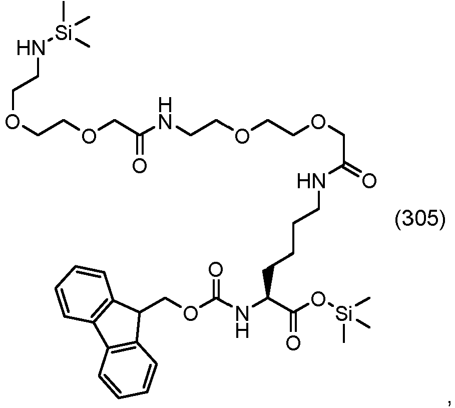 which is afterwards reacted with compound 203 (CAS-No. 1188328-22-4) in analogy to step 3b of EP 3819308 A1.

### Example B-10-3: synthesis of compound 501

Compound 501 (CAS-No. 1662688-20-1) is obtainable for example similarly as described in example 6 of WO 2021-205388 A2.

### Example B-11-1: synthesis of compound 502

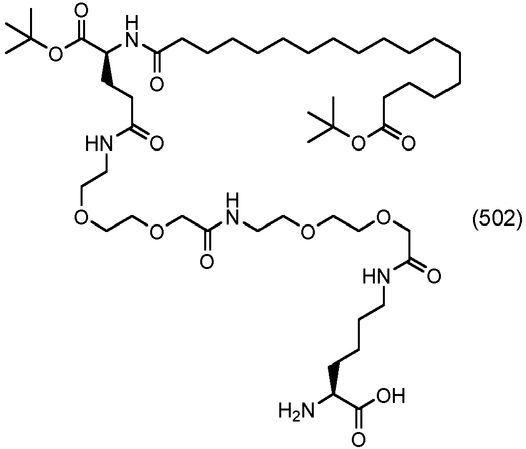

Compound 502 (CAS-No. 2682856-38-6) is obtainable for example as described in Organic Process Research and Development (2021), 25 (7), p. 1598-1611 for its molecule 10 in its scheme 3, i.e. by a coupling reaction of compound 306 (CAS-No. 1118767-16-0) 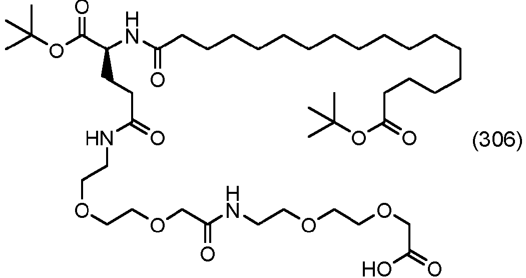 with compound 114 (CAS-No. 21512-99-2) 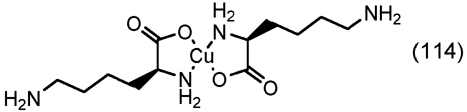 to obtain compound 503 (CAS-No. 2682856-37-5) 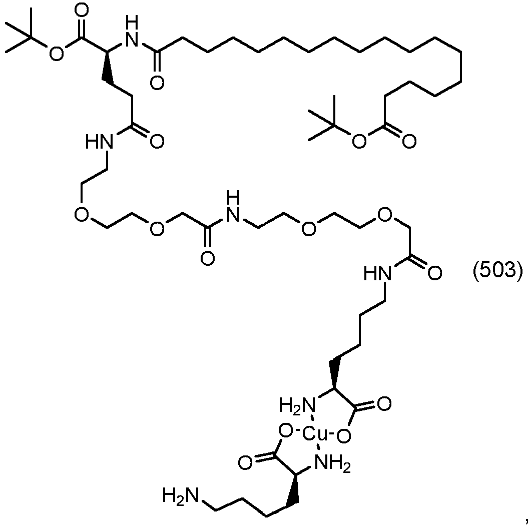 which is afterwards treated with tetrasodium ethylenediaminetetraacetate for dechelating.

### Example B-11-2: synthesis of compound 502

Compound 502 (CAS-No. 2682856-38-6) is obtainable for example by a deprotecting reaction of compound 501 (CAS-No. 1662688-20-1) with piperidine.

### Example B-11-3: synthesis of compound 502

100 g of compound 501 (84 mmol) are dissolved in 600 mL ACN at 40 °C and the clear solution is cooled to RT. To this solution, 33 mL piperidine are added and the resulting suspension stirred for 30 min, then additional 100 mL ACN are added and stirring continued for 1 h. The suspension is filtered over a suction filter and the filter cake washed with 5 x 200 mL and 2 x 300 mL ACN. The solid is dried under vacuum at 35 °C overnight. The resulting crude product (78 g) is dissolved in 600 mL ACN and 85 mL water at 50 °C. From the resulting solution, 200 mL are evaporated under reduced pressure using a rotary evaporator. To the formed emulsion, 400 mL ACN are added, and the same volume evaporated under reduced pressure. The addition / evaporation cycle is repeated twice more. The resulting suspension is then diluted with another 400 mL ACN and filtered over a vacuum filter. The filter cake is washed with 3 x 300 mL ACN and dried under vacuum at 35 °C for 15 h. 73.6 g (76 mmol, 90%) of compound 502 are obtained as a white solid.

HPLC method 1 with CAD detection: 98.7 area-%, retention time 6.19 min.

Gradient program at HPLC method 1

| time (min) | buffer A | buffer B |
|---|---|---|
| 0 | 70 | 30 |
| 3 | 28 | 72 |
| 8 | 0 | 100 |
| 12 | 0 | 100 |

### Example B-12-1: synthesis of compound 504

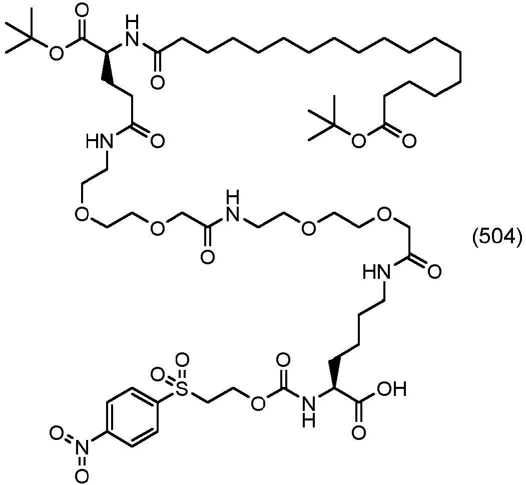

Compound 504 is obtainable for example by a protecting reaction of compound 502 (CAS-No. 2682856-38-6) with compound 105 (CAS-No. 102093-85-6) in analogy to example 1 of US 5616788.

### Example B-12-2: synthesis of compound 504

Compound 504 is obtainable for example by a protecting reaction of compound 502 (CAS-No. 2682856-38-6) with chlorotrimethylsilane to obtain compound 505 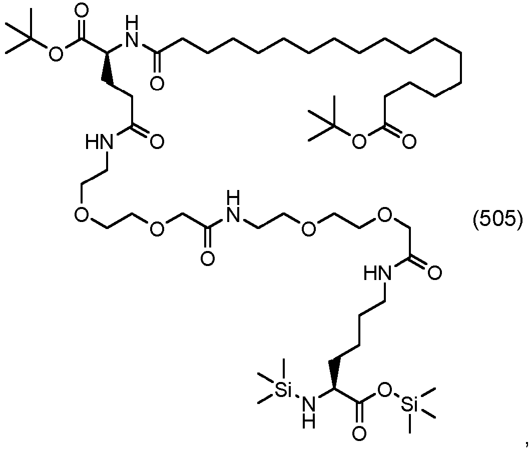 which is afterwards reacted with compound 105 (CAS-No. 102093-85-6) in analogy to example 3 of US 6165590.

### Example B-12-3: synthesis of compound 504

Compound 504 is obtainable for example by a protecting reaction of compound 502 (CAS-No. 2682856-38-6) with compound 115 (CAS-No. 122865-54-7) 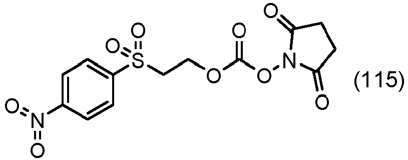 in analogy to the solid phase synthesis at example 7 of WO 2004-065412.

### Example B-12-4: synthesis of compound 504

Compound 504 is obtainable for example by a protecting reaction of compound 502 (CAS-No. 2682856-38-6) with compound 101 in analogy to p. 302 / general procedure method B in International Journal Peptide Research (1975) volume 7, p. 295-305.

### Example B-12-5: synthesis of compound 504

Compound 504 is obtainable for example by a protecting reaction of compound 502 (CAS-No. 2682856-38-6) with compound 104 in analogy to example 26 of US 3936452.

### Example B-12-6: synthesis of compound 504

Compound 504 is obtainable for example by a protecting reaction of compound 502 (CAS-No. 2682856-38-6) with compound 108 in analogy to the solid phase synthesis at example 7 of WO 2004-065412.

### Example B-12-7: synthesis of compound 504

Compound 504 is obtainable for example by a protecting reaction of compound 302 in the form of a hydrochloride salt with chlorotrimethylsilane in analogy to step 3b of EP 3819308 A1 to obtain compound 307 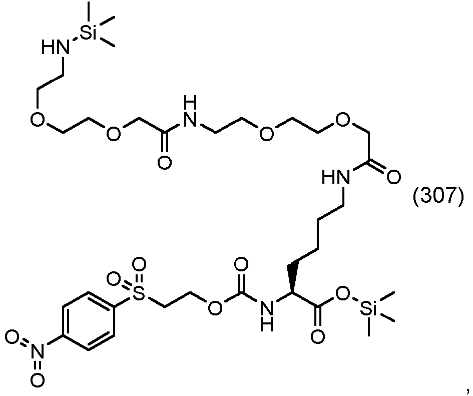 which is afterwards reacted with compound 203 (CAS-No. 1188328-22-4) in analogy to step 3b of EP 3819308 A1.

### Example B-12-8: synthesis of compound 504

19.5 g of compound 502 (20 mmol, 1.0 eq.) are suspended in 60 mL ACN and then 8.1 mL N-methyl-N-trimethylsilylacetamide (50 mmol, 2.5 eq.) are added. The mixture is heated to 40 °C to form a clear solution and then cooled to RT. 300 mL DMF and 1.6 ml pyridine (20 mmol, 1.0 eq.) are added to the solution, then 8.5 g of compound 101 (20 mmol, 1.0 eq.) is added over a period of 30 min. After 5 h, an additional 0.8 mL pyridine (10 mmol, 0.5 eq.) and 8.5 g of compound 101 (20 mmol, 1.0 eq.) are added and stirring is continued for 17 h. 120 mL of 0.25 N aqueous hydrochloric acid are added to the mixture and the solvent is evaporated under reduced pressure at 45 °C. The resulting thin oil is dissolved in 250 mL ethyl acetate and transferred to a separation funnel. 50 mL water are added and the pH is adjusted to 1-2 with 5.5 N aqueous hydrochloric acid. The phases are separated, the organic phase is washed with 3 x 50 mL water and then concentrated under reduced pressure to obtain a viscous oil. The purity of the viscous oil is 88.1 area% according HPLC method 1 and the below stated gradient program. The purity is even worse in case of a desirable extraction with an aqueous sodium hydrogencarbonate solution. The latter one has been left out. An assumption is a relatively high base sensitivity of the Nsc protecting group at the present molecule structure, which comes into play already with during pyridine contact and fortifies at the initially tried extraction with an aqueous sodium hydrogencarbonate solution. A purification of the viscous oil with a first preparative reverse phase HPLC (C4 column, 0.1% TFA - starting with a gradient of 3 vol.% ACN in water and going to 100 vol.% ACN) leads during the lyophilization of the collected and combined fractions to a partial decomposition. An assumption is that the TFA content is detrimental. The lyophilized material is purified with a second preparative HPLC without TFA addition (C4 column, no TFA - starting with a gradient of 3 vol.% ACN in water and going to 100 vol.% ACN). Lyophilization of the collected and combined fractions gives 2.1 g of compound 504 (1.7 mmol, yield 9%).

HPLC method 1 with CAD detection: 99.4 area%, retention time 5.14 min.

Gradient program at HPLC method 1

| time (min) | buffer A | buffer B |
|---|---|---|
| 0 | 40 | 60 |
| 5 | 0 | 100 |
| 7 | 0 | 100 |

### Example B-13-1: synthesis of compound 117

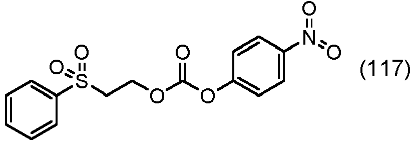

Compound 117 is obtainable for example from a condensing reaction of compound 116 (CAS-No. 20611-21-6) 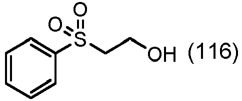 with compound 103 (CAS-No. 7693-46-1) 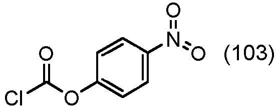 in analogy to molecule II in International Journal Peptide Research (1975) volume 7, p. 295-305.

### Example B-14-1: synthesis of compound 506

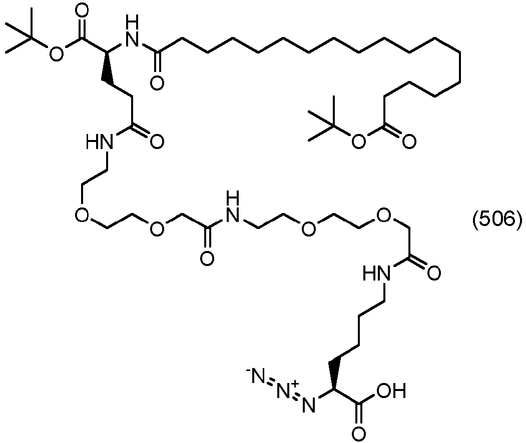

### Preparation of azidation reagent

23.35 g sodium azide (359 mmol, 10 eq.) are dissolved in 82 mL water and cooled in an ice bath. A solution of 12.1 mL trifluoromethanesulfonic anhydride (72 mmol, 2.0 eq.) in 135 mL cyclohexane is slowly added to the cooled solution over a period of 35 min under maintaining the internal temperature below 5 °C. The reaction mixture is stirred under cooling in the ice-bath for 2 h and then transferred to a separation funnel. The phases are separated. The aqueous phase is extracted with 2 x 120 mL cyclohexane. The combined organic phases are used as azidation reagent in the subsequent azide formation.

### Azide formation

35.0 g of compound 502 (36 mmol, 1.0 eq.) and 8.99 g potassium hydrogencarbonate (90 mmol, 2.5 eq.) are suspended in 65 mL water and 130 mL MeOH. The mixture is heated to 35 °C until remaining solids are dissolved. 90 mg CuSO₄ · 5 H₂O (0.4 mmol, 0.01 eq.) are added at 20 °C and then the solution of the azidation reagent is added over a period of 30 min to the mixture. The resulting emulsion is diluted with 56 mL dioxane and stirred for 20 h. 130 mL cyclohexane and 50 mL dioxane are added, the phases are separated in a separation funnel, and the aqueous phase is extracted with 3 x 240 mL diisopropyl ether and 3 x 250 mL MTBE. To the aqueous phase, 400 mL 5-methyl-tetrahydrofurane and 200 mL 5 % aqueous sodium hydrogencarbonate solution are added. The phases are separated, and the organic phase is extracted repeatedly with 5 % aqueous sodium hydrogencarbonate solution, diluted aqueous hydrochloric acid and water. The organic phase is evaporated to a white oil under reduced pressure, which is dissolved in 250 mL 5-methyl-tetrahydrofurane and again evaporated under reduced pressure. This operation is repeated four times and results in a yellow viscous oil that is dried under vacuum overnight. 29.5 g of compound 506 (29.5 mmol, yield 82%) are obtained as a yellow viscous oil.

HPLC method 1 with CAD detection: 94.8 area%, retention time 7.40 min.

Gradient program at HPLC method 1

| time (min) | buffer A | buffer B |
|---|---|---|
| 0 | 70 | 30 |
| 3 | 28 | 72 |
| 8 | 0 | 100 |
| 12 | 0 | 100 |

### Example B-15-1: synthesis of compound 507

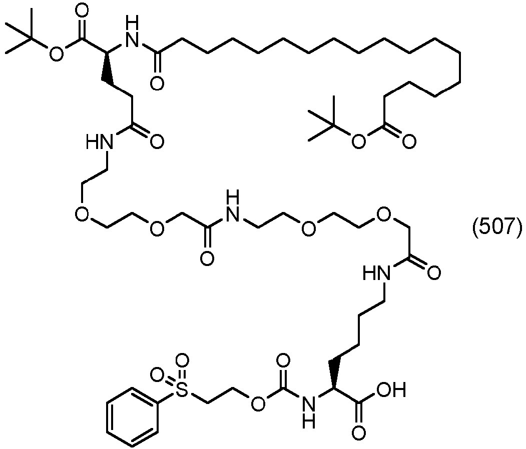

Compound 507 is prepared in analogy to compound 508 at example B-16-1 from 19.5 g of compound 502, 9.6 mL N-methyl-N-trimethylsilylacetamide (60 mmol, 3.0 eq.) and 8.3 g of compound 117 (20 mmol, 1.0 eq.). 22.8 g of compound 507 (19.2 mmol, yield 96%) are obtained as a viscous oil.

HPLC method 1 with CAD detection: 99.3 area%, retention time 7.11 min.

Gradient program at HPLC method 1

| time (min) | buffer A | buffer B |
|---|---|---|
| 0 | 80 | 20 |
| 5 | 0 | 100 |
| 10 | 0 | 100 |

### Example B-16-1: synthesis of compound 508

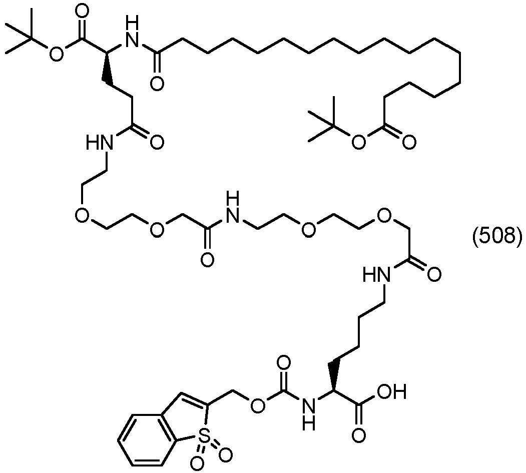

15.4 g of compound 502 (15.8 mmol, 1.0 eq.) is suspended in 47 mL ACN and then 6.3 mL N-methyl-N-trimethylsilylacetamide (39.5 mmol, 2.5 eq.) are added. The mixture is heated to 40 °C for 2 h to form a clear solution and then cooled to RT. 107 mL ACN and 2.5 mL pyridine (36.3 mmol, 2.3 eq.) are added to the solution and the mixture is cooled to 0 °C. A solution of 4.71 g 1,1-dioxobenzo[b]thiophene-2-yl-methyloxycarbonyl chloride (CAS-No. 135204-19-2, 18.2 mmol, 1.15 eq.) in 178 mL ACN are added over a period of 30 min under maintaining the internal temperature at 0 °C. Afterwards, additional 0.4 mL pyridine (5 mmol, 0.3 eq.) and a solution of 0.6 g 1,1-dioxobenzo[b]thiophene-2-yl-methyloxycarbonyl chloride (CAS-No. 135204-19-2, 2.3 mmol, 0.15 eq.) in 26 mL ACN are added and stirring is continued at 0 °C for 15 h. The reaction mixture is then transferred to a separation funnel, 1400 mL MTBE and 320 mL water are added and acidified with 1 N aqueous hydrochloric acid. The phases are separated and the organic phase is washed 3 times with 350 mL dilute aqueous hydrochloric acid and with 320 mL water. The organic phase is concentrated under reduced pressure to give the crude product as a viscous oil. The crude product is dissolved in a mixture of 170 mL water, 170 mL ACN and 170 mL diisopropyl ether at 35 °C and transferred to a separation funnel. 31 mL of 10 % aqueous sodium hydrogencarbonate solution are added and the phases are separated. The aqueous phase is washed with 340 mL of a mixture of diisopropyl ether and ACN, the phases are separated, the aqueous phase is acidified by the addition of 5.5 N aqueous hydrochloric acid and then extracted with 1200 mL MTBE. The phases are separated and the organic phase is washed with 250 mL dilute aqueous hydrochloric acid and 250 mL water. The organic phase is evaporated under reduced pressure and the resulting oil dried under vacuum to give 10.6 g of compound 508 (8.8 mmol, yield 56%) as a very viscous oil.

HPLC method 1 with CAD detection: 97.7 area%, retention time 7.16 min.

Gradient program at HPLC method 1

| time (min) | buffer A | buffer B |
|---|---|---|
| 0 | 80 | 20 |
| 5 | 0 | 100 |
| 10 | 0 | 100 |

### C) synthesis of solid-phase conjugated compounds

### Example C-01-1: synthesis of solid-phase conjugated compound 601-SP

Fmoc-Glu(OtBu)-Phe-Ile-Ala-Trp(Boc)-Leu-Val-Arg(Pbf)-Gly-Arg(Pbf)-Gly-[2-
chlorotritylamidomethyl resin] (SEQ ID NO:13)

(601-SP)

Compound 601-SP is obtainable for example by a solid phase synthesis starting with compound 602-SP

H-Gly-[2-chlorotritylamidomethyl resin] (602-SP)

A coupling cycle of compound 602-SP with Fmoc-Arg(Pbf)-OH is followed by coupling cycles of Fmoc-Gly-OH, Fmoc-Arg(Pbf)-OH, Fmoc-Val-OH, Fmoc-Leu-OH, Fmoc-Trp(Boc)-OH, Fmoc-Ala-OH, Fmoc-lle-OH, Fmoc-Phe-OH and Fmoc-Glu(OtBu)-OH.

A coupling cycle comprises a deprotecting reaction of the N-terminal alpha amino group of the solid-phase conjugated intermediate unless the N-terminal alpha amino group is already unprotected. The deprotecting reaction for Fmoc is conducted with 20 vol.% piperidine in DMF or when desired with 20 vol.% piperidine in NMP in a typical time range of 0.5 to 4.0 hours. The coupling cycle comprises also a coupling reaction of the solid-phase conjugated intermediate with its unprotected N-terminal alpha amino group, i.e. an acylation of the unprotected N-terminal alpha amino group by an activated alpha carboxylic acid group of respective amino acid derivative, which is activated by either DIC / OxymaPure, TBTU / DIPEA or DEPBT / DIPEA. A typical time range of the acylating reaction is 1.5 to 24 hours. The coupling cycle comprises also one or more washings of the solid phase after the acylating reaction. DMF or IPA is used as a solvent for the one or more washings. The coupling cycle comprises also optionally an acetylating reaction with acetic anhydride after the acylating reaction. The coupling cycle comprises also optionally one or more washings after the acetylating reaction with acetic anhydride. DMF or IPA is used as a solvent for the optional one or more washings after the acetylating reaction with acetic anhydride.

### Example C-02-1: synthesis of solid-phase conjugated compound 603-SP

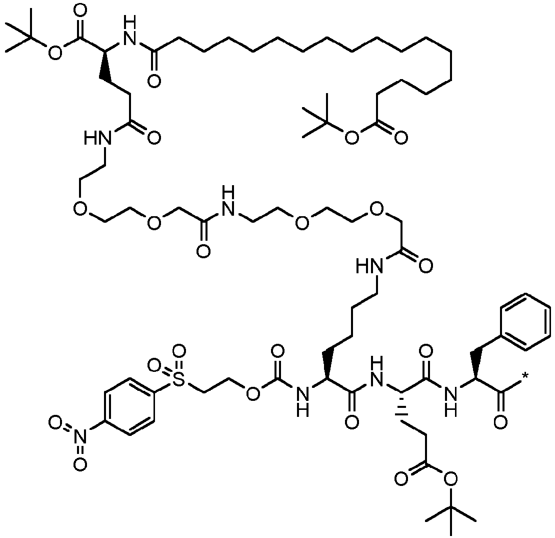

*-Ile-Ala-Trp(Boc)-Leu-Val-Arg(Pbf)-Gly-Arg(Pbf)-Gly-[2-chlorotritylamidomethyl resin] (SEQ ID NO:26) (603-SP)

Compound 603-SP (= Nsc-Lys(tBuO-CO-(CH₂)₁₆-CO-gamma-Glu(OtBu)-2-[2-(2-aminoethoxy)-ethoxyacetyl]-2-[2-(2-aminoethoxy)ethoxy]acetyl])-Glu(OtBu)-Phe-Ile-Ala-Trp(Boc)-Leu-Val-Arg(Pbf)-Gly-Arg(Pbf)-Gly-[2-chlorotritylamidomethyl resin]) (SEQ ID NO:26) is obtainable for example by a coupling cycle with compound 504 applied to compound 601-SP, i.e. a deprotecting reaction of compound 601-SP with 20 vol.% piperidine in DMF to obtain compound 604-SP

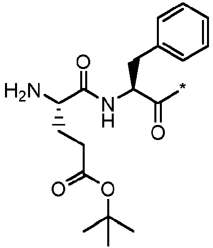

*-Ile-Ala-Trp(Boc)-Leu-Val-Arg(Pbf)-Gly-Arg(Pbf)-Gly-[2-chlorotritylamidomethyl resin] (SEQ ID NO:14) (604-SP),

which is afterwards reacted with compound 504 and DIC / OxymaPure.

As used herein, the residue tBu-O-CO-(CH₂)₁₆-CO-gamma-Glu-2-[2-(2-aminoethoxy)-ethoxyacetyl]-2-[2-(2-aminoethoxy)ethoxyacetyl]) may also be designated as O-tert-butyl-17-carboxy-heptadecacarbonyl-gamma-Glu-2-[2-(2-aminoethoxy)ethoxyacetyl]-2-[2-(2-aminoethoxy)ethoxyacetyl].

As used herein, the residue HO-CO-(CH₂)₁₆-CO-gamma-Glu-2-[2-(2-aminoethoxy)ethoxyacetyl]-2-[2-(2-aminoethoxy)ethoxyacetyl]) may also be designated as 17-carboxy-heptadecacarbonyl-gamma-Glu-2-[2-(2-aminoethoxy)ethoxyacetyl]-2-[2-(2-aminoethoxy)ethoxyacetyl].

### Example C-03-1: synthesis of solid-phase conjugated compound 605-SP

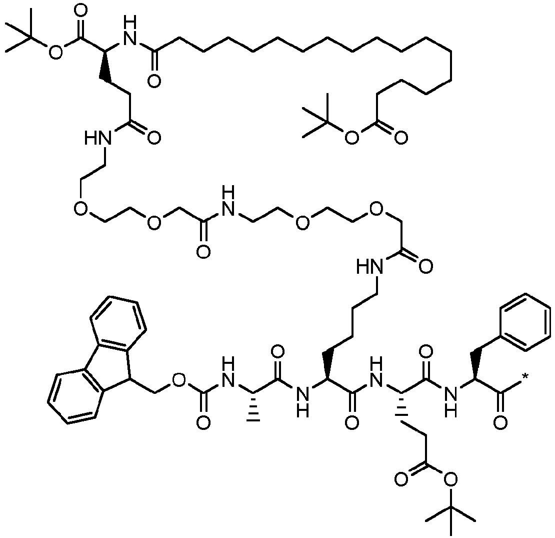

*-Ile-Ala-Trp(Boc)-Leu-Val-Arg(Pbf)-Gly-Arg(Pbf)-Gly-[2-chlorotritylamidomethyl resin] (SEQ ID NO:15) (605-SP)

Compound 605-SP (= Fmoc-Ala-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotritylamidomethyl resin]) (SEQ ID NO:15)

is obtainable for example by a coupling cycle with Fmoc-Ala-OH applied to compound 603-SP, i.e. a deprotecting reaction of compound 603-SP with 20 vol.% piperidine in DMF to obtain compound 606-SP

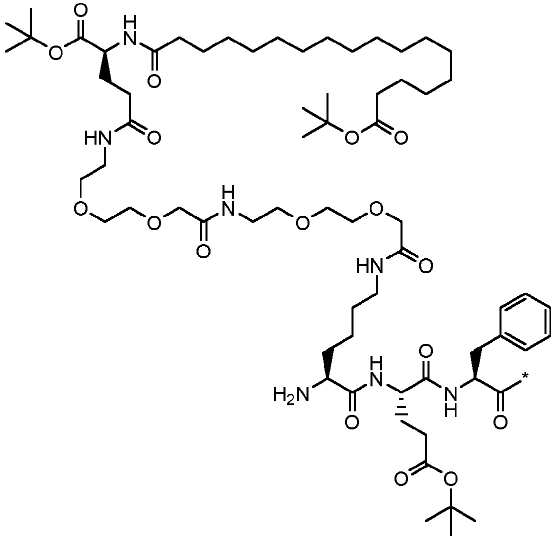

*-Ile-Ala-Trp(Boc)-Leu-Val-Arg(Pbf)-Gly-Arg(Pbf)-Gly-[2-chlorotritylamidomethyl resin] (SEQ ID NO:28) (606-SP),

which is afterwards reacted with Fmoc-Ala-OH and DIC / OxymaPure.

### Example C-04-1: synthesis of solid-phase conjugated compound 607-SP

Boc-His(Trt)-Aib-Glu(OtBu)-Gly-Thr(tBu)-Phe-Thr(Psi(Me,Me)pro)-Ser(tBu)-Asp(OMpe)-Val-Ser(tBu)-Ser(tBu)-Tyr(tBu)-Leu-Glu(OtBu)-Gly-Gln(Trt)-Ala-Ala-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorofritylamidomethyl resin] (SEQ ID NO:16) (607-SP)

Compound 607-SP (= a protected and solid-phase conjugated derivative of Semaglutide (CAS-No. 910463-68-2)) is obtainable for example by several coupling cycles starting at a coupling cycle with Fmoc-Ala-OH applied to compound 605-SP, i.e. a deprotecting reaction of compound 605-SP with 20 vol.% piperidine in DMF to obtain compound 608-SP

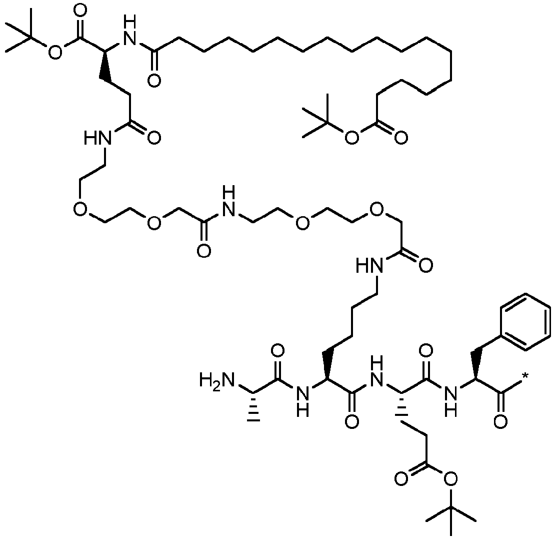

*-Ile-Ala-Trp(Boc)-Leu-Val-Arg(Pbf)-Gly-Arg(Pbf)-Gly-[2-chlorotritylamidomethyl resin] (SEQ ID NO:29) (608-SP),

which is afterwards reacted with Fmoc-Ala-OH and DIC /OxymaPure.

Coupling cycles with and in the sequence of Fmoc-Gln(Trt)-OH, Fmoc-Gly-OH, Fmoc-Glu(OtBu)-OH, Fmoc-Leu-OH, Fmoc-Tyr(tBu)-OH, Fmoc-Ser(tBu)-OH, Fmoc-Ser(tBu)-OH, Fmoc-Val-OH, Fmoc-Asp(OMpe)-OH, Fmoc-Ser(tBu)-OH, Fmoc-Phe-Thr(Psi(Me,Me))pro)-OH, Fmoc-Thr(tBu)-OH, Fmoc-Gly-OH, Fmoc-Glu(OtBu)-OH and Boc-His(1-Trt)-Aib-OH are following.

The coupling cycles are conducted as described in example C-01-1.

### Example C-05-1: synthesis of solid-phase conjugated compound 611-SP

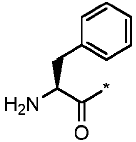

*-Ile-Ala-Trp(Boc)-Leu-Val-Arg(Pbf)-Gly-Arg(Pbf)-Gly-[2-chlorotrityl resin] (SEQ ID NO:17) (611-SP)

Compound 611-SP (= H-Phe-Ile-Ala-Trp(Boc)-Leu-Val-Arg(Pbf)-Gly-Arg(Pbf)-Gly-2-chlorotrityl resin) (SEQ ID NO:17) is synthesized at a 30 mmol scale using an automated synthesizer (Sonata, Gyros Protein Technologies). Compound 610-SP (= H-Gly-2-chlorotrityl resin, 66.7 g, 0.45 mmol / g resin, 30 mmol), i.e. as depicted by

H-Gly-[2-chlorotrityl resin] (610-SP),

is pre-swelled in two cycles each with 30 min in DMF (10 mL / g resin). The first Fmoc-protected amino acid, i.e. Fmoc-Arg(Pbf)-OH), is pre-activated for 3 min with TBTU / DIPEA (1.8 eq./3.0 eq.), added to the resin and the reaction mixture is stirred for 2 h at RT. The following Fmoc-protected amino acid derivatives (2.0 eq.) are pre-activated for 15 min using DIC / OxymaPure (2.6 eq. / 3.1 eq., Fmoc-Arg(Pbf)-OH is pre-activated for 5 min) and added to the resin. After 20 min reaction time, additional DIC (1.3 eq.) is added to the reaction mixture (total reaction volume is 670 mL) and the reaction of the coupling step is continued. The total reaction time of the couplings steps are 1.5 h for Fmoc-Gly-OH, Fmoc-Val-OH, Fmoc-Leu-OH, Fmoc-Trp(Boc)-OH, Fmoc-Ala-OH, 2 h for Fmoc-Arg(Pbf)-OH and 5 h for Fmoc-lle-OH and Fmoc-Phe-OH. At the end of each coupling step, the resin is drained and an acetylation reaction using a solution 0.05 M acetic acid anhydride / 17 mM 2,4,6-collidine / 1.6 mM OxymaPure (670 mL) in DMF is conducted. This acetylation serves to acetylate remaining unreacted amino-groups and avoids their reaction in a following coupling step (so-called capping). Afterwards, the resin is then filtered and washed with DMF and Fmoc deprotection is carried out using twice or three times 670 mL of a solution of 20 vol.% piperidine in DMF (5 min duration for a first treatment and 10 min duration for a second treatment prior to Fmoc-Trp(Boc)-OH and Fmoc-Ala-OH coupling; 5 min duration for a first treatment, 10 min duration for a second treatment and 15 min duration for a third treatment prior to Fmoc-Gly-OH, Fmoc-Arg(Pbf)-OH, Fmoc-Val-OH, Fmoc-Leu-OH, Fmoc-lle-OH and Fmoc-Phe-OH). After the Fmoc deprotection, the resin is washed with 670 mL DMF, 670 mL IPA and two times 670 mL DMF. After coupling of Fmoc-Phe-OH, acetylation and Fmoc deprotection, the resin material is washed three times alternatingly with 670 mL DMF, 670 mL IPA and three times with 670 mL IPA. The washed resin material is dried under vacuum at RT for three days to yield 132 g of dried compound 611-SP.

For analytical purposes, a small-scale test cleavage from compound 611-SP is conducted. This is described at example D-06-1.

### Example C-06-1: synthesis of solid-phase conjugated compound 612-SP

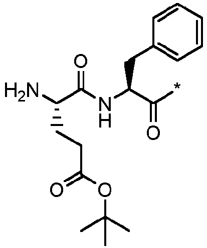

*-Ile-Ala-Trp(Boc)-Leu-Val-Arg(Pbf)-Gly-Arg(Pbf)-Gly-[2-chlorotrityl resin] (SEQ ID NO:21) (612-SP)

10.00 g of compound 611-SP (2.2 mmol, 0.22 mmol / g) are pre-swelled two times in 100 mL DMF (10 mL / g resin) each for 15 min at RT. 1.95 g Fmoc-Glu(OtBu)-OH (4.40 mmol, 2.0 eq.) and 0.969 g OxymaPure (6.82 mmol, 3.1 eq.) are dissolved in 100 mL DMF (10 mL / g resin). To this solution, 886 µL DIC(5.72 mmol, 2.6 eq.) are added, the solution is stirred for 15 min at RT and the solution is then added to the pre-swelled compound 611-SP. After 20 min of reaction time at RT, an additional portion of 443 µL DIC (2.86 mmol, 1.3 eq.) are added to the reaction mixture and stirred for 1.5 h at RT. After removing the liquid parts from the reaction mixture by drainage, an additional coupling step with the Fmoc-Glu(OtBu)-OH is performed (so-called recoupling). Therefore, 1.95 g Fmoc-Glu(OtBu)-OH (4.40 mmol, 2.0 eq.) and 0.969 g OxymaPure (6.82 mmol, 3.1 eq.) are dissolved in 100 mL DMF (10 mL DMF / g resin). To this solution, 886 µL DIC (5.72 mmol, 2.6 eq.) are added and the solution is stirred for 15 min at RT and is afterwards added afterwards to the reaction mixture. After 20 min of reaction time at RT, an additional portion of 443 µL DIC (2.86 mmol, 1.3 eq.) are added to the reaction mixture and stirred for 16 h at RT. The liquid parts are removed, the remaining resin material is washed with 20 mL DMF and is then treated for Fmoc-deprotection with 20 vol.% piperidine in DMF (10 mL / g resin) three times with a duration of 5 min for a first treatment, of 10 min for a second treatment and of 60 min for a third treatment. After this Fmoc-deprotection step, the resin material is washed three times with alternatingly 670 mL DMF and 670 mL IPA, then finally with three times 100 mL IPA and then two times 670 mL DMF. The washed resin material is dried under vacuum at RT for three days to yield 11.0 g of compound 612-SP.

For analytical purposes, a small-scale test cleavage is performed. This is described at example D-07-1.

### Example C-07-1: synthesis of solid-phase conjugated compound 613-SP

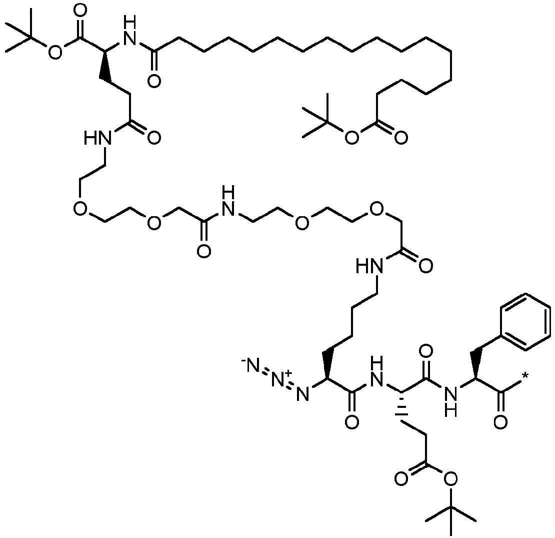

*-Ile-Ala-Trp(Boc)-Leu-Val-Arg(Pbf)-Gly-Arg(Pbf)-Gly-[2-chlorotrityl resin] (SEQ ID NO:18) (613-SP)

2.00 g of compound 611-SP (0.88 mmol, 0.44 mmol/g) is pre-swelled two times in 20 mL DMF (10 mL / g resin) each for 15 min at RT. 0.781 g Fmoc-Glu(OtBu)-OH (1.76 mmol, 2.0 eq.) and 0.388 g OxymaPure (2.728 mmol, 3.1 eq.) are dissolved in 20 mL DMF (10 mL / g resin). To this solution, 354 µL DIC (2.288 mmol, 2.6 eq.) are added and the solution is stirred for 15 min at RT and added afterwards to the solution is then added to the pre-swelled compound 611-SP. After 20 min of reaction time at RT, an additional portion of 177 µL DIC (1.144 mmol, 1.3 eq.) are added to the reaction mixture and stirred for 5 h at RT. After removing the liquid parts from the reaction mixture by drainage, an additional coupling step with the Fmoc-Glu(OtBu)-OH is performed (so-called recoupling). Therefore, 0.781 g Fmoc-Glu(OtBu)-OH (1.76 mmol, 2.0 eq.) and 0.388 g OxymaPure (2.728 mmol, 3.1 eq.) are dissolved in 20 mL DMF (10 mL / g resin). To this solution, 354 µL DIC (2.288 mmol, 2.6 eq.) are added, the solution is stirred for 15 min at RT and added afterwards to the reaction mixture. After 20 min of reaction time at RT, 177 µL DIC (1.144 mmol, 1.3 eq.) are added to the reaction mixture and stirred for 15 h at RT. The liquid parts are removed, the resin material is washed with 20 mL DMF and is treated with 20 vol.% piperidine in DMF (10 mL / g resin) in three times with a duration of 5 min for a first treatment, of 10 min for a second treatment and of 60 min for a third treatment. Afterwards, 1.76 g of compound 506 (1.760 mmol, 2.0 eq.) and 0.388 g OxymaPure (2.728 mmol, 3.1 eq.) are dissolved in 20 mL DMF (10 mL / g resin). To this solution, 354 µL DIC (2.288 mmol, 2.6 eq.) are added and the solution is stirred for 15 min at RT and added afterwards to the reaction mixture. After 20 min of reaction time at RT, an additional portion of 177 µL DIC (177 µL, 1.144 mmol, 1.3 eq.) are added to the reaction mixture and stirred for 22 h at RT. The liquid parts are removed, the resin material is washed three times alternating with DMF / IPA and three times with IPA (each washing step with 10 mL / g resin). The washed resin material is dried 24 h in high vacuum at RT to yield 2.22 g of the compound 613-SP (= N₃-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:18).

For analytical purposes, a small-scale test cleavage is performed. This is described at example D-08-1.

### Example C-08-1: synthesis of solid-phase conjugated compound 614-SP

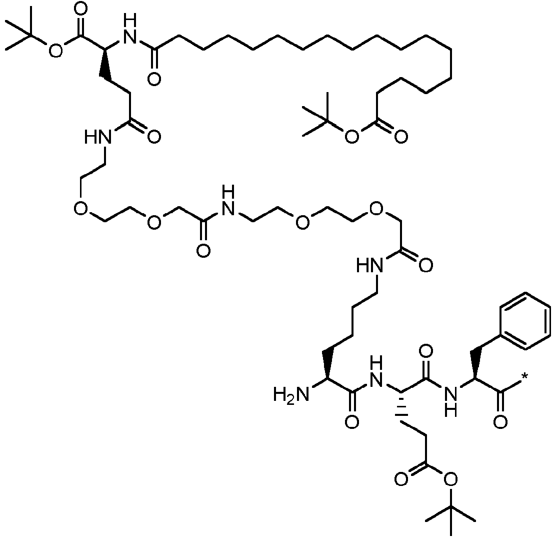

*-Ile-Ala-Trp(Boc)-Leu-Val-Arg(Pbf)-Gly-Arg(Pbf)-Gly-[2-chlorotrityl resin] (SEQ ID NO:19) (614-SP)

100 mg of compound 613-SP (0.21 mmol / g, 0.022 mmol) is pre-swelled two times in DMF (10 mL / g resin) each for 15 min at RT. 31.5 mg tris(2-carboxyethyl)phosphine (= TCEP, 0.11 mmol, 5.0 eq.) are dissolved in 250 µL of a 10 vol.% aqueous DMF solution (2.5 mL / g resin), 18.8 µL DIPEA (0.11 mmol, 5.0 eq.) are added and the solution was added to the pre-swelled compound 613-SP. The reaction mixture is then stirred for 3 h at RT and additional 16 h at 50 °C. The liquid parts are removed, the resin material is washed three times with 10 vol.% aqueous DMF, three times alternating with DMF / IPA and three times with IPA (each washing step with 10 mL / g resin). A part of the resin material is further processed at example C-09-1. without drying. A part of the resin material is dried 24 h in high vacuum at RT to yield compound 614-SP (= H-Lys(tBuO-CO-(CH₂)₁₆-CO-gamma-Glu(OtBu)-2-[2-(2-aminoethoxy)ethoxy]acetyl-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:19).

For analytical purposes, a small-scale test cleavage is performed. This is described at example D-02-2.

### Example C-08-2: synthesis of solid-phase conjugated compound 614-SP

100 mg of compound 617-SP (0.022 mmol, 0.21 mmol / g resin) is pre-swelled two times in DMF (10 mL / g resin) each for 10 min RT. The pre-swelled compound 617-SP is treated with 20 vol.% piperidine in DMF solution (10 mL / g resin) in twice with a duration of 20 min for a first treatment and a duration of 60 min for a second treatment. Afterwards, the resin material is washed three times alternating with DMF / IPA and three times with IPA (each washing step with 10 mL / g resin). The washed resin-material is dried 24 h in high vacuum at RT to obtain compound 614-SP.

For analytical purposes, a small-scale test cleavage is performed. This is described at example D-02-3.

### Example C-08-3: synthesis of solid-phase conjugated compound 614-SP

200 mg of compound 618-SP (0.21 mmol / g resin, 0.042 mmol) is pre-swelled two times in DMF (10 mL / g resin) each for 10 min at RT. The pre-swelled resin material is treated with 2 vol.% DBU in a 20 vol.% piperidine in DMF solution (10 mL / g resin) twice with a duration of 20 min for a first treatment and a duration of 60 min for a second treatment. Afterwards, the resin material is washed three times alternating with DMF / IPA and three times with IPA (each washing step with 10 mL / g resin). A part of the washed peptide material is further processed without drying according to example C-09-3. A part of the washed peptide material is dried 24 h in high vacuum at RT to obtain compound 614-SP.

For analytical purposes, a small-scale test cleavage is performed. This is described at example D-02-4.

### Example C-08-4: synthesis of solid-phase conjugated compound 614-SP

200 mg of compound 619-SP (0.21 mmol / g resin, 0.042 mmol) is pre-swelled two times in DMF (10 mL / g resin) each for 10 min at RT. The pre-swelled resin material is treated with 2 vol.% DBU in a 20 vol.% piperidine in DMF solution (10 mL / g resin) twice with a duration of 20 min for a first treatment and a duration of 60 min for a second treatment. Afterwards, the resin material is washed three times alternating with DMF / IPA and three times with IPA (each washing step with 10 mL / g resin). A part of the washed peptide material is further processed without drying according to example C-09-4. A part of the washed peptide material is dried 24 h in high vacuum at RT to obtain compound 614-SP.

For analytical purposes, a small-scale test cleavage is performed. This is described at example D-02-5.

### Example C-08-5: synthesis of solid-phase conjugated compound 614-SP

200 mg of compound 620-SP (0.21 mmol / g resin, 0.042 mmol) is pre-swelled two times in DMF (10 mL / g resin) each for 10 min at RT. The pre-swelled resin material is treated with 20 vol.% piperidine in DMF solution (10 mL / g resin) twice with a duration of 20 min for a first treatment and a duration of 60 min for a second treatment. Afterwards, the resin material is washed three times alternating with DMF / IPA and three times with IPA (each washing step with 10 mL / g resin). A part of the washed peptide material is further processed without drying according to example C-09-5. A part of the washed peptide material is dried 24 h in high vacuum at RT to obtain compound 614-SP.

For analytical purposes, a small-scale test cleavage is performed. This is described at example D-02-6.

### Example C-09-1: synthesis of solid-phase conjugated compound 616-SP

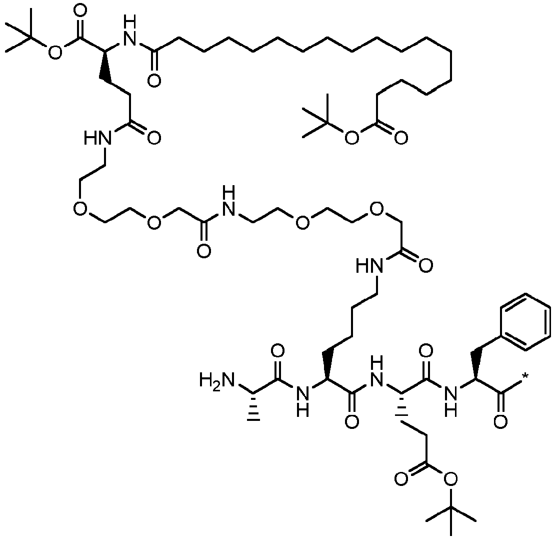

*-Ile-Ala-Trp(Boc)-Leu-Val-Arg(Pbf)-Gly-Arg(Pbf)-Gly-[2-chlorotrityl resin] (SEQ ID NO:27) (616-SP)

Compound 616-SP (= H-Ala-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:27).is synthesized from compound 614-SP obtained at example C-08-1 according to general procedure 3 via the Fmoc-protected intermediate compound 615-SP (= Fmoc-Ala-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:20).

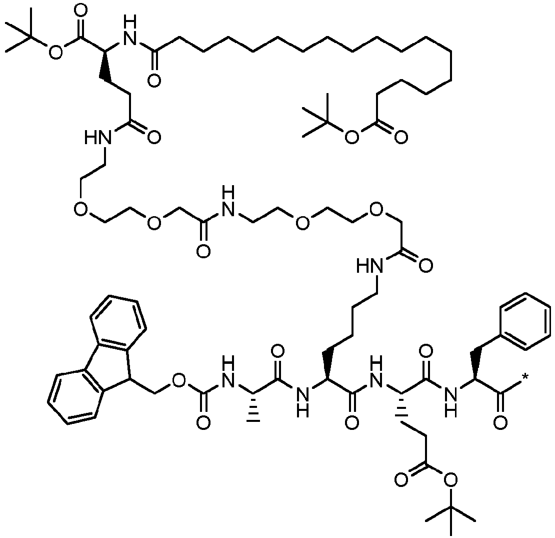

*-Ile-Ala-Trp(Boc)-Leu-Val-Arg(Pbf)-Gly-Arg(Pbf)-Gly-[2-chlorotrityl resin] (SEQ ID NO:20) (615-SP).

For analytical purposes, a small-scale test cleavage is performed. This is described at example D-04-2.

### Example C-09-2: synthesis of solid-phase conjugated compound 616-SP

Compound 616-SP is synthesized from 100 mg of compound 614-SP (0.022 mmol, 0.22 mmol / g resin) obtained at example C-08-2 according to general procedure 3 via the Fmoc-protected intermediate compound 615-SP.

For analytical purposes, a small-scale test cleavage is performed. This is described at example D-04-3.

### Example C-09-3: synthesis of solid-phase conjugated compound 616-SP

Compound 616-SP is synthesized from 100 mg of compound 614-SP (0.022 mmol, 0.22 mmol / g resin) obtained at example C-08-3 according to general procedure 3 via the Fmoc-protected intermediate compound 615-SP.

For analytical purposes, a small-scale test cleavage is performed. This is described at example D-04-4.

### Example C-09-4: synthesis of solid-phase conjugated compound 616-SP

Compound 616-SP is synthesized from 100 mg of compound 614-SP (0.022 mmol, 0.22 mmol / g resin) obtained at example C-08-4 according to general procedure 3 via the Fmoc-protected intermediate compound 615-SP.

For analytical purposes, a small-scale test cleavage is performed. This is described at example D-04-5.

### Example C-09-5: synthesis of solid-phase conjugated compound 616-SP

Compound 616-SP is synthesized from 100 mg of compound 614-SP (0.022 mmol, 0.22 mmol / g resin) obtained at example C-08-5 according to general procedure 3 via the Fmoc-protected intermediate compound 615-SP.

For analytical purposes, a small-scale test cleavage is performed. This is described at example D-04-6.

### Example C-10-1: synthesis of solid-phase conjugated compound 617-SP

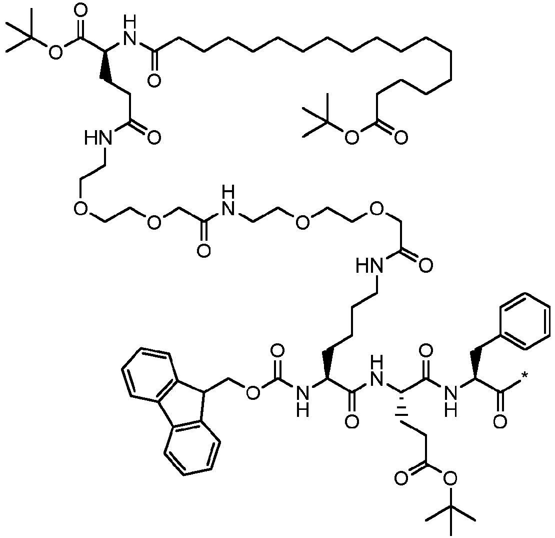

*-Ile-Ala-Trp(Boc)-Leu-Val-Arg(Pbf)-Gly-Arg(Pbf)-Gly-[2-chlorotrityl resin] (SEQ ID NO:22) (617-SP)

200 mg of compound 612-SP (0.044 mmol, 0.22 mmol / g resin) and 105 mg compound 501 (0.088 mmol, 2.0 eq.) are reacted according to general procedure 1 to obtain compound 617-SP (= Fmoc-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:22).

For analytical purposes, a small-scale test cleavage is performed. This is described at example D-09-1.

### Example C-11-1: synthesis of solid-phase conjugated compound 618-SP

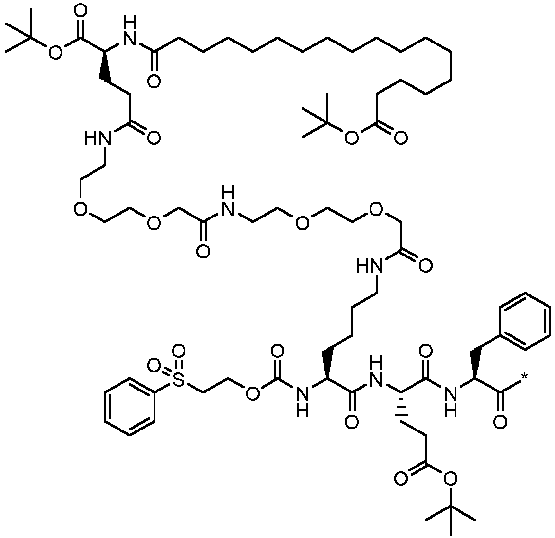

*-Ile-Ala-Trp(Boc)-Leu-Val-Arg(Pbf)-Gly-Arg(Pbf)-Gly-[2-chlorotrityl resin] (SEQ ID NO:24) (618-SP)

200 mg of compound 612-SP (0.044 mmol, 0.22 mmol / g resin) and 105 mg compound 507 (0.088 mmol, 2.0 eq.) are reacted according to general procedure 1 to obtain compound 618-SP (= Psc-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:24).

For analytical purposes, a small-scale test cleavage is performed. This is described at example D-10-1.

### Example C-12-1: synthesis of solid-phase conjugated compound 619-SP

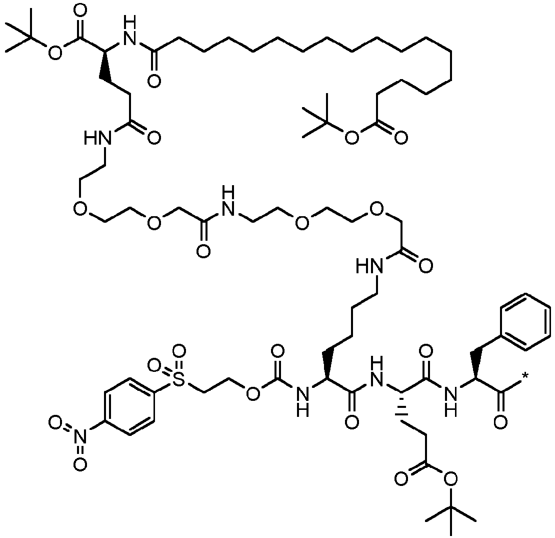

*-Ile-Ala-Trp(Boc)-Leu-Val-Arg(Pbf)-Gly-Arg(Pbf)-Gly-[2-chlorotrityl resin] (SEQ ID NO:25) (619-SP)

200 mg of compound 612-SP (0.044 mmol, 0.22 mmol / g resin) and 105 mg compound 504 (0.088 mmol, 2.0 eq.) are reacted according to general procedure 1 to obtain compound 619-SP (= Nsc-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:25).

For analytical purposes, a small-scale test cleavage is performed. This is described at example D-01-2.

### Example C-13-1: synthesis of solid-phase conjugated compound 620-SP

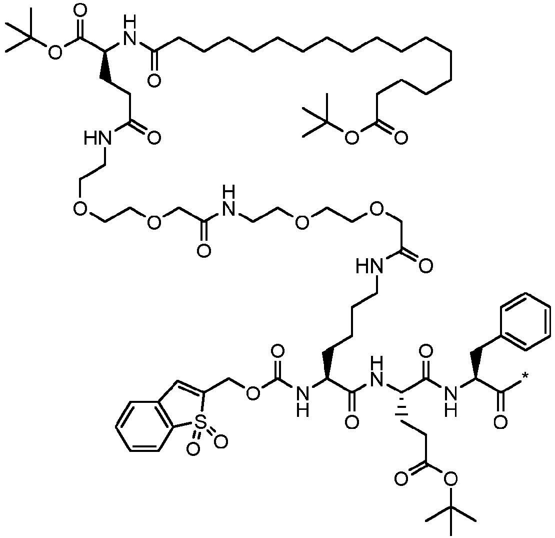

*-Ile-Ala-Trp(Boc)-Leu-Val-Arg(Pbf)-Gly-Arg(Pbf)-Gly-[2-chlorotrityl resin] (SEQ ID NO:23) (620-SP)

200 mg of compound 612-SP (0.044 mmol, 0.22 mmol / g resin) and 105 mg compound 508 (0.088 mmol, 2.0 eq.) are reacted according to general procedure 1 to obtain compound 620-SP (= Bsmoc-Lys(tBuO-CO-(CH₂)₁₆-CO-gamma-Glu(OtBu)-2-[2-(2-aminoethoxy)ethoxyacetyl]-2-[2-(2-aminoethoxy)ethoxyacetyl])-Glu(OtBu)-Phe-Ile-Ala-Trp(Boc)-Leu-Val-Arg(Pbf)-Gly-Arg(Pbf)-Gly-[2-chlorotrityl resin]) (SEQ ID NO:23).

For analytical purposes, a small-scale test cleavage is performed. This is described at example D-11-1.

### D) cleavage of solid-phase conjugated compounds

### Example D-01-1: synthesis of compound 701

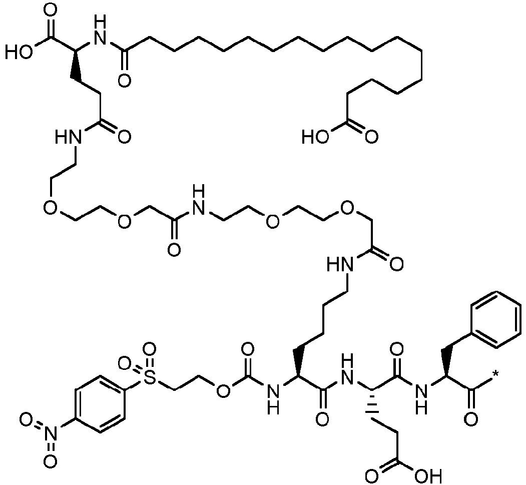

*-Ile-Ala-Trp-Leu-Val-Arg-Gly-Arg-Gly-OH (SEQ ID NO:1) (701)

Compound 701 (= Nsc-Lys(HO-CO-(CH₂)₁₆-CO-gamma-Glu-2-[2-(2-aminoethoxy)ethoxyacetyl]-2-[2-(2-aminoethoxy)ethoxyacetyl])-Glu-Phe-Ile-Ala-Trp-Leu-Val-Arg-Gly-Arg-Gly-OH) (SEQ ID NO:1) is obtainable for example by a deprotecting reaction of compound 603-SP with a cleavage composition of TFA / EDT / H₂O / TIS (90 / 5 / 2.5 / 2.5 in vol.% based on volume of cleavage composition).

### Example D-01-2: synthesis of compound 701

A small sample of the dried compound 619-SP from example C-12-1 is cleaved according to general procedure 2 to obtain a solid containing compound 701 (SEQ ID NO:1).

HPLC method 2: 45.7 area% + 27.8 area%, retention times 18.42 min + 19.39 min / one of both peaks is assumed to be compound 701 and one a decomposition product. Whether the decomposition occurs during TFA-based cleavage, during TFA-containing analytical HPLC method 2 or both, is unknown. TFA-sensitivity of Nsc-protected compound 504 is also observed at Example B-12-8.

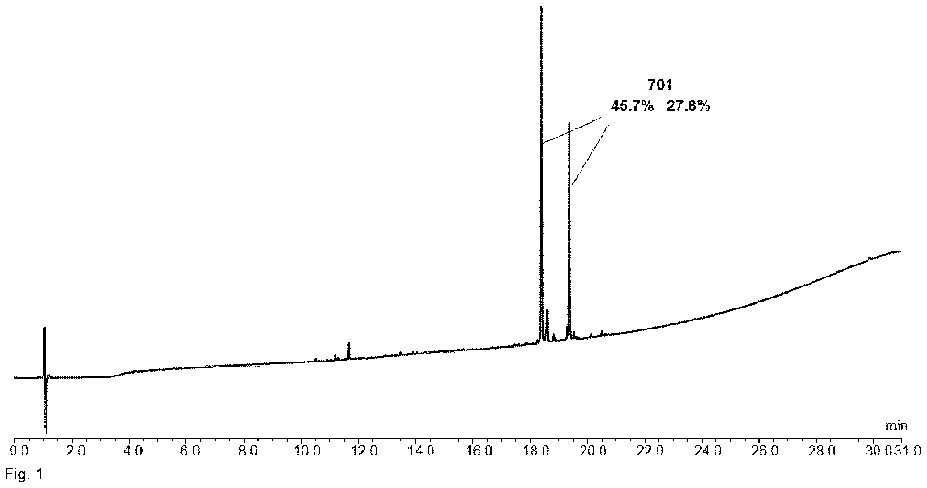

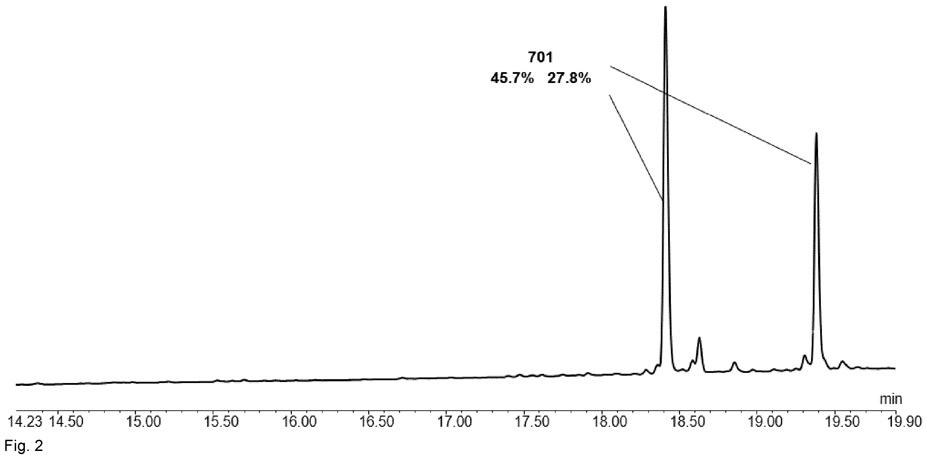

Fig. 1 depicts a complete HPLC-UV chromatogram. Fig. 2 depicts an extract of the complete HPLC-UV chromatogram.

### Example D-02-1: synthesis of compound 702

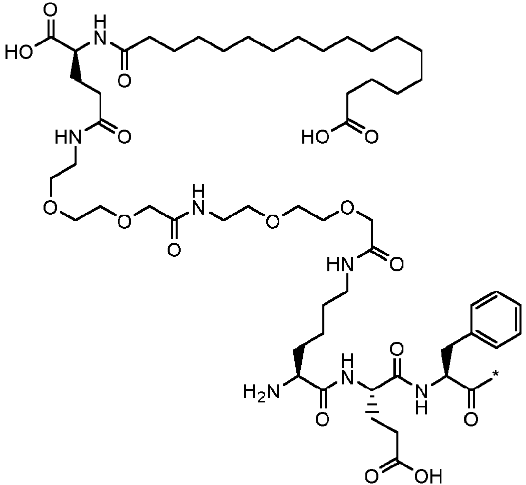

*-Ile-Ala-Trp-Leu-Val-Arg-Gly-Arg-Gly-OH (SEQ ID NO:2) (702)

Compound 702 (= H-Lys(HO-CO-(CH₂)₁₆-CO-gamma-Glu-2-[2-(2-aminoethoxy)ethoxyacetyl]-2-[2-(2-aminoethoxy)ethoxyacetyl])-Glu-Phe-Ile-Ala-Trp-Leu-Val-Arg-Gly-Arg-Gly-OH) (SEQ ID NO:2) is obtainable for example by a deprotecting reaction of compound 606-SP with a cleavage composition of TFA / EDT / H₂O / TIS (90 / 5 / 2.5 / 2.5 in vol.% based on volume of cleavage composition).

### Example D-02-2: synthesis of compound 702

A small sample of the dried compound 614-SP from example C-08-1 is cleaved according to general procedure 2 to obtain a solid containing compound 702 (SEQ ID NO:2).

HPLC method 2: 80.9 area%, retention time 16.65 min.

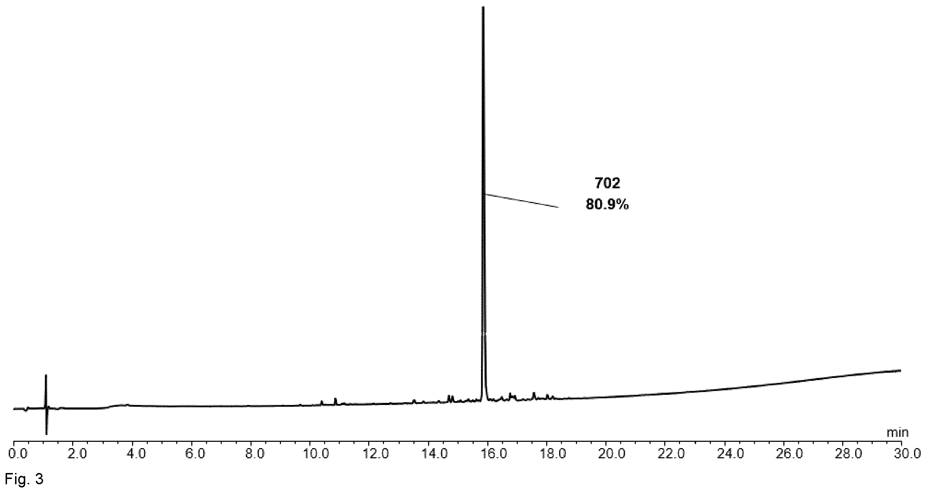

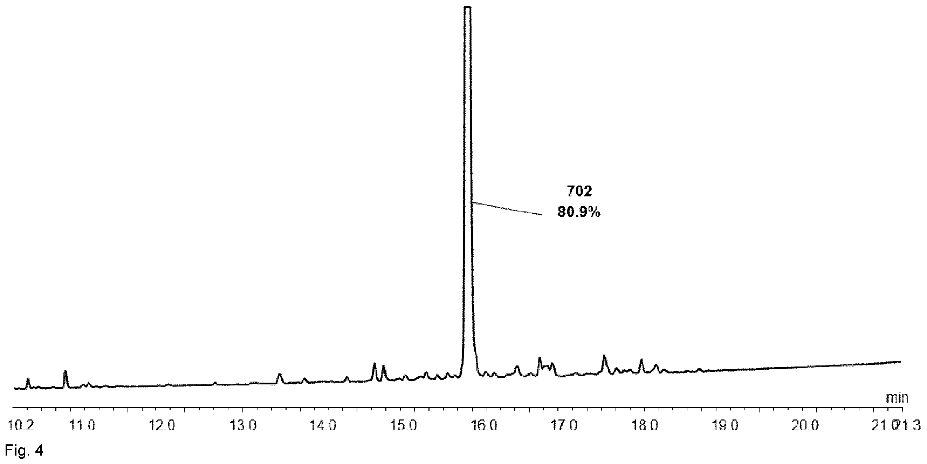

Fig. 3 depicts a complete HPLC-UV chromatogram. Fig. 4 depicts an extract of the complete HPLC-UV chromatogram.

### Example D-02-3: synthesis of compound 702

A small sample of the dried compound 614-SP from example C-08-2 is cleaved according to general procedure 2 to obtain a solid containing compound 702 (SEQ ID NO:2).

HPLC method 2: 76.2 area%, retention time 16.65 min.

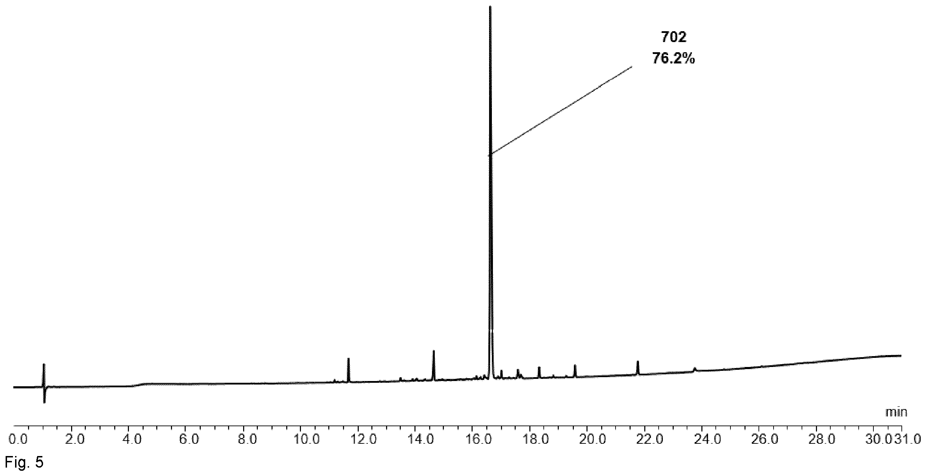

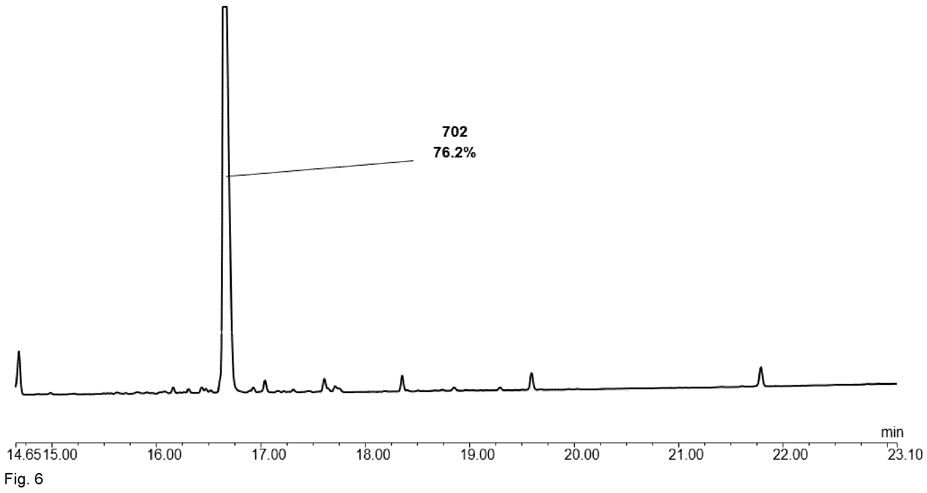

Fig. 5 depicts a complete HPLC-UV chromatogram. Fig. 6 depicts an extract of the complete HPLC-UV chromatogram.

### Example D-02-4: synthesis of compound 702

A small sample of the dried compound 614-SP from example C-08-3 is cleaved according to general procedure 2 to obtain a solid containing compound 702 (SEQ ID NO:2).

HPLC method 2: 76.4 area%, retention time 16.66 min.

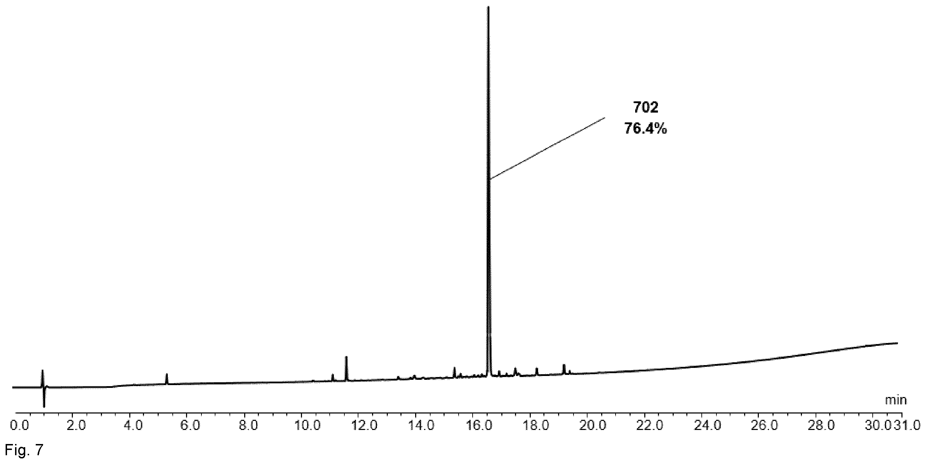

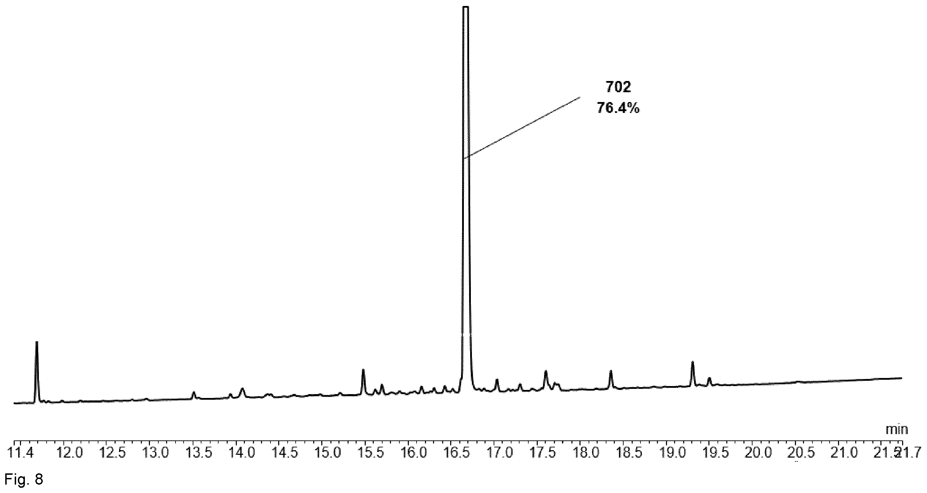

Fig. 7 depicts a complete HPLC-UV chromatogram. Fig. 8 depicts an extract of the complete HPLC-UV chromatogram.

### Example D-02-5: synthesis of compound 702

A small sample of the dried compound 614-SP from example C-08-4 is cleaved according to general procedure 2 to obtain a solid containing compound 702 (SEQ ID NO:2).

HPLC method 2: 81.4 area%, retention time 16.66 min.

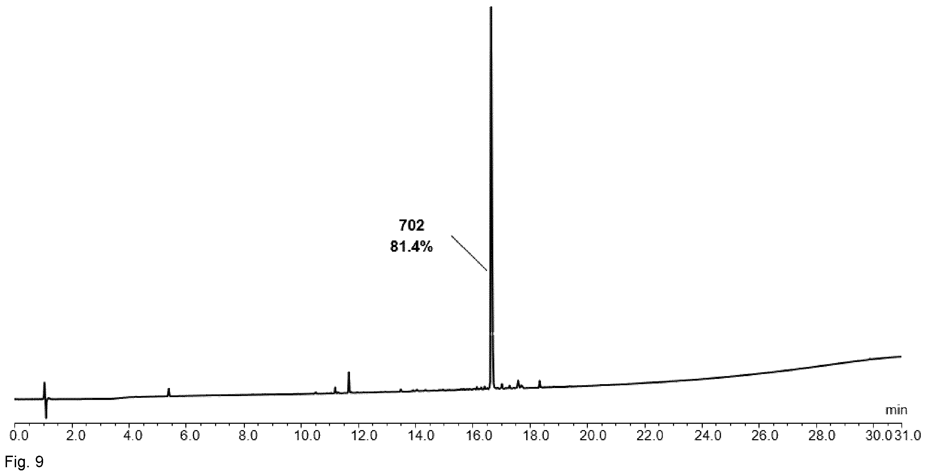

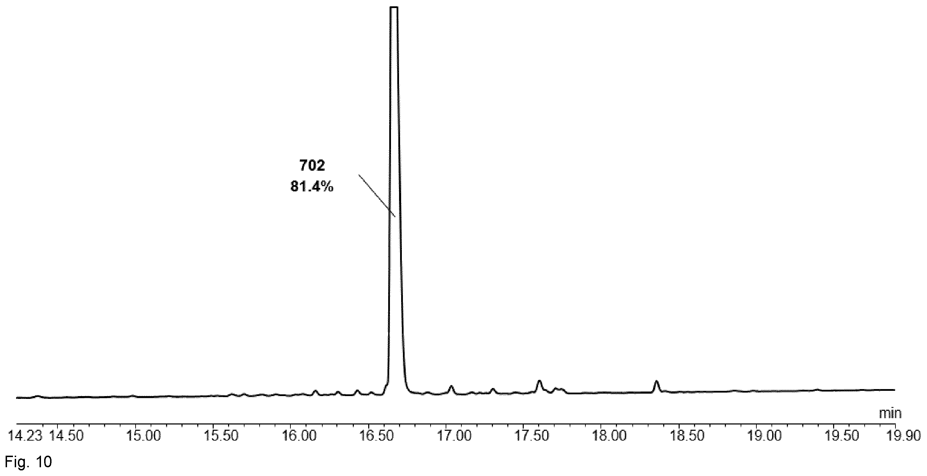

Fig. 9 depicts a complete HPLC-UV chromatogram. Fig. 10 depicts an extract of the complete HPLC-UV chromatogram.

### Example D-02-6: synthesis of compound 702

A small sample of the dried compound 614-SP from example C-08-5 is cleaved according to general procedure 2 to obtain a solid containing compound 702 (SEQ ID NO:2).

HPLC method 2: 81.1 area%, retention time 16.66 min.

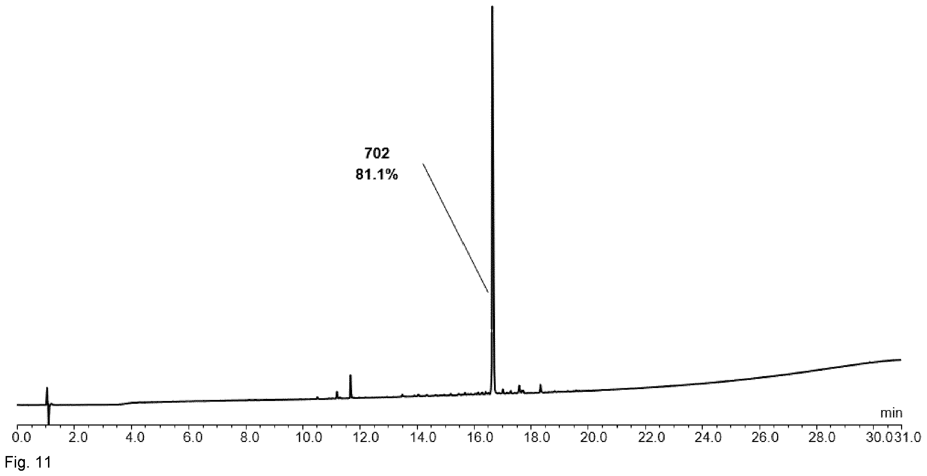

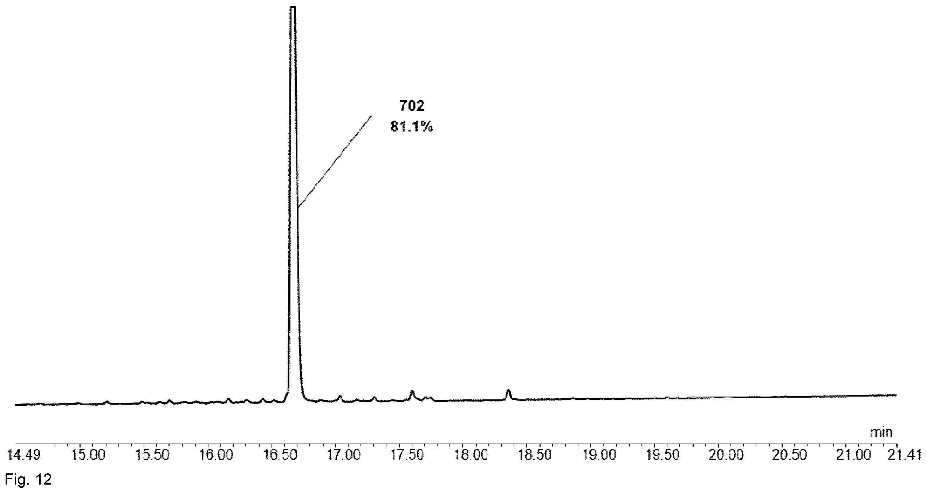

Fig. 11 depicts a complete HPLC-UV chromatogram. Fig. 12 depicts an extract of the complete HPLC-UV chromatogram.

### Example D-03-1: synthesis of compound 703

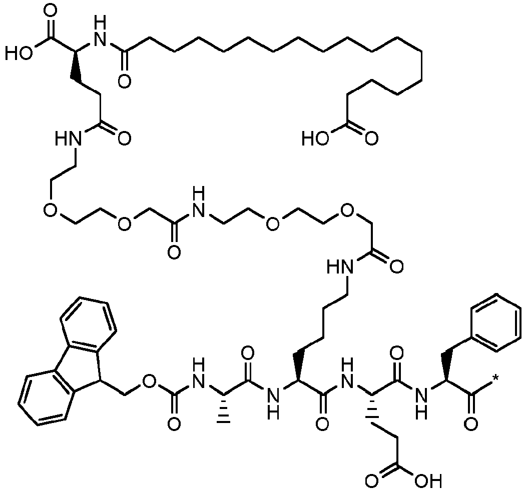

*-Ile-Ala-Trp-Leu-Val-Arg-Gly-Arg-Gly-OH (SEQ ID NO:3) (703)

Compound 703 (= Fmoc-Ala-Lys(HO-CO-(CH₂)₁₆-CO-gamma-Glu-2-[2-(2-aminoethoxy)-ethoxyacetyl]-2-[2-(2-aminoethoxy)ethoxyacetyl])-Glu-Phe-Ile-Ala-Trp-Leu-Val-Arg-Gly-Arg-Gly-OH) (SEQ ID NO:3) is obtainable for example by a deprotecting reaction of compound 605-SP with a cleavage composition of TFA / EDT / H₂O / TIS (90 / 5 / 2.5 / 2.5 in vol.% based on volume of cleavage composition).

### Example D-04-1: synthesis of compound 704

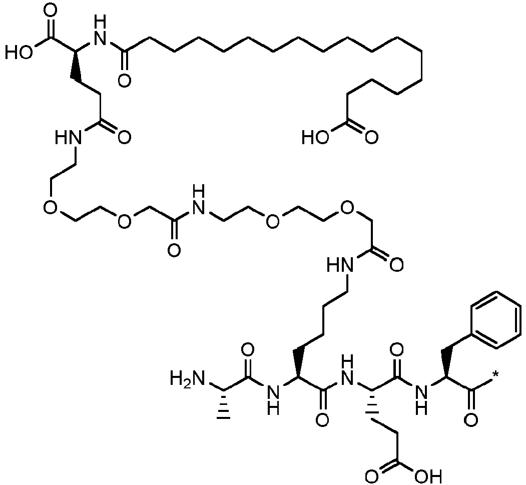

*-Ile-Ala-Trp-Leu-Val-Arg-Gly-Arg-Gly-OH (SEQ ID NO:4) (704)

Compound 704 (= H-Ala-Lys(HO-CO-(CH₂)₁₆-CO-gamma-Glu-2-[2-(2-aminoethoxy)-ethoxyacetyl]-2-[2-(2-aminoethoxy)ethoxyacetyl])-Glu-Phe-Ile-Ala-Trp-Leu-Val-Arg-Gly-Arg-Gly-OH) (SEQ ID NO:4) is obtainable for example by a deprotecting reaction of compound 608-SP with a cleavage composition of TFA / EDT / H₂O / TIS (90 / 5 / 2.5 / 2.5 in vol.% based on volume of cleavage composition).

### Example D-04-2: synthesis of compound 704

A small sample of the dried compound 616-SP from example C-09-1 is cleaved according to general procedure 2 to obtain compound 704 (SEQ ID NO:4).

HPLC method 2: 73.6 area%.

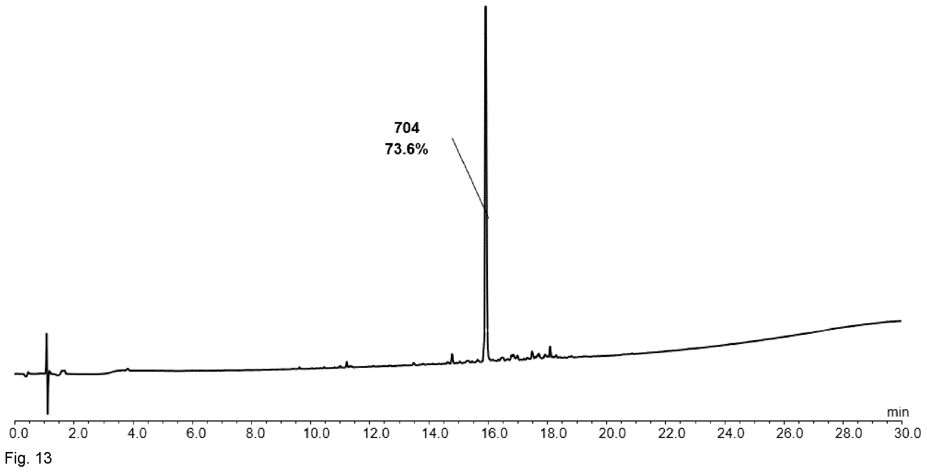

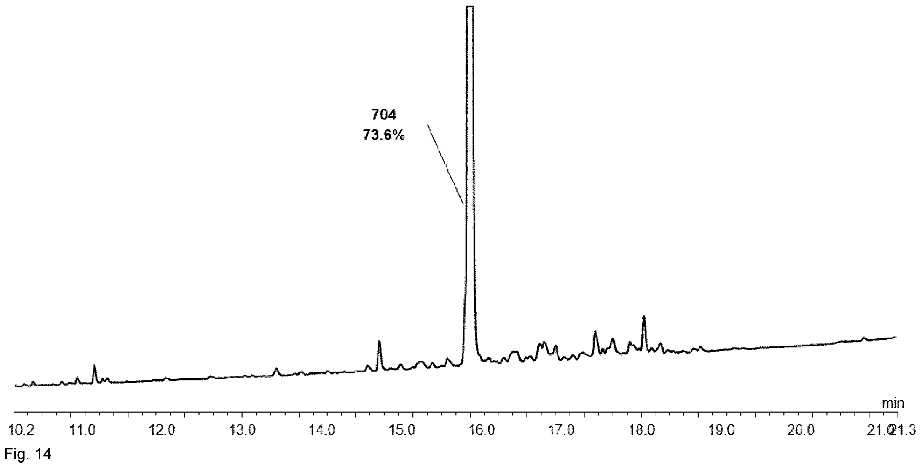

Fig. 13 depicts a complete HPLC-UV chromatogram. Fig. 14 depicts an extract of the complete HPLC-UV chromatogram.

High resolution mass spectroscopy: calculated for [C₁₀₅H₁₇₅N₂₄O₂₈]³⁺ m/z 740.0997, measured 740.1031.

### Example D-04-3: synthesis of compound 704

A small sample of the dried compound 616-SP from example C-09-2 is cleaved according to general procedure 2 to obtain compound 704 (SEQ ID NO:4).

HPLC method 2: 74.6 area%, retention time 16.76 min.

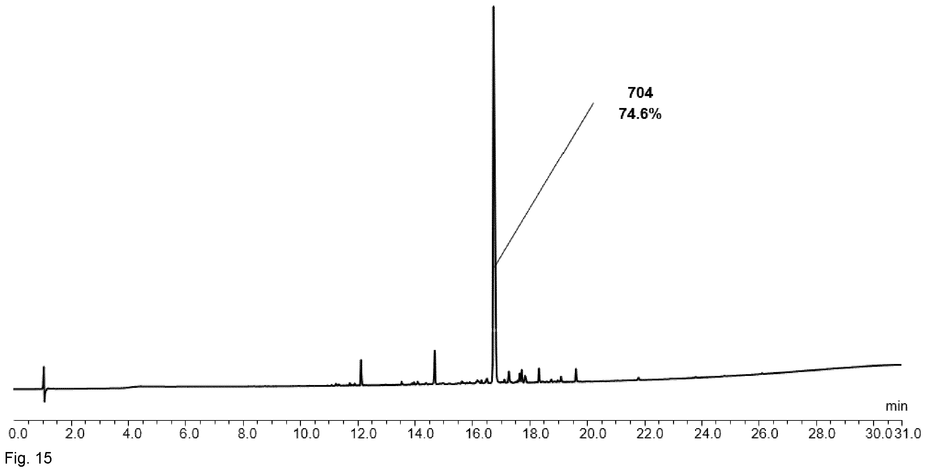

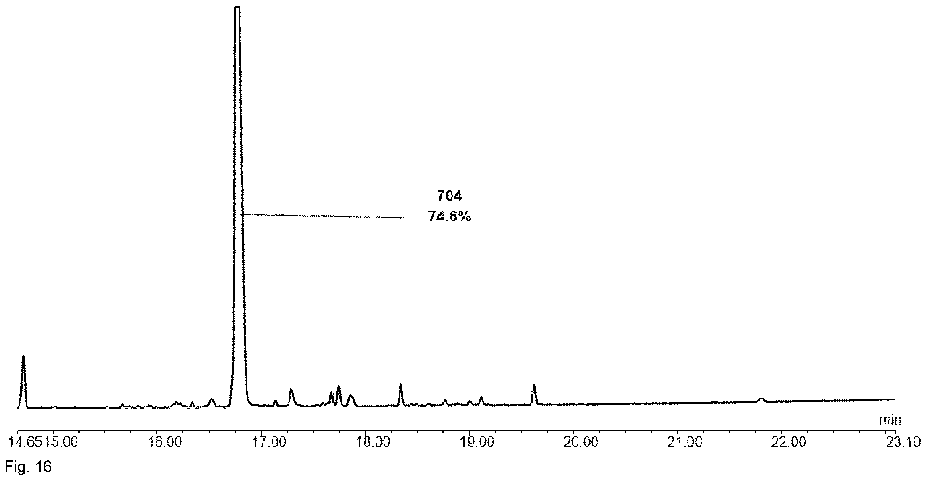

Fig. 15 depicts a complete HPLC-UV chromatogram. Fig. 16 depicts an extract of the complete HPLC-UV chromatogram.

High resolution mass spectroscopy: calculated for [C₁₀₅H₁₇₅N₂₄O₂₈]³⁺ m/z 740.0997, measured 740.1021.

### Example D-04-4: synthesis of compound 704

A small sample of the dried compound 616-SP from example C-09-3 is cleaved according to general procedure 2 to obtain compound 704 (SEQ ID NO:4).

HPLC method 2: 74.9 area%, retention time 16.75 min.

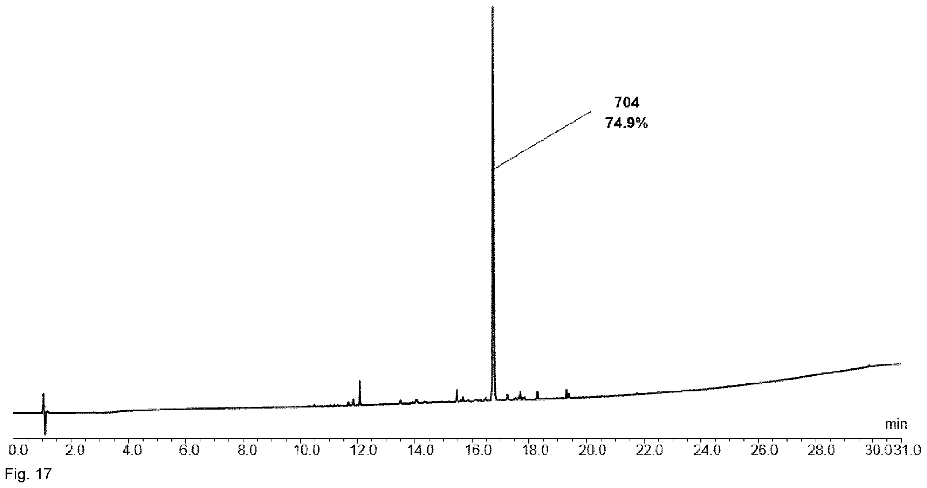

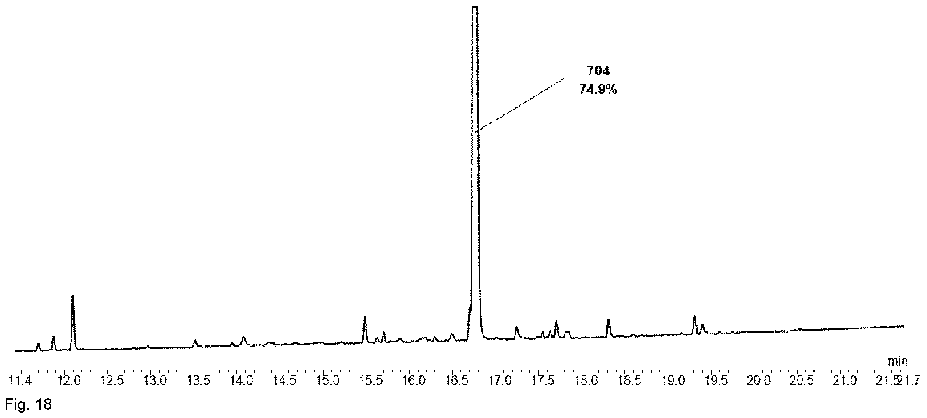

Fig. 17 depicts a complete HPLC-UV chromatogram. Fig. 18 depicts an extract of the complete HPLC-UV chromatogram.

### Example D-04-5: synthesis of compound 704

A small sample of the dried compound 616-SP from example C-09-4 is cleaved according to general procedure 2 to obtain compound 704 (SEQ ID NO:4).

HPLC method 2: 80.7 area%, retention time 16.75 min.

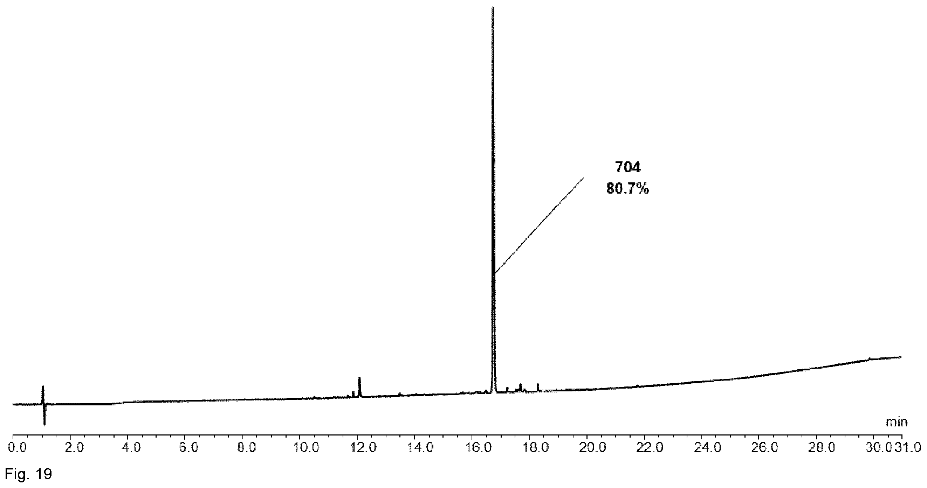

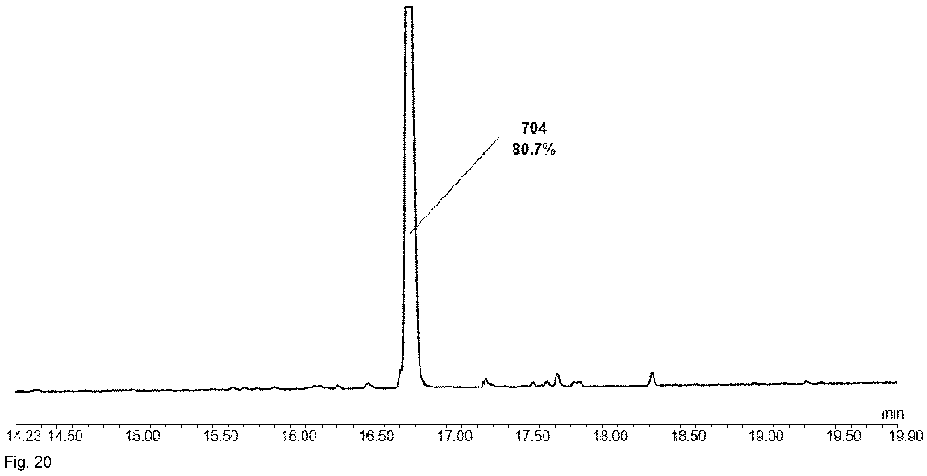

Fig. 19 depicts a complete HPLC-UV chromatogram. Fig. 20 depicts an extract of the complete HPLC-UV chromatogram.

### Example D-04-6: synthesis of compound 704

A small sample of the dried compound 616-SP from example C-09-5 is cleaved according to general procedure 2 to obtain compound 704 (SEQ ID NO:4).

HPLC method 2: 78.2 area%, retention time 16.76 min.

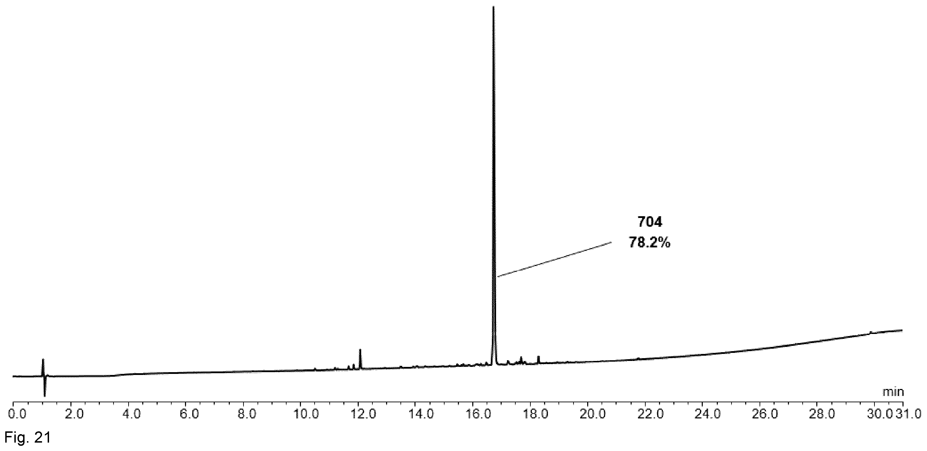

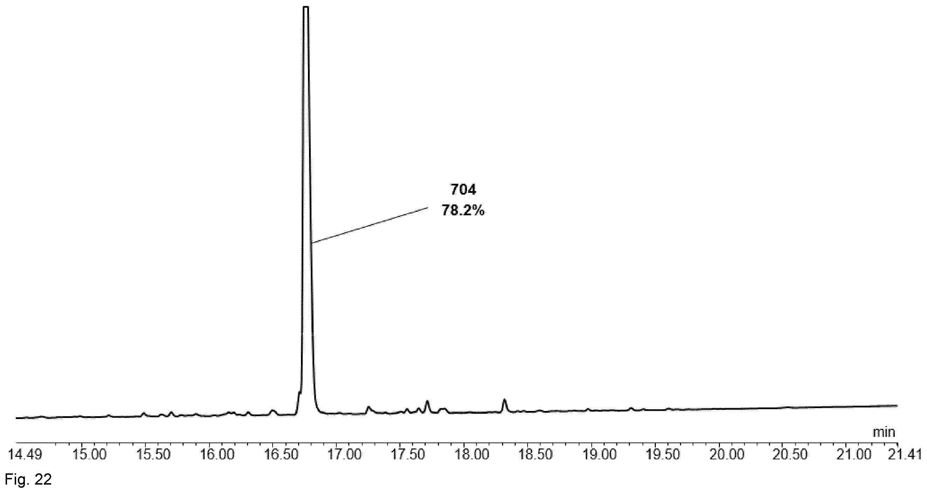

Fig. 21 depicts a complete HPLC-UV chromatogram. Fig. 22 depicts an extract of the complete HPLC-UV chromatogram.

### Example D-05-1: synthesis of compound 705

H-His-Aib-Glu-Gly-Thr-Phe-Thr-Ser-Asp-Val-Ser-Ser-Tyr-Leu-Glu-Gly-Gln-Ala-Ala-Lys(HO-CO-
(CH2)16-CO-gamma-Glu-2-[2-(2-aminoethoxy)ethoxyacetyl]-2-[2-(2-aminoethoxy)ethoxyacetyl])-
Glu-Phe-Ile-Ala-Trp-Leu-Val-Arg-Gly-Arg-Gly-OH
(SEQ ID NO:5) (705)

Compound 705 (= H-His-Aib-Glu-Gly-Thr-Phe-Thr-Ser-Asp-Val-Ser-Ser-Tyr-Leu-Glu-Gly-Gln-Ala-Ala-Lys(HO-CO-(CH₂)₁₆-CO-gamma-Glu-2-[2-(2-aminoethoxy)ethoxyacetyl]-2-[2-(2-aminoethoxy)ethoxyacetyl])-Glu-Phe-Ile-Ala-Trp-Leu-Val-Arg-Gly-Arg-Gly-OH (SEQ ID NO:5) respectively Semaglutide (CAS-No. 910463-68-2)) (SEQ ID NO:5) is obtainable for example by a deprotecting reaction of compound 607-SP with a cleavage composition of TFA / EDT / H₂O / TIS (90 / 5 / 2.5 / 2.5 in vol.% based on volume of cleavage composition).

### Example D-06-1: synthesis of compound 706

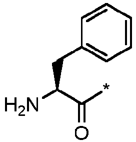

*-Ile-Ala-Trp-Leu-Val-Arg-Gly-Arg-Gly-OH (SEQ ID NO:6) (706)

A small sample of the dried compound 611-SP from example C-05-1 is cleaved according to general procedure 2 to obtain compound 706 (SEQ ID NO:6).

HPLC method 2: 88.4 area%, retention time 11.18 min.

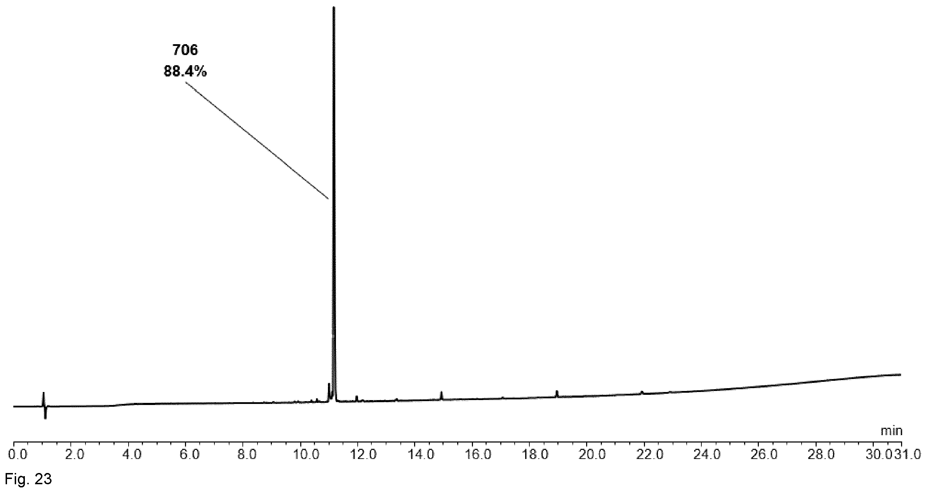

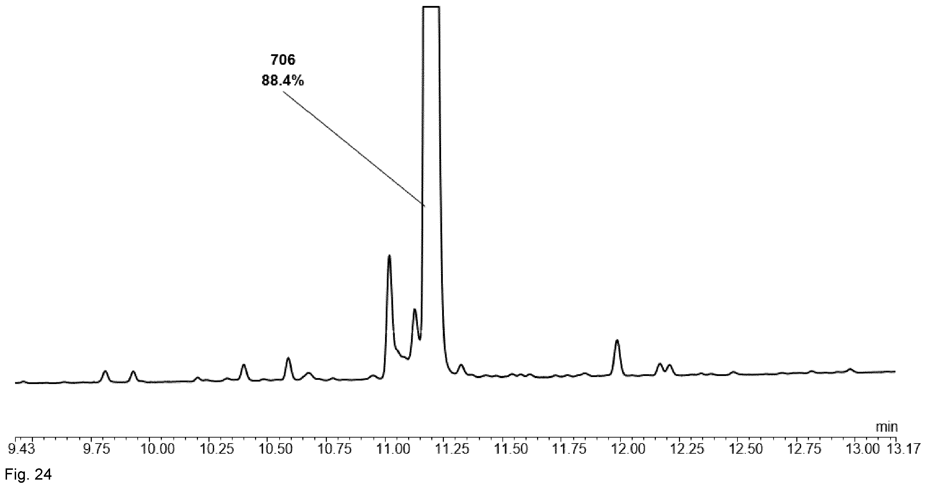

Fig. 23 depicts a complete HPLC-UV chromatogram. Fig. 24 depicts an extract of the complete HPLC-UV chromatogram.

### Example D-07-1: synthesis of compound 707

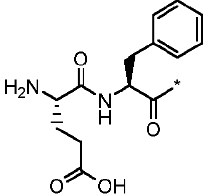

*-Ile-Ala-Trp-Leu-Val-Arg-Gly-Arg-Gly-OH (SEQ ID NO:7) (707)

A small sample of the dried compound 612-SP from example C-06-1 is cleaved according to general procedure 2 to obtain compound 707 (SEQ ID NO:7).

HPLC method 2: 84.0 area%, retention time 11.65 min.

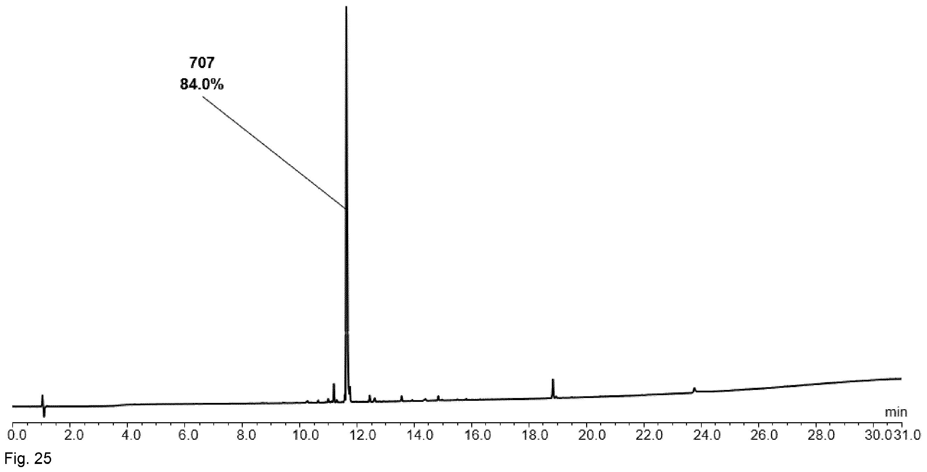

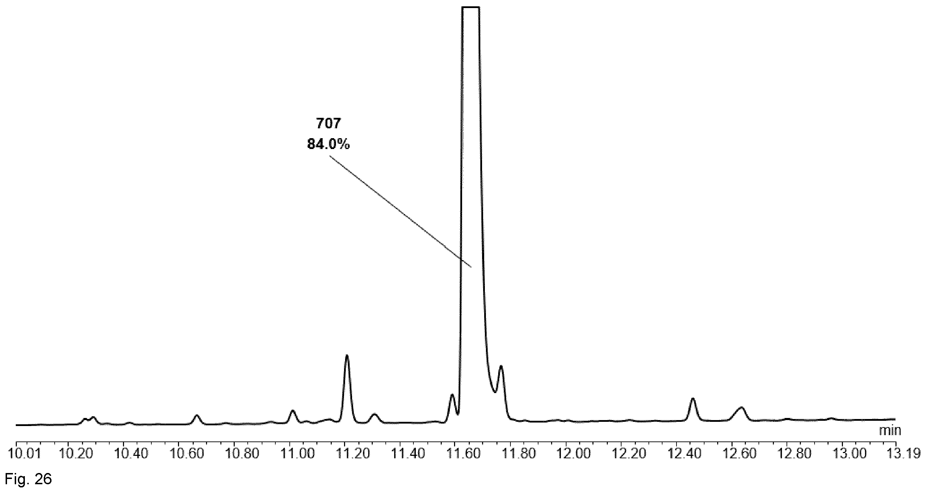

Fig. 25 depicts a complete HPLC-UV chromatogram. Fig. 26 depicts an extract of the complete HPLC-UV chromatogram.

### Example D-08-1: synthesis of compound 708

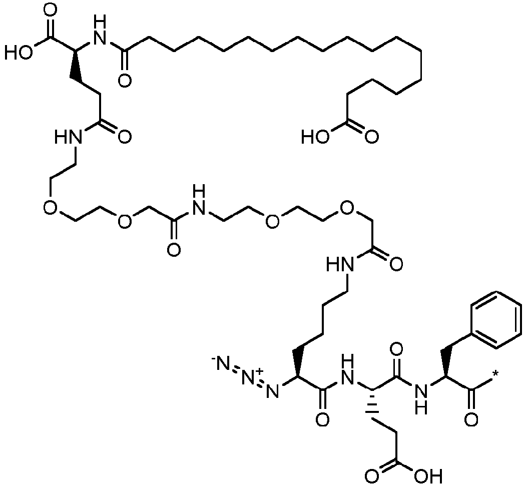

*-Ile-Ala-Trp-Leu-Val-Arg-Gly-Arg-Gly-OH (SEQ ID NO:8) (708)

A small sample of the dried compound 613-SP from example C-07-1 is cleaved according to general procedure 2 to obtain compound 708 (SEQ ID NO:8).

HPLC method 2: 81.6 area%, retention time 21.64 min.

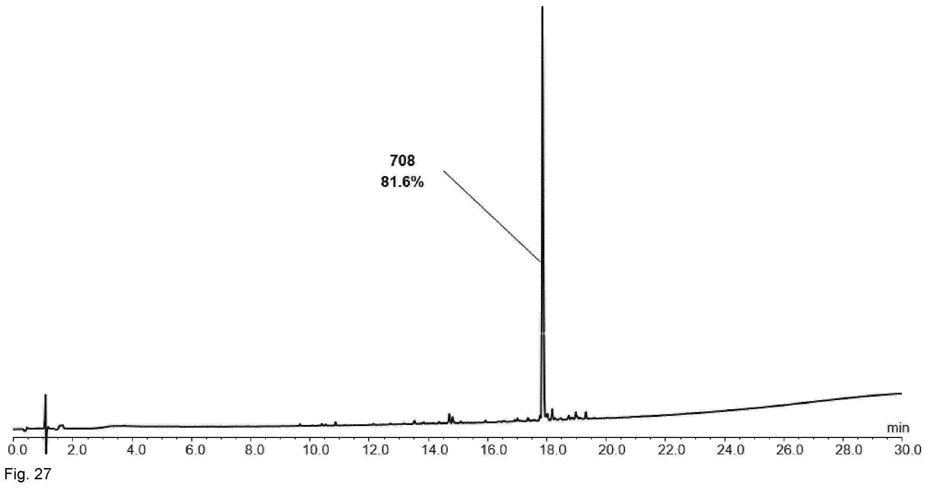

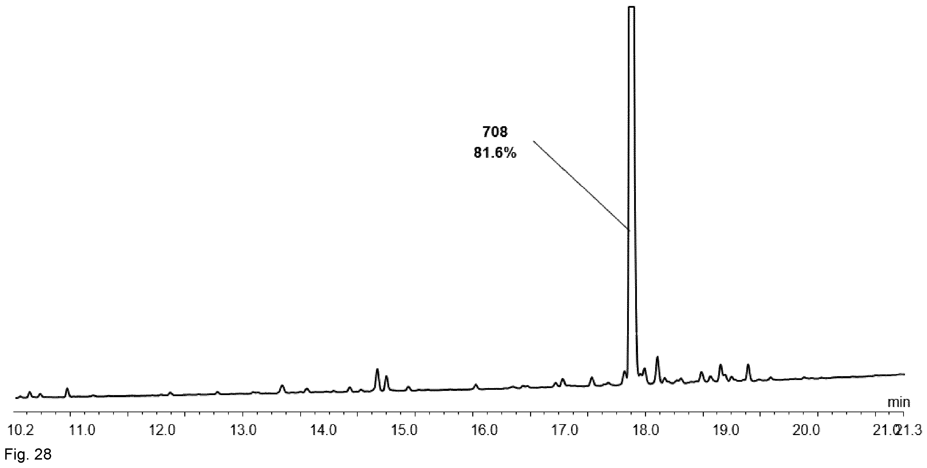

Fig. 27 depicts a complete HPLC-UV chromatogram. Fig. 28 depicts an extract of the complete HPLC-UV chromatogram.

### Example D-09-1: synthesis of compound 709

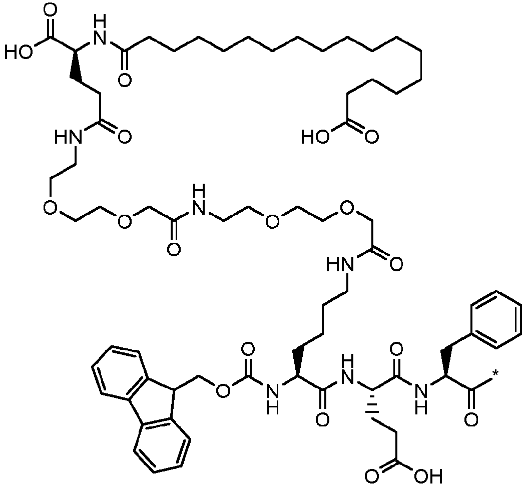

*-Ile-Ala-Trp-Leu-Val-Arg-Gly-Arg-Gly-OH (SEQ ID NO:9) (709)

A small sample of the dried compound 617-SP from example C-10-1 is cleaved according to general procedure 2 to obtain compound 709 (SEQ ID NO:9).

HPLC method 2: 80.2 area%, retention time 21.72 min.

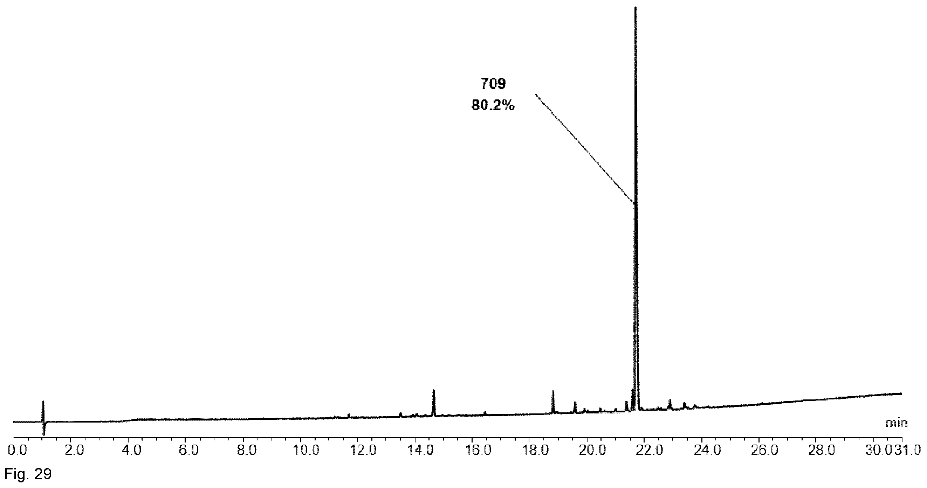

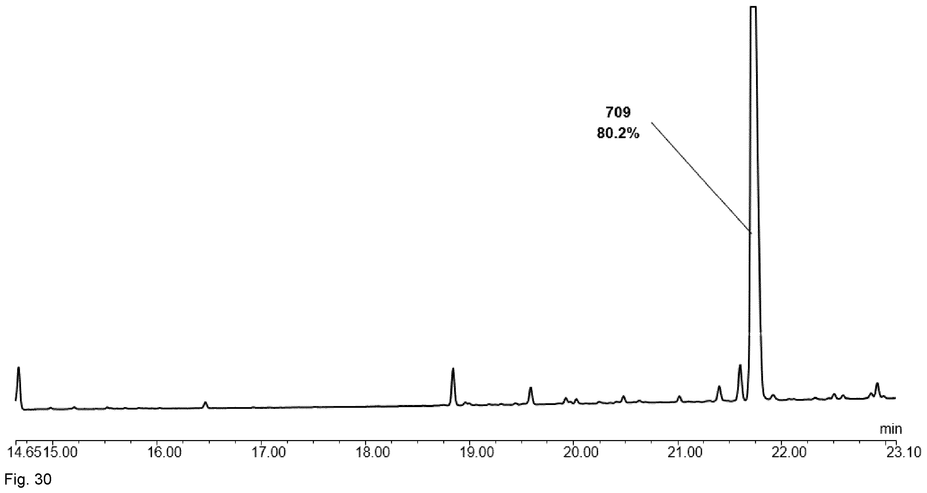

Fig. 29 depicts a complete HPLC-UV chromatogram. Fig. 30 depicts an extract of the complete HPLC-UV chromatogram.

### Example D-10-1: synthesis of compound 710

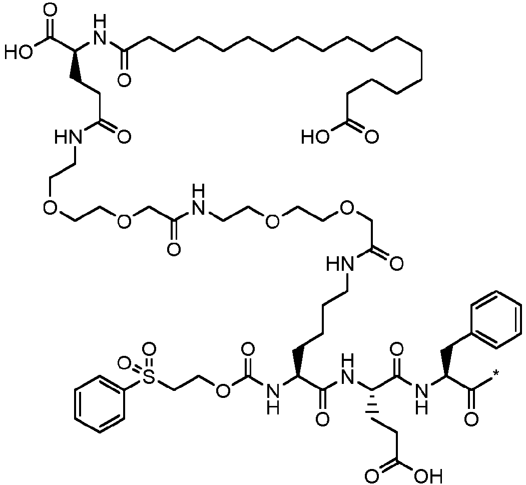

*-Ile-Ala-Trp-Leu-Val-Arg-Gly-Arg-Gly-OH (SEQ ID NO:10) (710)

A small sample of the dried compound 618-SP from example C-11-1 is cleaved according to general procedure 2 to obtain compound 710 (SEQ ID NO:10).

HPLC method 2: 76.4 area%, retention time 19.26 min.

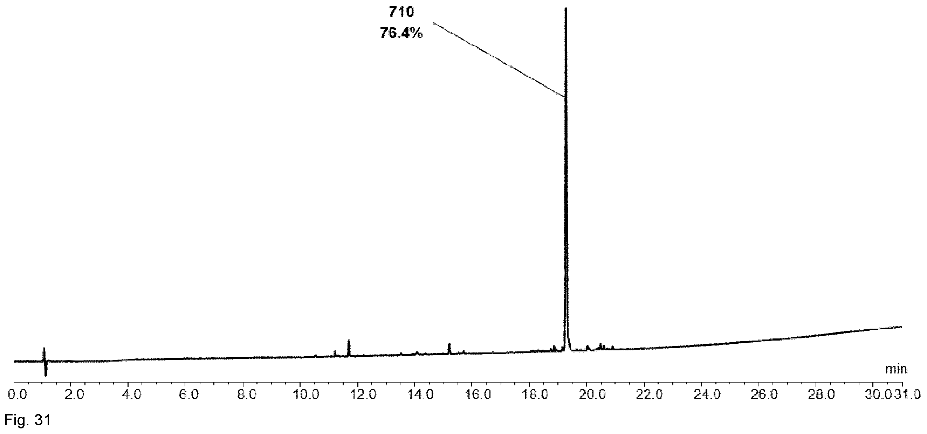

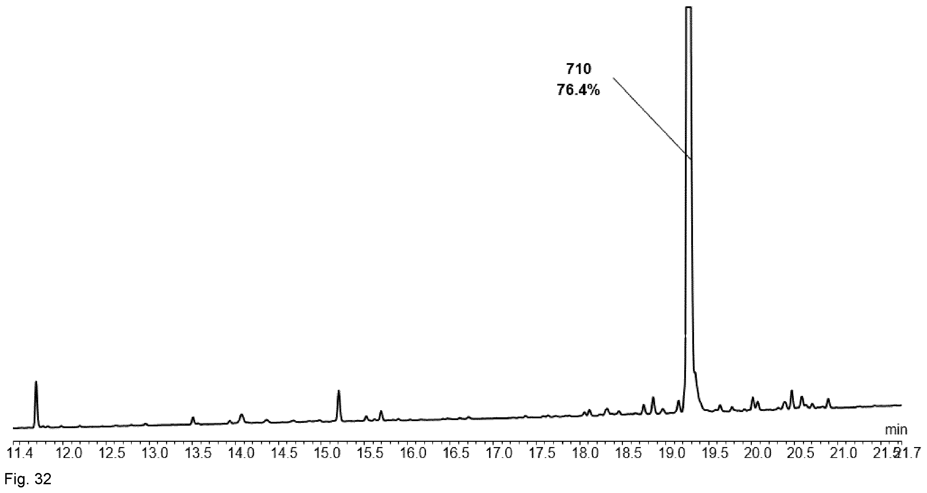

Fig. 31 depicts a complete HPLC-UV chromatogram. Fig. 32 depicts an extract of the complete HPLC-UV chromatogram.

### Example D-11-1: synthesis of compound 711

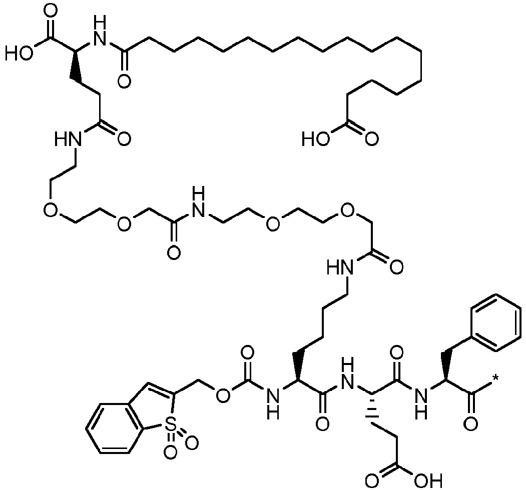

*-Ile-Ala-Trp-Leu-Val-Arg-Gly-Arg-Gly-OH (SEQ ID NO:11) (711)

A small sample of the dried compound 620-SP from example C-13-1 is cleaved according to general procedure 2 to obtain compound 711 (SEQ ID NO:11).

HPLC method 2: 81.0 area%, retention time 19.45 min.

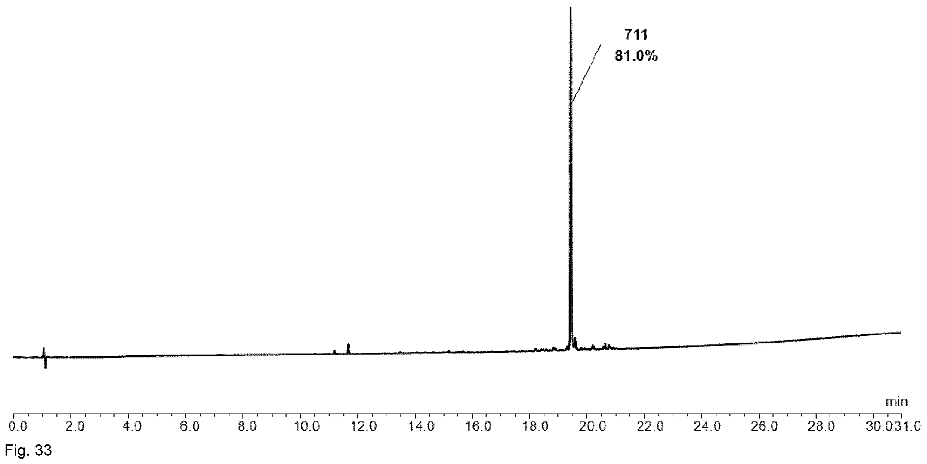

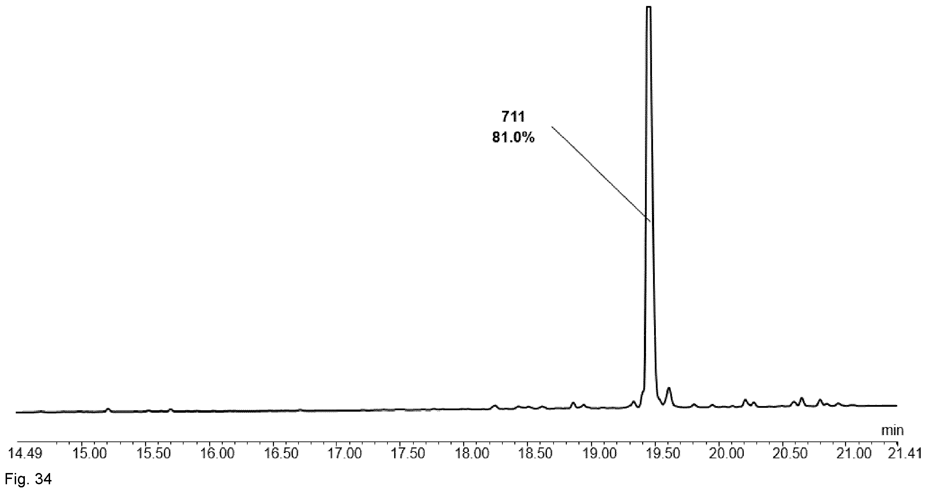

Fig. 33 depicts a complete HPLC-UV chromatogram. Fig. 34 depicts an extract of the complete HPLC-UV chromatogram.

### E) comparison

Results obtained at cleaving small scale samples from solid-phase conjugated compounds under section D) are put into relation to conducted reactions with solid-phase conjugated compounds under section C) at a table E-1.

**Table E-1**

| | amino protecting group | Fmoc^{a)} | N3^{a)} | Psc^{a)} | Nsc^{a)} | Bsmoc ^{b)} |
|---|---|---|---|---|---|---|
| cycle I | | | | | | |
| step (c) | condensing I [example No.] | C-10-1 | C-07-1 | C-11-1 | C-12-1 | C-13-1 |
| | lysine derivative | 501 | 506 | 507 | 504 | 508 |
| | solid-phase conjugated compound | 617-SP | 613-SP | 618-SP | 619-SP | 620-SP |
| [step (x) ^{c)}] | cleaved and side-chain deprotected compound / ([area%]) ^{d)} | 709 (80.2%) | 708 (73.5%) | 710 (76.4%) | 701 ^{e)} | 711 (81.0%) |
| | | | | | (45.7% or 27.8%) | |
| | | | | | | |
| step (d) | removing amino protecting group I ^{f)} [example No.] | C-08-2 | C-08-1 | C-08-3 | C-08-4 | C-08-5 |
| | solid-phase conjugated compound | 614-SP | 614-SP | 614-SP | 614-SP | 614-SP |
| [step (x) ^{c)}] | cleaved and fully deprotected compound / [area%] | 702 | 702 | 702 | 702 | 702 |
| | | 76.2% | 80.9% | 76.2% | 81.4% | 81.1% |
| | | | | | | |
| | average (c) + (d) ^{g)} ([area%]) ^{d)} | (78.1%) | (77.2%) | (76.3%) | ( -^{h)}) | (81.1%) |
| | | | | | | |
| cycle II | | | | | | |
| step (e) + ... ⁱ⁾ | condensing II and removing Fmoc-group II^{j)} [example No.] | C-09-2 | C-09-1 | C-09-3 | C-09-4 | C-09-5 |
| | solid-phase conjugated compound | 616-SP | 616-SP | 616-SP | 616-SP | 616-SP |
| [step (x) ^{c)}] | cleaved and deprotected compound / total (e)+ ... ^{i), k)} [area%] | 704 | 704 | 704 | 704 | 704 |
| | | 74.6% | 73.6% | 74.9% | 80.7% | 78.2% |
| | | | | | | |
| | average 702 + 704 ^{l)} [area%] | 75.4% | 77.3% | 75.6% | 81.1% | 79.7% |
| | | | | | | |

| | | | | | | |
|---|---|---|---|---|---|---|
| Footnotes: a) comparative b) inventive c) for a quantification of an effectiveness of the solid-phase reaction, a cleavage of the solid-phase conjugated reaction product from the resin is conducted and an area percentage for the obtained compound at a HPLC-UV chromatogram determined d) different amino protecting groups have different UV-absorbing properties at 220 nm, which requires some caution at an interpretation and therefore the brackets e) only one of the observed peaks with 45.7 area% or 27.8 area% is compound 701, the other one is a decomposition product f) optimized deprotection conditions are employed for the respective amino protecting group, i.e. a phosphine reduction for N3, 20 vol.% piperidine in DMF for Fmoc and Bsmoc, 2 vol.% DBU and 20 vol.% piperidine in DMF for Psc and Nsc g) due to different deprotection conditions at step (d), a direct comparison of the combined effectiveness of the solid-phase reactions at step (c) and step (d) is formally not possible - however, an averaged cycle I effectiveness is on the one hand a confirmation of a condensing effectiveness at step (c) and shows on the other hand a removing effectiveness for the amino protecting group at step (d) h) decomposition of compound 702 makes an average not possible i) three dots represent a step of removing Fmoc-group at cycle II i) condensing with Fmoc-Ala-OH and removing Fmoc with 20 vol. % piperidine in DMF k) it is noted that the starting resin material is not similar, i.e. synthesis at the previous cycle I has occurred with compounds 501, 504, 506, 507 or 508 and partially different deprotection conditions due to the involved respective amino protecting group I) average over cycle I and cycle II is based on area% of cleaved and fully deprotected compound 702 and on area% of cleaved and fully deprotected compound 704 | | | | | | |

The results of table E-1 show
- that dependent on the amino protecting group of the lysine derivative, which is employed as one reactant of the condensing step (c) at a solid-phase cycle I, an effectiveness of the cycle I as indicated by area-percentages of cleaved and fully deprotected compound 702 and an effectiveness of a subsequent solid-phase cycle II as indicated by area-percentages of cleaved and deprotected compound 704 is influenced as indicated by area-percentages of the peptides cleaved from the solid-phase;
- that Nsc provides the highest effectiveness based on area percentages followed closely by Bsmoc, whereas N3 is next and followed by Psc and Fmoc;
- that a reasonably presumable influence of a sterical hindrance of the amino proctecting group at the lysine derivative is not decisive, i.e. in view of the least hindered N3, which is followed by Psc / Nsc with two methylene units after the oxy-carbonyl unit and then Fmoc / Bsmoc with one methylene unit after the oxycarbonyl unit;
- that compound 701 with Nsc as the amino protecting group has a high sensitivity against TFA and thus confirms the high sensitivity of Nsc as the amino protecting group at compound 504 versus an exposure against TFA (and an exposure against of aqueous sodium hydrogencarbonate) as shown in Example B-12-8.

## Claims

1. A method of manufacturing a peptide P, the method comprises a step (c)
(c) condensing an alpha amino acid derivative S-am, which has one unprotected alpha amino group or one unprotected alpha imino group,
with a compound of formula Pr-L 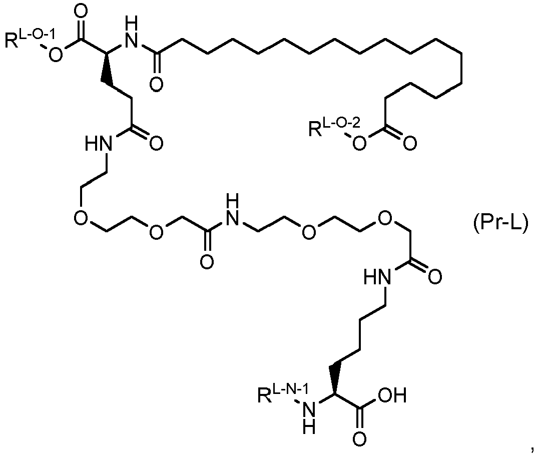
wherein
R^{L-O-1} and R^{L-O-2} are independently of each other a carboxylic acid protecting group,
to obtain a peptide Pr-L-S,
wherein R^{L-N-1} is an amino protecting group of formula Bsmoc 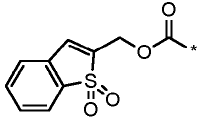 with * indicating the bond to the nitrogen atom
(Bsmoc).

2. The method according to claim 1, wherein the alpha amino acid derivative S-am is a compound of formula S-I-am or S-II-am 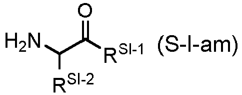 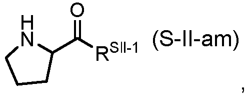 wherein
R^{SI-1} is -(AA^{nx})ₘ-O-[resin-1], -(AA^{nx})ₘ-N-[resin-2], -O-[resin-1], -N-[resin-2], -(AA^{nx})ₘ-OR^{SI-1-1}, OR^{SI-1-1} or NH₂,
R^{SI-2} is H, C₁₋₆ alkyl or C₁₋₆ alkyl mono-substituted with OR^{SI-2-1}, SR^{SI-2-2}, SCH₃, NR^{SI-2-3}R^{SI-2-4}, CO-OR^{SI-2-5}, CO-NR^{SI-2-6}R^{SI-2-7}, N'-R^{SI-2-8}-N"-R^{SI-2-9}-guanidino, phenyl, para-(R^{SI-2-10}O)-phenyl, 1-R^{SI-2-11}-imidazol-4-yl or 1-R^{SI-2-12}-indol-3-yl, -(AA^{nx})ₘ- is a substituent consisting of m condensed alpha amino acid residues AA^{nx} with each x being an integer and x running from 1 to m,
each condensed alpha amino acid residue AA^{nx} is independently chosen and in case it possesses a side chain with a functional group, the functional group is unprotected or protected by a protecting group provided that an interfering functional group is protected,
m is an integer from 1 to 30,
R^{SI-1-1} is H or a carboxylic acid protecting group
R^{SI-2-1} is H or a hydroxy protecting group,
R^{SI-2-2} is a thiol protecting group,
R^{SI-2-3} and R^{SI-2-4} are H, an amino protecting group or form together an amino protecting group provided that not both are H,
R^{SI-2-5} is H or a carboxylic acid protecting group,
R^{SI-2-6} and R^{SI-2-7} are H or an amide protecting group,
R^{SI-2-8} and R^{SI-2-9} are H or a guanidino protecting group provided that not both are H,
R^{SI-2-10} is a protecting group for an aromatic hydroxy group,
R^{SI-2-11} is H or a protecting group for an imidazole nitrogen atom,
R^{SI-2-12} is H or a protecting group for an indole nitrogen atom,
R^{SII-1} is as defined for R^{SI-1},
and the peptide Pr-L-S is a compound of formula Pr-L-S-I or Pr-L-S-II 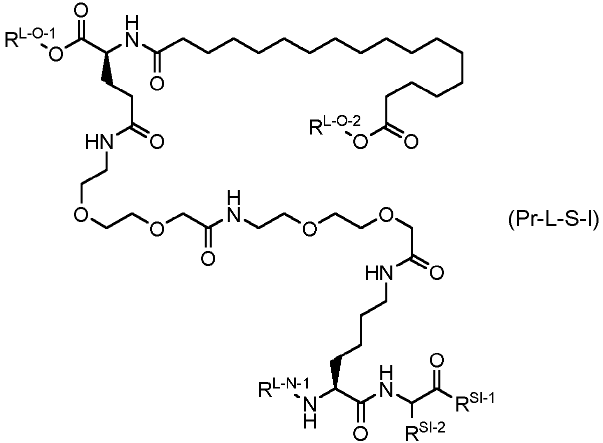 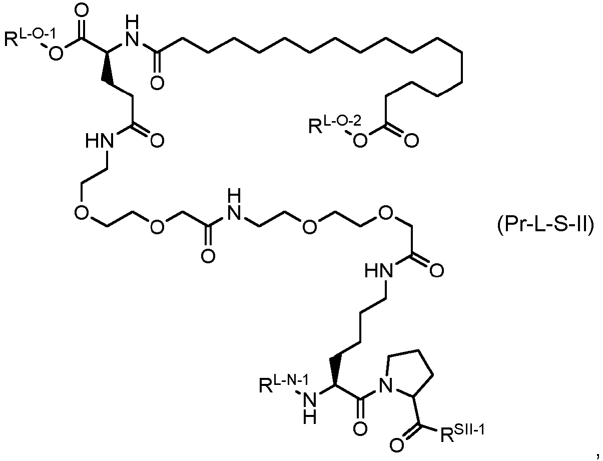
wherein
R^{L-O-1}, R^{L-O-2} and R^{L-N-1} are defined as for formula Pr-L,
R^{SI-1}, R^{SI-2} and R^{SII-1} are defined as for formula S-I-am or S-II-am.

3. The method according to claim 2, which comprises the step (d)
(d) removing the amino protecting group R^{L-N-1} at the compound of formula Pr-L-S-I or Pr-L-S-II to obtain a compound of formula L-S-I-am or L-S-II-am 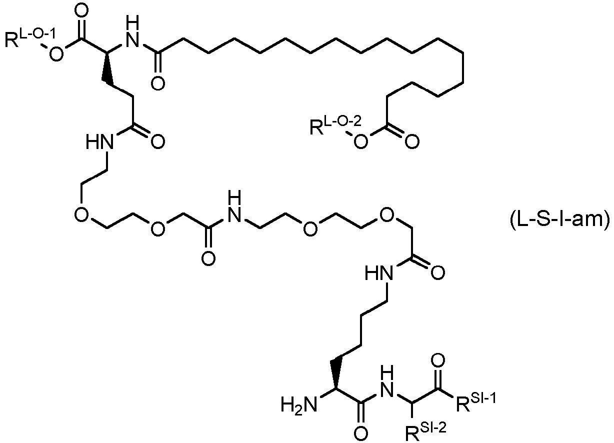 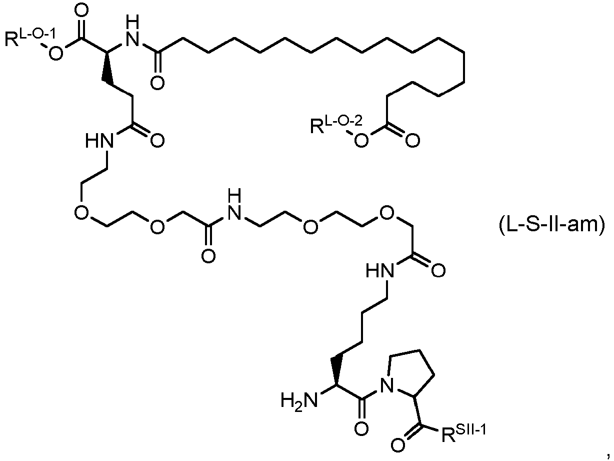 wherein
R^{L-O-1}, R^{L-O-2}, R^{SI-1}, R^{SI-2} and R^{SII-2} are defined as for formula Pr-L-S-I or Pr-L-S-II.

4. The method according to claim 3, which comprises the step (e)
(e) condensing the compound of formula L-S-I-am or L-S-II-am with an alpha-amino acid derivative of formula T
R^{T-N-1}-(AA^{py})_{q}-OH (T)
wherein
R^{T-N-1} is an amino protecting group,
-(AA^{py})_{q}- is a substituent consisting of q condensed alpha amino acid residues AA^{py} with each y being an integer and y running from 1 to q,
each condensed alpha amino acid residue AA^{py} is independently chosen and in case it possesses a side chain with a functional group, the functional group is unprotected or protected by a protecting group provided that an interfering functional group is protected,
q is an integer from 1 to 30,
to obtain a compound of formula T-L-S-I or T-L-S-II 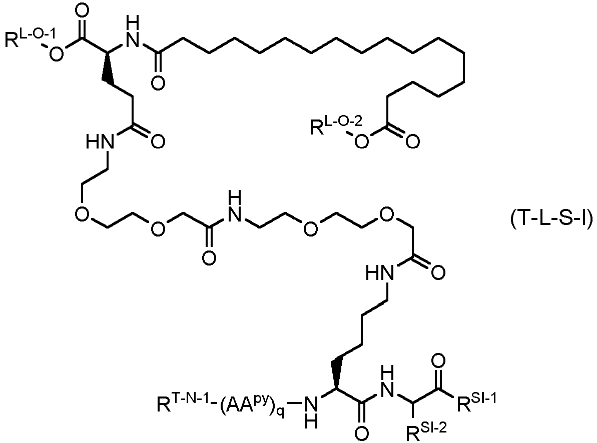 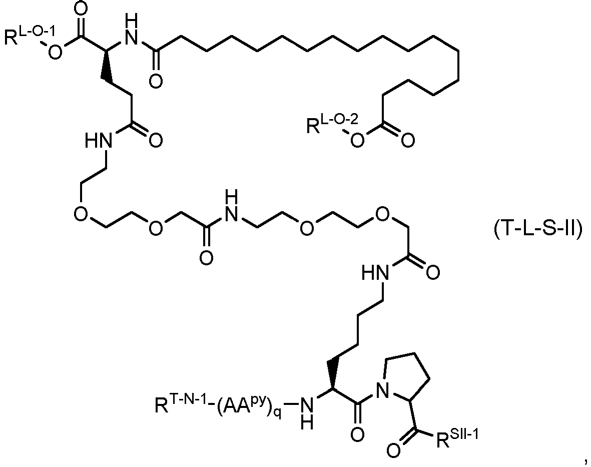 wherein
R^{L-O-1}, R^{L-O-2}, R^{SI-1}, R^{SI-2} and R^{SII-2} are defined as for formula L-S-I-am or L-S-II-am,
R^{T-N-1} and -(AA^{py})_{q}- are defined as for formula T.

5. The method according to claim 4, which comprises one or more further condensing cycles applied to the compound of formula T-L-S-I or T-L-S-II, each of the one or more further condensing cycles comprises
a first step of removing the amino protecting group R^{T-N-1} of the compound of formula T-L-S-I or T-L-S-II respectively the amino protecting group of the alpha amino group of the N-terminal amino acid residue of the peptide resulting from the previous condensing cycle to obtain the related peptide with one unprotected amino group, and
a second step of condensing the unprotected amino group of the related peptide obtained at the first step with an alpha amino acid derivative, which has an alpha amino group at its N-terminal amino acid residue, which is protected by an amino protecting group and which has one unprotected alpha carboxylic acid group, which is located at its C-terminal amino acid residue, to obtain a peptide resulting from the condensing cycle.

6. The method according to any preceding claim, wherein the alpha amino acid derivative Sam is covalently linked to a resin, and which comprises a step (x)
(x) cleaving the peptide obtained from the last conducted condensing step from the resin by a cleaving composition to obtain a cleaved peptide.

7. The method according to any one of claims 2 to 6, wherein the alpha amino acid derivative S-am of step (c) is of formula S-I-am,
wherein
R^{SI-1} is a -(AA^{nx})₁₀-O-[resin-1],
AAⁿ¹ is Phe, AAⁿ² is Ile, AAⁿ³ is Ala, AAⁿ⁴ is a Trp with a protected sidechain, AAⁿ⁵ is Leu, AAⁿ⁶ is Val, AAⁿ⁷ is an Arg with a protected sidechain, AAⁿ⁸ is Gly, AAⁿ⁹ is an Arg with a protected sidechain and AAⁿ¹⁰ is Gly,
resin-1 is a 2-chlorotritylamidomethyl resin or a 2-chlorotrityl resin,
R^{SI-2} is 2-(tert-butyloxycarbonyl)ethyl.

8. The method according to any preceding claim, which comprises a step (y)
(y) removing all remaining protecting groups from the peptide obtained from the last conducted condensing step to obtain a peptide free of protecting groups.

9. The method according to any preceding claim, wherein R^{L-O-1} and R^{L-O-2} are independently from each other tert-butyl or 3-methyl-pent-3-yl.

10. The method according to any preceding claim, wherein at step (c) the compound of formula Pr-L is activated with a condensing agent of step (c).

11. The method according to any preceding claim, wherein at step (c) a coupling additive of step (c) is present.

12. The method according to claims 10 and 11, wherein the condensing agent of step (c) is a carbodiimide derivative, and the coupling additive of step (c) is cyano-hydroxyimino-acetic acid ethyl ester.

13. The method according to any preceding claim, wherein the step (c) is conducted in a solvent of step (c) and the solvent of step (c) comprises N,N-dimethylformamide.

14. A compound of formula Pr-L 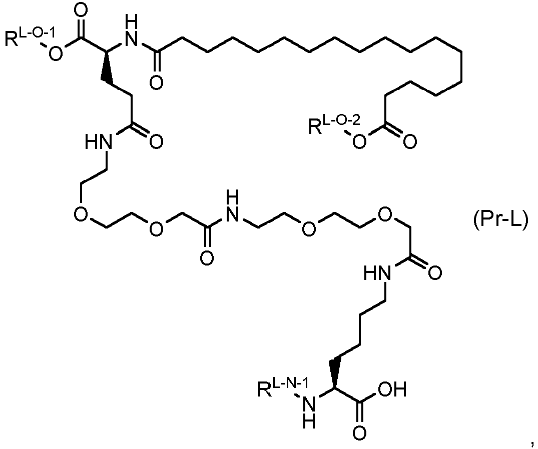 wherein
R^{L-O-1} and R^{L-O-2} are independently of each other a carboxylic acid protecting group,
R^{L-N-1} is an amino protecting group of formula Bsmoc 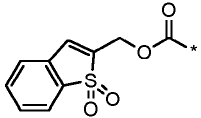 with * indicating the bond to the nitrogen atom
(Bsmoc).

15. Use of a compound of formula Pr-L as defined in claim 1 in the synthesis of a peptide P.

## Patentansprüche

1. Verfahren zur Herstellung eines Peptids P, wobei das Verfahren einen Schritt (c) umfasst
(c) Kondensieren eines Alpha-Aminosäurederivats S-am, das eine ungeschützte Alpha-Aminogruppe oder eine ungeschützte Alpha-Iminogruppe aufweist,
mit einer Verbindung der Formel Pr-L 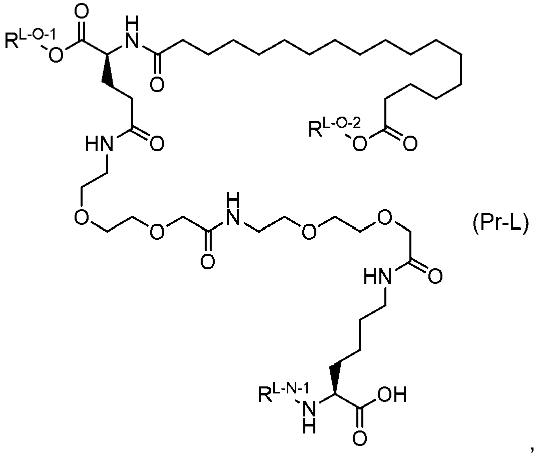
wobei
R^{L-O-1} und R^{L-O-2} unabhängig voneinander eine Carbonsäure-Schutzgruppe sind,
um ein Peptid Pr-L-S zu erhalten,
wobei R^{L-N-1} eine Aminoschutzgruppe der Formel Bsmoc ist 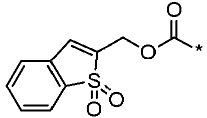 wobei * die Bindung an das Stickstoffatom
(Bsmoc) anzeigt.

2. Verfahren nach Anspruch 1, wobei das Alpha-Aminosäurederivat S-am eine Verbindung der Formel S-I-am oder S-II-am ist 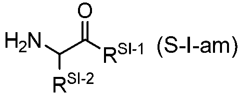 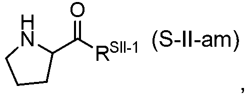 wobei
R^{SI-1} -(AA^{nx} )ₘ -O-[Harz-1], -(AA^{nx} )ₘ -N-[Harz-2], -O-[Harz-1], -N-[Harz-2], -(AA^{nx} )ₘ -OR^{SI-1-1} , OR^{SI-1-1} oder NH₂ ist,
R^{SI-2} H, C₁₋₆-Alkyl oder C₁₋₆-Alkyl ist, das mit OR^{SI-2-1} , SR^{SI-2-2} , SCH₃ , NR^{SI-2-3}R^{SI-2-4}, CO-OR^{SI-2-5}, CO-NR^{SI-2-6}R^{SI-2-7}, N'-R^{SI-2-8}-N"-R^{SI-2-9} -Guanidino, Phenyl, para-(R^{SI-2-10}O)-Phenyl, 1-R^{SI-2-11} -Imidazol-4-yl oder 1-R^{SI-2-12} -Indol-3-yl monosubstituiert ist,
-(AA^{nx})ₘ - ein Substituent ist, bestehend aus m kondensierten Alpha-Aminosäureresten AA^{(nx)} wobei jedes x eine ganze Zahl ist und x von 1 bis m reicht,
jeder kondensierte Alpha-Aminosäurerest AA^{nx} unabhängig ausgewählt ist und, falls er eine Seitenkette mit einer funktionellen Gruppe besitzt, die funktionelle Gruppe ungeschützt oder durch eine Schutzgruppe geschützt ist, vorausgesetzt, dass eine störende funktionelle Gruppe geschützt ist,
m eine ganze Zahl von 1 bis 30 ist,
R^{SI-1-1} H oder eine Carbonsäure-Schutzgruppe ist,
R^{SI-2-1} H oder eine Hydroxyschutzgruppe ist,
R^{SI-2-2} eine Thiolschutzgruppe ist,
R^{SI-2-3} und R^{SI-2-4} H, eine Aminoschutzgruppe sind oder zusammen eine Aminoschutzgruppe bilden, vorausgesetzt, dass nicht beide H sind,
R^{SI-2-5} H oder eine Carbonsäure-Schutzgruppe, ist
R^{SI-2-6} und R^{SI-2-7} H oder eine Amidschutzgruppe sind,
R^{SI-2-8} und R^{SI-2-9} H oder eine Guanidino-Schutzgruppe sind, vorausgesetzt, dass nicht beide H sind,
R^{SI-2-10} eine Schutzgruppe für eine aromatische Hydroxygruppe ist,
R^{SI-2-11} H oder eine Schutzgruppe für ein Imidazol-Stickstoffatom ist,
R^{SI-2-12} H oder eine Schutzgruppe für ein Indol-Stickstoffatom ist,
R^{SII-1} wie für R^{SI-1} definiert ist,
und das Peptid Pr-L-S ist eine Verbindung der Formel Pr-L-S-I oder Pr-L-S-II ist 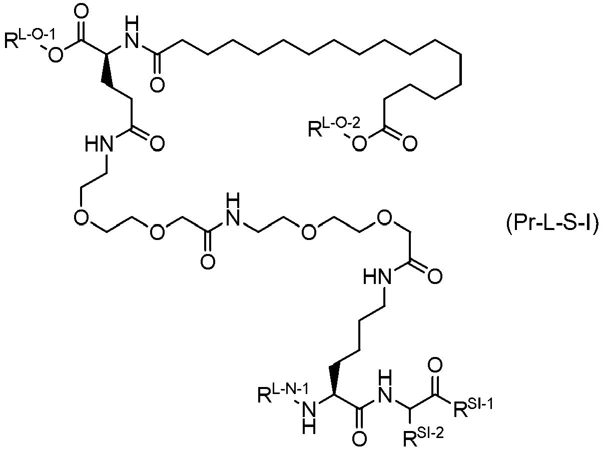 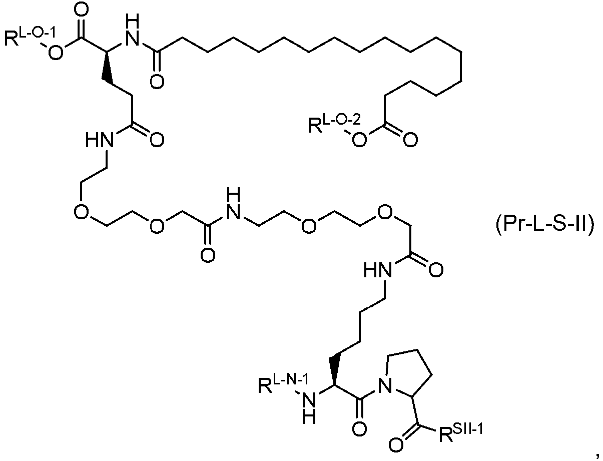
wobei
R^{L-O-1}, R^{L-O-2} und R^{L-N-1} wie für die Formel Pr-L definiert sind,
R^{SI-1}, R^{SI-2} und R^{SII-1} wie für die Formel S-I-am oder S-II-am definiert sind.

3. Verfahren nach Anspruch 2, das den Schritt (d) umfasst
(d) Entfernen der Aminoschutzgruppe R^{L-N-1} an der Verbindung der Formel Pr-L-S-I oder Pr-L-S-II, um eine Verbindung der Formel L-S-I-am oder L-S-II-am zu erhalten 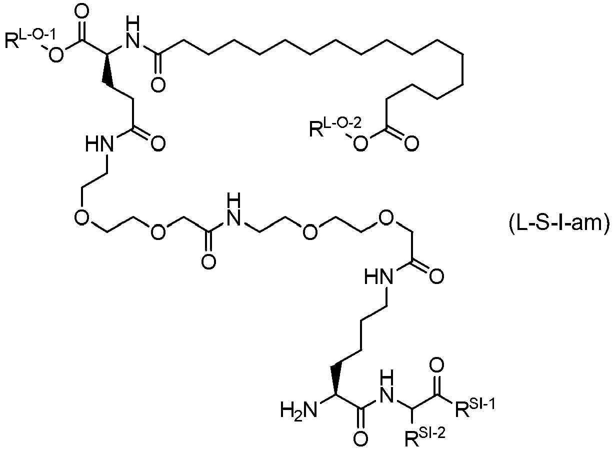 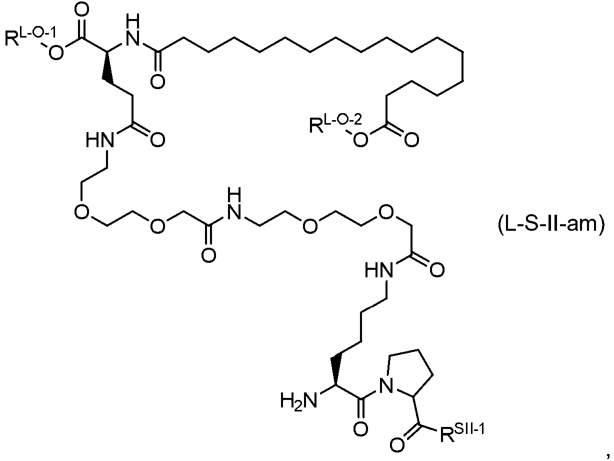 wobei
R^{L-O-1}, R^{L-O-2}, R^{SI-1}, R^{SI-2} und R^{SII-2} wie für die Formel Pr-L-S-I oder Pr-L-S-II definiert sind.

4. Verfahren nach Anspruch 3, das den Schritt (e) umfasst
(e) Kondensieren der Verbindung der Formel L-S-I-am oder L-S-II-am mit einem Alpha-Aminosäurederivat der Formel T
R^{T-N-1} -(AA^{py})_{q} -OH (T)
wobei
R^{T-N-1} eine Aminoschutzgruppe ist,
-(AA^{py})_{q} - ein Substituent ist, der aus q kondensierten Alpha-Aminosäureresten AA^{py} besteht, wobei jedes y eine ganze Zahl ist und y von 1 bis q reicht,
jeder kondensierte Alpha-Aminosäurerest AA^{py} unabhängig ausgewählt ist und, falls er eine Seitenkette mit einer funktionellen Gruppe besitzt, die funktionelle Gruppe ungeschützt oder durch eine Schutzgruppe geschützt ist, vorausgesetzt, dass eine störende funktionelle Gruppe geschützt ist,
q eine ganze Zahl von 1 bis 30 ist,
um eine Verbindung der Formel T-L-S-I oder T-L-S-II zu erhalten 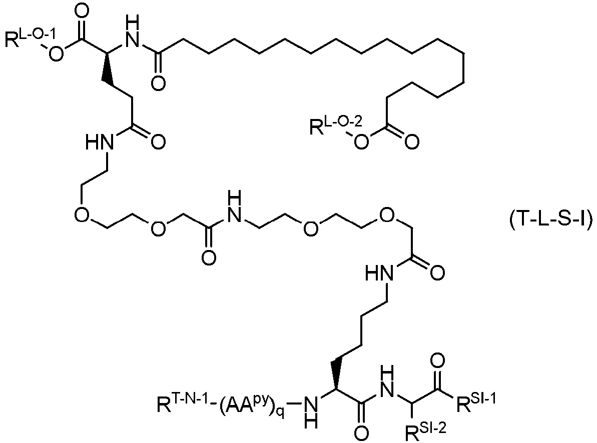 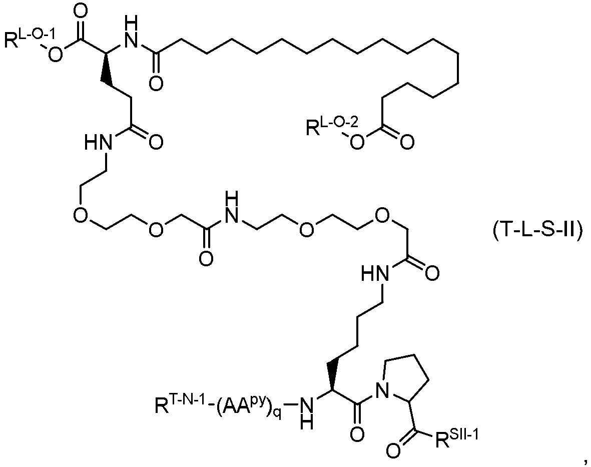 wobei
R^{L-O-1}, R^{L-O-2}, R^{SI-1}, R^{SI-2} und R^{SII-2} wie für die Formel L-S-I-am oder L-S-II-am definiert sind,
R^{T-N-1} und -(AA^{py})_{q} - wie für die Formel T definiert sind.

5. Verfahren nach Anspruch 4, das einen oder mehrere weitere Kondensationszyklen umfasst, die auf die Verbindung der Formel T-L-S-I oder T-L-S-II angewendet werden, wobei jeder der einen oder mehreren weiteren Kondensationszyklen umfasst
einen ersten Schritt zum Entfernen der Aminoschutzgruppe R^{T-N-1} der Verbindung der Formel T-L-S-I bzw. T-L-S-II, d. h. der Aminoschutzgruppe der Alpha-Aminogruppe des N-terminalen Aminosäurerests des aus dem vorherigen Kondensationszyklus resultierenden Peptids, um das entsprechende Peptid mit einer ungeschützten Aminogruppe zu erhalten, und
einen zweiten Schritt der Kondensation der ungeschützten Aminogruppe des im ersten Schritt erhaltenen Peptids mit einem Alpha-Aminosäurederivat, das eine Alpha-Aminogruppe an seinem N-terminalen Aminosäurerest aufweist, die durch eine Aminoschutzgruppe geschützt ist, und das eine ungeschützte Alpha-Carbonsäuregruppe aufweist, die sich an seinem C-terminalen Aminosäurerest befindet, um ein aus dem Kondensationszyklus resultierendes Peptid zu erhalten.

6. Verfahren nach einem der vorstehenden Ansprüche, wobei das Alpha-Aminosäurederivat S-am kovalent an ein Harz gebunden ist und das einen Schritt (x) umfasst
(x) Abspalten des aus dem zuletzt durchgeführten Kondensationsschritt erhaltenen Peptids vom Harz mittels einer Spaltzusammensetzung, um ein gespaltenes Peptid zu erhalten.

7. Verfahren nach einem der Ansprüche 2 bis 6, wobei das Alpha-Aminosäurederivat S-am aus Schritt (c) die Formel S-I-am aufweist,
wobei
R^{SI-1} ein -(AA^{n(x})₁₀-O-[Harz-1] ist,
AAⁿ¹ Phe ist, AAⁿ² Ile ist, AAⁿ³ Ala ist, AAⁿ⁴ ein Trp mit einer geschützten Seitenkette ist, AAⁿ⁵ Leu ist, AAⁿ⁶ Val ist, AAⁿ⁷ ein Arg mit einer geschützten Seitenkette ist, AAⁿ⁸ Gly ist, AAⁿ⁹ ein Arg mit einer geschützten Seitenkette ist und AAⁿ¹⁰ Gly ist,
Harz-1 ein 2-Chlortritylamidomethylharz oder ein 2-Chlortritylharz ist,
R^{SI-2} 2-(tert-Butyloxycarbonyl)ethyl ist.

8. Verfahren nach einem der vorstehenden Ansprüche, das einen Schritt (y) umfasst
(y) Entfernen aller verbleibenden Schutzgruppen aus dem aus dem zuletzt durchgeführten Kondensationsschritt erhaltenen Peptid, um ein von Schutzgruppen freies Peptid zu erhalten.

9. Verfahren nach einem der vorstehenden Ansprüche, wobei R^{L-O-1} und R^{L-O-2} unabhängig voneinander tert-Butyl oder 3-Methyl-pent-3-yl sind.

10. Verfahren nach einem der vorstehenden Ansprüche, wobei in Schritt (c) die Verbindung der Formel Pr-L mit einem Kondensationsmittel aus Schritt (c) aktiviert wird.

11. Verfahren nach einem der vorstehenden Ansprüche, wobei in Schritt (c) ein Kupplungsadditiv von Schritt (c) vorhanden ist.

12. Verfahren nach den Ansprüchen 10 und 11, wobei das Kondensationsmittel des Schritts (c) ein Carbodiimid-Derivat ist und das Kupplungsadditiv des Schritts (c) Cyano-hydroxyiminoessigsäureethylester ist.

13. Verfahren nach einem der vorstehenden Ansprüche, wobei der Schritt (c) in einem Lösungsmittel des Schritts (c) durchgeführt wird und das Lösungsmittel des Schritts (c) N,N-Dimethylformamid umfasst.

14. Eine Verbindung der Formel Pr-L 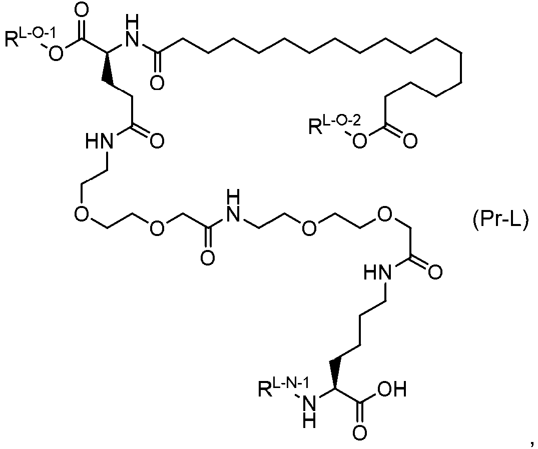 wobei
R^{L-O-1} und R^{L-O-2} unabhängig voneinander eine Carbonsäure-Schutzgruppe sind,
R^{L-N-1} eine Aminoschutzgruppe der Formel Bsmoc ist 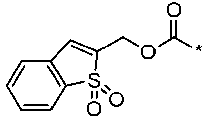 wobei * die Bindung an das Stickstoffatom
(Bsmoc) anzeigt.

15. Verwendung einer Verbindung der Formel Pr-L gemäß Anspruch 1 bei der Synthese eines Peptids P.

## Revendications

1. Procédé de fabrication d'un peptide P, le procédé comprend une étape (c)
(c) condensation d'un dérivé d'acide alpha aminé S-am, qui possède un groupe alpha amino non protégé ou un groupe alpha imino non protégé, avec un composé de formule Pr-L 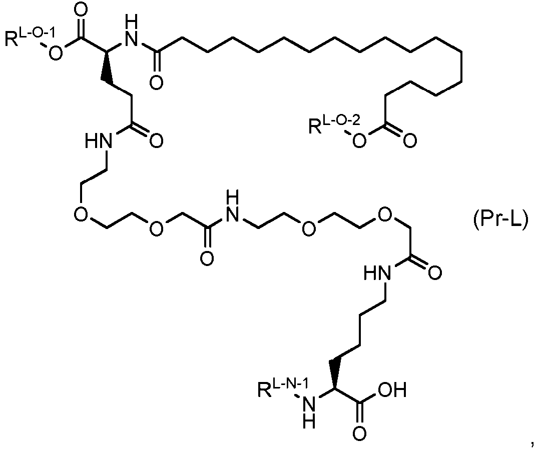 dans laquelle
R^{L-O-1} et R^{L-O-2} sont indépendamment l'un de l'autre groupe protecteur d'acide carboxylique,
pour obtenir un peptide Pr-L-S,
dans laquelle R^{L-N-1} est un groupe protecteur d'amino de formule Bsmoc 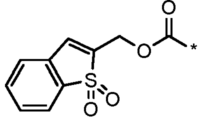
* indiquant la liaison à l'atome d'azote
(Bsmoc).

2. Procédé selon la revendication 1, dans lequel le dérivé d'acide alpha aminé S-am est un composé de formule S-I-am ou S-II-am 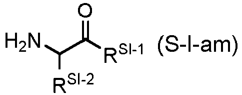 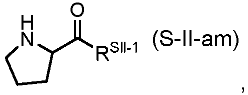 dans laquelle
R^{SI-1} est -(AA^{nx})ₘ-O-[résine-1], -(AA^{nx})ₘ-N-[résine-2], -O-[résine-1], -N-[résine-2], -(AA^{nx})ₘ-OR^{SI-1-1}, OR^{SI-1-1} ou NH₂,
R^{SI-2} est H, C₁₋₆ alkyle ou C₁₋₆ alkyle monosubstitué par OR^{SI-2-1}, SR^{SI-2-2}, SCH₃, NR^{SI-2-3}R^{SI-2-4}, CO-OR^{SI-2-5}, CO-NR^{SI-2-6}R^{SI-2-7}, N'-R^{SI-2-8}-N"-R^{SI-2-9}-guanidino, phényle, para-(R^{SI-2-10}O)-phényle, 1-R^{SI-2-11}-imidazol-4-yle ou 1-R^{SI-2-12}-indol-3-yle, -(AA^{nx})ₘ- est un substituant constitué de m résidus d'acides alpha aminés condensés AA^{nx}, chaque x étant un entier et x allant de 1 à m,
chaque résidu d'acide alpha aminé condensé AA^{nx} est choisi indépendamment et dans le cas où il possède une chaîne latérale avec un groupe fonctionnel, le groupe fonctionnel n'est pas protégé ou protégé par un groupe protecteur à condition qu'un groupe fonctionnel interférant soit protégé,
m est un entier de 1 à 30,
R^{SI-1-1} est H ou un groupe protecteur d'acide carboxylique
R^{SI-2-1} est H ou un groupe protecteur d'hydroxy,
R^{SI-2-2} est un groupe protecteur de thiol,
R^{SI-2-3} et R^{SI-2-4} sont H, un groupe protecteur d'amino ou forment ensemble un groupe protecteur d'amino à condition que tous deux ne soient pas H,
R^{SI-2-5} est H ou un groupe protecteur d'acide carboxylique,
R^{SI-2-6} et R^{SI-2-7} sont H ou un groupe protecteur d'amide,
R^{SI-2-8} et R^{SI-2-9} sont H ou un groupe protecteur de guanidino à condition que tous deux ne soient pas H,
R^{SI-2-10} est un groupe protecteur pour un groupe hydroxy aromatique,
R^{SI-2-11} est H ou un groupe protecteur pour un atome d'azote d'imidazole,
R^{SI-2-12} est H ou un groupe protecteur pour un atome d'azote d'indole,
R^{SII-1} est tel que défini pour R^{SI-1},
et le peptide Pr-L-S est un composé de formule Pr-L-S-I ou Pr-L-S-II 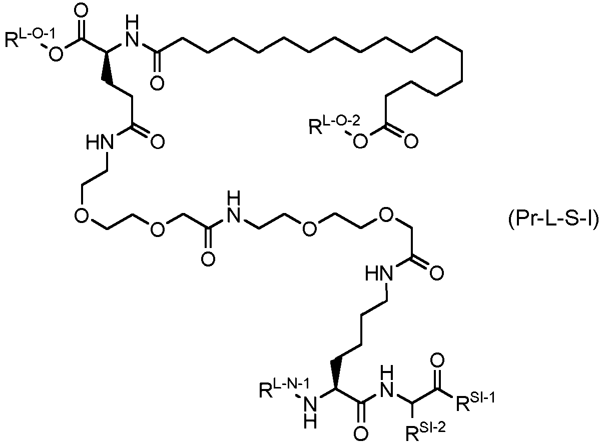 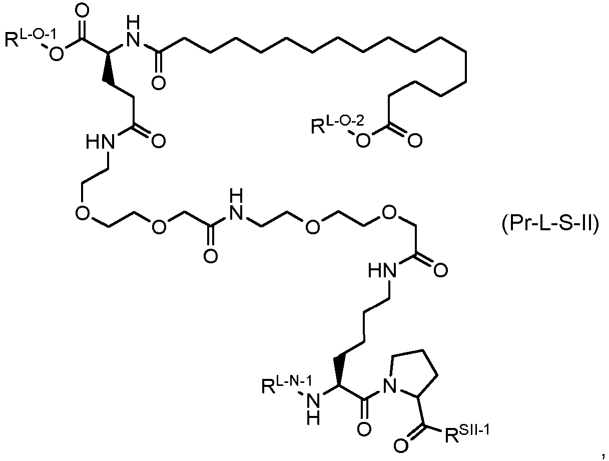
dans laquelle
R^{L-O-1}, R^{L-O-2} et R^{L-N-1} sont définis comme pour la formule Pr-L,
R^{SI-1} R-^{SI-2} et R^{SII-1} sont définis comme pour la formule S-I-am ou S-II-am.

3. Procédé selon la revendication 2, qui comprend l'étape (d)
(d) élimination du groupe protecteur d'amino R^{L-N-1} au niveau du composé de formule Pr-L-S-I ou Pr-L-S-II pour obtenir un composé de formule L-S-I-am ou L-S-II-am 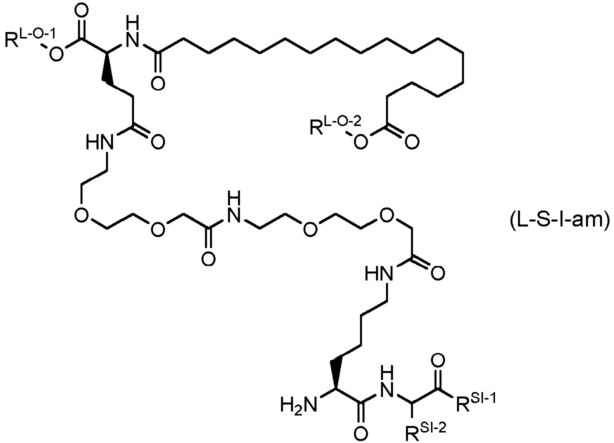 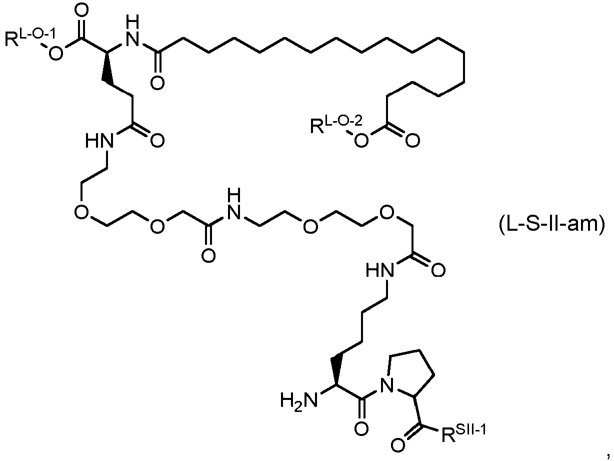 dans laquelle
R^{L-O-1}, R^{L-O-2}, R^{SI-1}, R^{SI-2} et R^{SII-2} sont définis comme pour la formule Pr-L-S-I ou Pr-L-S-II.

4. Procédé selon la revendication 3, qui comprend l'étape (e)
(e) condensation du composé de formule L-S-I-am ou L-S-II-am avec un dérivé d'acide alpha aminé de formule T
R^{T-N-1}-(AA^{py})_{q}-OH (T)
dans laquelle
R^{T-N-1} est un groupe protecteur d'amino,
-(AA^{py})_{q}- est un substituant constitué de q résidus d'acides alpha aminés condensés AA^{py}, chaque y étant un entier et y allant de 1 à q,
chaque résidu d'acide alpha aminé condensé AA^{py} est choisi indépendamment et dans le cas où il possède une chaîne latérale avec un groupe fonctionnel, le groupe fonctionnel n'est pas protégé ou protégé par un groupe protecteur à condition qu'un groupe fonctionnel interférant soit protégé,
q est un entier de 1 à 30,
pour obtenir un composé de formule T-L-S-I ou T-L-S-II 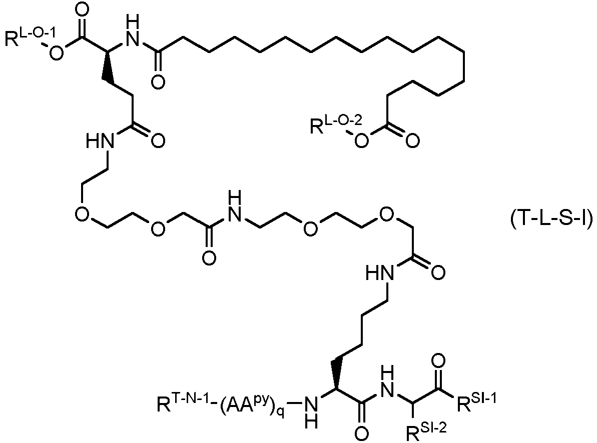 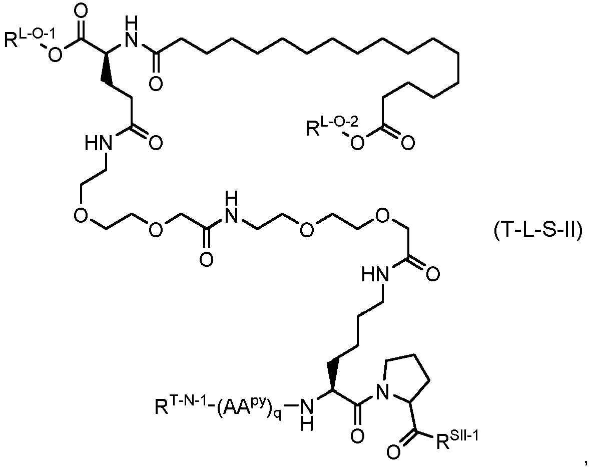
dans laquelle
R^{L-O-1}, R^{L-O-2}, R^{SI-1}, R^{SI-2} et R^{SII-2} sont définis comme pour la formule L-S-I-am ou L-S-II-am,
R^{T-N-1} et -(AA^{py})_{q}- sont définis comme pour la formule T.

5. Procédé selon la revendication 4, qui comprend un ou plusieurs cycles de condensation supplémentaires appliqués au composé de formule T-L-S-I ou T-L-S-II, chacun des un ou plusieurs cycles de condensation supplémentaires comprenant
une première étape d'élimination du groupe protecteur d'amino R^{T-N-1} du composé de formule T-L-S-I ou T-L-S-II respectivement du groupe protecteur d'amino du groupe alpha amino du résidu d'acide aminé N-terminal du peptide résultant du cycle de condensation précédent pour obtenir le peptide apparenté comportant un groupe amino non protégé, et une deuxième étape de condensation du groupe amino non protégé du peptide apparenté obtenu à la première étape avec un dérivé d'acide alpha aminé, qui a un groupe alpha amino au niveau de son résidu d'acide aminé N-terminal, qui est protégé par un groupe protecteur d'amino et qui a un groupe acide carboxylique en alpha non protégé, qui est situé au niveau de son résidu d'acide aminé C-terminal, pour obtenir un peptide résultant du cycle de condensation.

6. Procédé selon l'une quelconque des revendications précédentes, dans lequel le dérivé d'acide alpha aminé S-am est lié de manière covalente à une résine, et qui comprend une étape (x)
(x) clivage du peptide obtenu de la dernière étape de condensation conduite à partir de la résine par une composition de clivage pour obtenir un peptide clivé.

7. Procédé selon l'une quelconque des revendications 2 à 6, dans lequel le dérivé d'acide alpha aminé S-am de l'étape (c) est de formule S-I-am, dans laquelle
R^{SI-1} est un -(AA^{nx})₁₀-O-[résine-1],
AAⁿ¹ est Phe, AAⁿ² est lie, AAⁿ³ est Ala, AAⁿ⁴ est un Trp ayant une chaîne latérale protégée, AAⁿ⁵ est Leu, AAⁿ⁶ est Val, AAⁿ⁷ est une Arg ayant une chaîne latérale protégée, AAⁿ⁸ est Gly, AAⁿ⁹ est une Arg ayant une chaîne latérale protégée et AAⁿ¹⁰ est Gly,
la résine-1 est une résine de 2-chlorotritylamidométhyle ou une résine de 2-chlorotrityle,
R^{SI-2} est 2-(tert-butyloxycarbonyl)éthyle.

8. Procédé selon l'une quelconque des revendications précédentes, qui comprend une étape (y)
(y) élimination de tous les groupes protecteurs restants du peptide obtenu lors de la dernière étape de condensation effectuée pour obtenir un peptide exempt de groupes protecteurs.

9. Procédé selon l'une quelconque des revendications précédentes, dans lequel R^{L-O-1} et R^{L-O-2} sont indépendamment l'un de l'autre tert-butyle ou 3-méthyl-pent-3-yle.

10. Procédé selon l'une quelconque des revendications précédentes, dans lequel, à l'étape (c), le composé de formule Pr-L est activé avec un agent de condensation de l'étape (c).

11. Procédé selon l'une quelconque des revendications précédentes, dans lequel, à l'étape (c), un additif de couplage de l'étape (c) est présent.

12. Procédé selon les revendications 10 et 11, dans lequel l'agent de condensation de l'étape (c) est un dérivé de carbodiimide, et l'additif de couplage de l'étape (c) est l'ester éthylique de l'acide cyano-hydroxyimino-acétique.

13. Procédé selon l'une quelconque des revendications précédentes, dans lequel l'étape (c) est conduite dans un solvant de l'étape (c) et le solvant de l'étape (c) comprend du N,N-diméthylformamide.

14. Composé de formule Pr-L 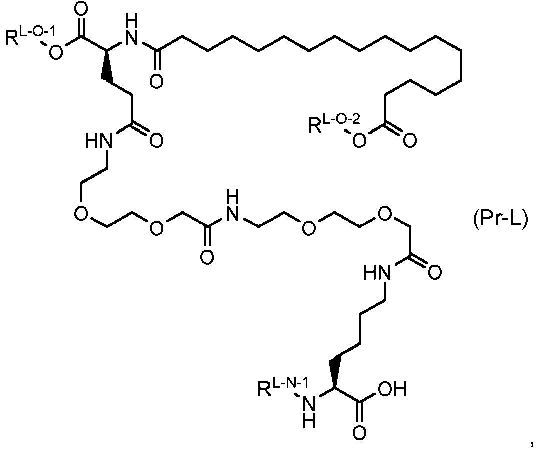 dans laquelle
R^{L-O-1} et R^{L-O-2} sont indépendamment l'un de l'autre groupe protecteur d'acide carboxylique,
R^{L-N-1} est un groupe protecteur d'amino de formule Bsmoc 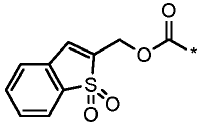
* indiquant la liaison à l'atome d'azote
(Bsmoc).

15. Utilisation d'un composé de formule Pr-L tel que défini dans la revendication 1 dans la synthèse d'un peptide P.
